# EUROPEAN PATENT SPECIFICATION

(11) **EP 1 007 530 B1**
(45) Date of publication and mention of the grant of the patent: **16.11.2005**
(21) Application number: 97924605.5
(22) Date of filing: 06.05.1997
(51) Int. Cl.: C07H 17/08, A61K 31/70

(54) **6-O-SUBSTITUTED ERYTHROMYCIN COMPOUNDS AND METHOD FOR MAKING SAME**
6-0-SUBSTITUTIERTE ERYTHTHROMYCIN-VERBINDUNGEN UND VERFAHRENEN ZU IHRER HERSTELLUNG
COMPOSES A BASE D'ERYTHROMYCINE 6-0 SUBSTITUEE ET TECHNIQUE DE PRODUCTION

(30) Priority: 07.05.1996 US 646477; 29.04.1997 US 841038
(43) Date of publication of application: 14.06.2000
(73) Proprietor: ABBOTT LABORATORIES, Abbott Park, Illinois 60064-3500 (US)
(72) Inventor: OR, Yat, Sun, Libertyville, IL 60048 (US); CLARK, Richard, F., Mundelein, IL 60060 (US); MA, Zhenkun, Gurnee, IL 60031 (US); GRIESGRABER, George, Eagan, MN 55123-3046 (US); LI, Leping, Gurnee, IL 60031 (US); CHU, Daniel, T., Santa Clara, CA 95051 (US)
(74) Representative: Modiano, Micaela Nadia
(86) International application number: PCT/US1997/007702
(87) International publication number: WO 1997/042206

(56) References cited:
- WO-A-97/19095
- CHEMICAL ABSTRACTS, vol. 118, no. 11, 15 March 1993 Columbus, Ohio, US; abstract no. 93800d, S.OMURA ET AL.: "Preparation of Erythromycin A Derivatives as Intermediates for 6-O-Alkylerythromycin" page 18; column 1; XP002035993 & YAKUGAKU ZASSHI , vol. 112, no. 9, 1992, pages 593-614,
- CHEMICAL ABSTRACTS, vol. 113, no. 15, 8 October 1990 Columbus, Ohio, US; abstract no. 132694r, S.MORIMOTO ET AL.: "Preparation of Erythromycin A Derivatives as Intermediates for 6-O-Alkylerythromycin " page 706; column 1; XP002035994 & JP 02 076 893 A (TAISHO PHARMACEUTICAL CO., LTD.)
- JOURNAL OF PHARMACEUTICAL SCIENCES, vol. 79, no. 9, 1989, pages 783-784, XP002035991 T.SUWA ET AL.: "Uptake of O-Alkyl Erythromycin Derivatives in the Lung Tissue and Cells of Rats."
- CANADIAN JOURNAL OF CHEMISTRY, vol. 63, no. 10, 1985, pages 2814-2818, XP002035992 B.BERNET ET AL.: "Formal Total Synthesis of Erythromycin A. Part II. Preparation of a 1,7-Dioxaspiro(5.5)undecane Derivative of Erythronolide A Seco Acid Methyl Ester from Erythromycin A."

## Description

The present invention relates to novel semisynthetic macrolides having antibacterial activity and useful in the treatment and prevention of bacterial infections. More particularly, the invention relates to 6-O-substituted erythromycin derivatives, compositions containing such compounds and methods for using the same, as well as processes for making such compounds.

### Background of the Invention

Erythromycins A through D, represented by formula (I),

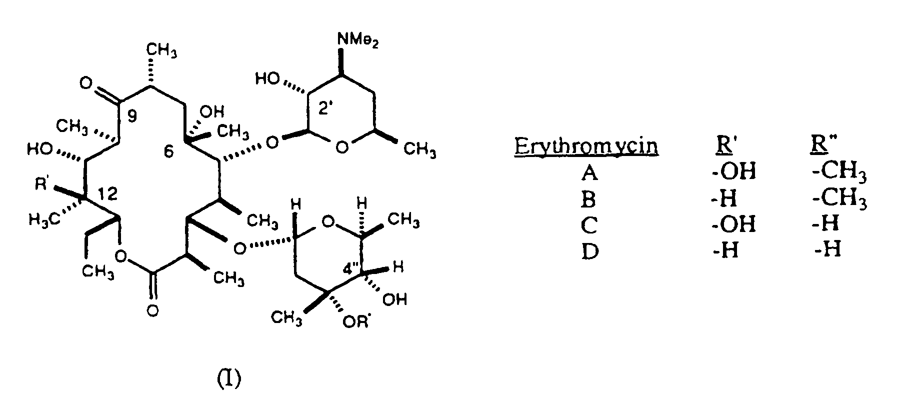

are well-known and potent antibacterial agents, used widely to treat and prevent bacterial infection. As with other antibacterials, however, bacterial strains having resistance or insufficient susceptibility to erythromycin have been identified. Also, erythromycin A has only weak activity against Gram-negative bacteria. Therefore, there is a continuing need to identify new erythromycin derivative compounds which possess improved antibacterial activity, which have less potential for developing resistance, which possess the desired Gram-negative activity, or which possess unexpected selectivity against target microorganisms. Consequently, numerous investigators have prepared chemical derivatives of erythromycin in an attempt to obtain analogs having modified or improved profiles of antibiotic activity.

Morimoto *et al.* described the preparation of 6-O-methyl erythromycin A in J. Antibiotics 37:187 (1984). Morimoto *et al.* further disclosed a series of O-alkyl erythromycin A derivatives in J. Antibiotics 43: 286 (1990). In their experience, "O-alkylation, other than methylation, took place at the C-11 hydroxyl group exclusively." However, in European Patent Application 272,110, published June 22, 1988, Morimoto *et al.* disclose 6-O-C₁-C₃-alkyl erythromycin A compounds.

In European Patent Application 215,355, published March 28, 1987, Omura and Itoh disclose 6-O-loweralkyl erythromycins as stimulants of gastrointestinal contractile motion. Chem Abstr., 113(15), abstract no. 1326942 and JP 02 076 893 discloses 6-O-(C₁₋₃) alkylerythromycin derivatives obtained by alkylating the protected hydroxy group in position 6 with C₁-C₃ alkylating agent. Chem Abstr., 118(11), abstract no. 93800d, Omura S. ET AL . YAKUGAKU ZASSHI, 112 (9), 1992, 593-614, discloses a series of 6-O-alkylated erythromycin derivatives among which clarithromycin proved to be the most patent and Suna, T. et al JOURNAL OF PHARMACEUTICAL SCIENCES, 79(9), 1989, 783-784 discloses 6-O-ethylerythromycin.

### Summary of the Invention

The present invention provides a novel class of 6-O-substituted erythromycin compounds which possess antibacterial activity.

In one aspect of the present invention is disclosed a novel 6-O-substituted erythromycin compound selected from the formula: 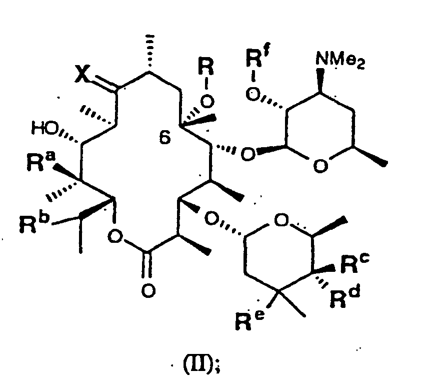 as well as the pharmaceutically acceptable salts. In formulae (II) -
X is selected from the group consisting of
(1) =O,
   R^{a} is hydroxy;
   R^{b} is hydrogen;
   R^{c} is hydrogen and R^{d} is hydroxy.
   R^{e} is methoxy;
   R^{f} is hydrogen;
   R is selected from the group consisting of
(2) C₂-C₁₀-alkyl substituted with one or more substituents selected from the group consisting of
   (a) halogen,
   (b) hydroxy,
   (c) C₁-C₃-alkoxy,
   (d) C₁-C₃-alkoxy-C₁-C₃-alkoxy,
   (e) oxo,
   (f) -N₃,
   (g) -CHO,
   (h) O-SO₂-(substituted C₁-C₆-alkyl),
   (i) -NR¹⁵R¹⁶ wherein R¹⁵ and R¹⁶ are selected from the group consisting of
      (i) hydrogen,
      (ii) C₁-C₁₂-alkyl,
      (iii) substituted C₁-C₁₂-alkyl,
      (iv) C₁-C₁₂-alkenyl,
      (v) substituted C₁-C₁₂-alkenyl,
      (vi) C₁-C₁₂-alkynyl,
      (vii) substituted C₁-C₁₂-alkynyl,
      (viii) aryl,
      (ix) C₃-C₈-cycloalkyl,
      (x) substituted C₃-C₈-cycloalkyl,
      (xi) substituted aryl,
      (xii) heterocycloalkyl,
      (xiii) substituted heterocycloalkyl,
      (xiv) C₁-C₁₂-alkyl substituted with aryl,
      (xv) C₁-C₁₂-alkyl substituted with substituted aryl,
      (xvi) C₁-C₁₂-alkyl substituted with heterocycloalkyl,
      (xvii) C₁-C₁₂-alkyl substituted with substituted heterocycloalkyl,
      (xviii) C₁-C₁₂-alkyl substituted with C₃-C₈-cycloalkyl.
      (xix) C₁-C₁₂-alkyl substituted with substituted C₃-C₈-cycloalkyl,
      (xx) heteroaryl,
      (xxi) substituted heteroaryl,
      (xxii) C₁-C₁₂-alkyl substituted with heteroaryl,
         and
      (xxiii) C₁-C₁₂-alkyl substituted with substituted heteroaryl,
   or
   R¹⁵ and R¹⁶ taken together with the atom to which they are attached form morpholinyl
(3) C₄-C₁₀-alkenyl; and
(4) C₃-C₁₀-alkynyl.

In another aspect of the present invention are disclosed pharmaceutical compositions comprising a therapeutically effective amount of a compound of the invention in combination with a pharmaceutically acceptable carrier and treatment of antibacterial infections with such compositions. Suitable carriers and methods of formulation are also disclosed. The compounds and compositions of the present invention have antibacterial activity.

In a further aspect of the present invention are provided processes for the preparation of 6-O-substituted macrolide derivatives of Formula (II).

### Detailed Description of the Invention

One embodiment of the present invention comprises a compound of formula (II) above, wherein X, R, R^{a}, R^{b}, R^{c}, R^{d}, R^{e} and R^{f} are as defined above.

An embodiment of the present invention is the compound of formula (X): 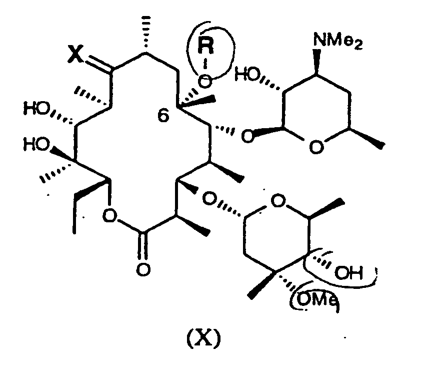 where X and R are as defined above.

A preferred intermediate in the preparation of the compound of formula (X) is the compound of formula (XII) 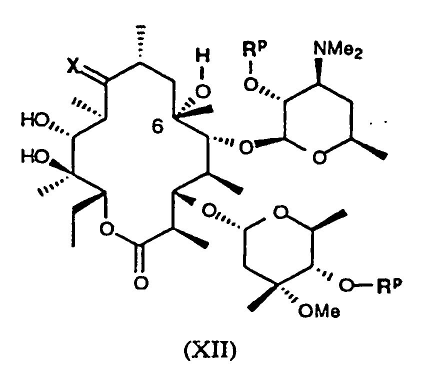 where X is as defined above and R^{p} is a hydroxy protecting group.

(G) a compound wherein R^{a} is OH; R^{b} is H; R^{c} is H; R^{d} is OH; R^{e} is methoxy;
R^{f} is H; selected from the group consisting of compounds wherein
(1) X is =O, R is -CH₂-CH=CH₂-(3-quinolinyl);
(2) X is =O, R is allyl;
(5) X is =O, R is 2,3-dihydroxypropyl;
(6) X is =O, R is 2,3-epoxypropyl;
(7) X is =O, R is 2-hydroxy-3-(imidazol-1-yl)propyl;
(8) X is =O, R is 2-hydroxy-3-(morpholin-4-yl)propyl;
(9) X is =O, R is 2-hydroxy-3-(benzylamino)propyl;
(10) X is =O, R is 2-oxoethyl;
(11) X is =O, R is 2-oxopropyl;
(14) X is =O, R is -CH₂-C≡CH;
(15) X is =O, R is -CH₂-CHOH-CH₂-N₃;
(16) X is =O, R is -CH₂-CH=N-OH;
(17) X is =O, R is -CH₂-CH₂OH;
(18) X is =O, R is -CH₂-CH₂NH2;
(19) X is =O, R is -CH₂-CN;
(20) X is =O, R is -CH₂-Phenyl;
(21) X is =O, R is -CH₂-CH=CH-Phenyl-;
(22) X is =O, R is -CH₂-CH=N-O-CH₃;
(23) X is =O, R is -CH₂-CH=N-O-CH₂-Phenyl;
(24) X is =O, R is -CH₂-CH=N-N(CH₃)₂;
(25) X is =O, R is -CH₂-CH=N-NH(CH₃);
(26) X is =O, R is -CH₂-CH=N-(4-Morpholinyl);
(27) X is =O, R is -CH₂-CH=N-NH(Phenyl); and
(28) X is =O, R is -CH₂-CH=N-N(Phenyl)₂;
(29) X=O, R=Phenylpropyl;
(30) X=O, R is -CH₂CH=CH-(4-methylphenyl);
(31) X=O, R is -CH₂-CH(OH)-Phenyl;
(32) X=O, R is -CH₂-CH(Br)-CH₂Br;
(33) X=O, R is -CH₂CH₂NHCH₂CH₂CH₂-Phenyl;
(34) X=O, R is -CH₂CH₂NHCH(CH₂Phenyl)CO₂Me;
(35) X=O, R is -CH₂CH₂NHCH₂CH₂CH₃;
(36) X=O, R is -CH₂CH₂NHCH₂CO₂CH₂CH₂;
(37) X=O, R is -CH₂CH₂NHCH₂CH₂-Phenyl;
(38) X=O, R is -CH2CH₂NHCH₂CH₂-(4-hydroxyphenyl);
(39) X=O, R is -CH₂CH₂NHCH₂CH₂-(3-hydroxyphenyl);
(40) X=O, R is -CH₂CH₂NHCH₂CH₂-(3-methoxyphenyl);
(41) X=O, R is -CH2CH2NHCH₂CH₂-(2-methoxyphenyl);
(42) X=O, R is -CH₂CH₂NHCH₂CH₂-(4-methoxyphenyl);
(43) X=O, R is -CH₂CH₂NHCH₂-phenyl;
(45) X=O, R is -CH₂CH₂NHCH₂CH₂-(3-chlorophenyl);
(46) X=O, R is -CH₂CH₂NHCH₂CH₂-(2-chlorophenyl);
(47) X=O, R is -CH₂CH₂NHCH₂CH₂-(4-chlorophenyl);
(48) X=O, R is -CH₂CH₂NHCH₂CH₂-O-phenyl);
(49) X=O, R is -CH₂CH₂NHCH₂CH₂CH₂-(4-quinotinyl);
(50) X=O, R is -CH₂CH₂NHCH₂CH₂CH₂-(3-quinolinyl);
(51) X=O, R is -CH₂CH₂NHCH₂CH₂CH₂CH₂-phenyl;
(52) X=O, R is -CH₂-CH=N-NH-C(O)-NH₂;
(53) X=O, R is -CH₂-CH=N-NH-(2-pyridinyl);
(54) X=O, R is -CH₂-CH=N-(4-methylpiperazinyl);
(55) X=O, R is -CH₂-CH=N-O-phenyl;
(56) X=O, R is -CH₂CH(OH)CH₂NHCH₂CH₂-phenyl;
(57) X=O, R is -CH₂CH(OH)CH₂NHCH₂-(4-pyridinyl;
(58) X is =O, R is (3-iodophenyl)methyl; and
(59) X is =O, R is (4-fluorbophenyl)methyl;
and pharmaceutically acceptable salts therof.

Preferred compounds are those selected from the group consisting of
(A) a compound wherein R^{a} is OH; R^{b} is H; R^{c} is H; R^{d} is H; R^{e} is methoxy;
   R^{f} is H; selected from the group consisting of compounds wherein
   (1) X is =O, R is -CH₂-CH=CH₂-(3-quinolinyl);
   (2) X is =O, R is allyl;
   (5) X is =O, R is 2,3-dihydroxypropyl;
   (6) X is =O, R is 2,3-epoxypropyl;
   (7) X is =O, R is 2-hydroxy-3-(imidazol-1-yl)propyl;
   (8) X is =O, R is 2-hydroxy-3-(morpholin-4-yl)propyl;
   (9) X is =O, R is 2-hydroxy-3-(benzylamino)propyl;
   (10) X is =O, R is 2-oxoethyl;
   (11) X is =O, R is 2-oxopropyl;
   (14) X is =o, R is -CH₂-C≡CH;
   (15) X is =O, R is -CH₂-CHOH-CH₂-N₃;
   (16) X is =O, R is -CH₂-CH=N-OH;
   (17) X is =O, R is -CH₂-CH₂OH;
   (18) X is =O, R is -CH₂-CH₂NH₂;
   (19) X is =O, R is -CH₂-CN;
   (20) X is =O, R is -CH₂-Phenyl;
   (21) X is =O, R is -CH₂-CH=CH-Phenyl-;
   (22) X is =O, R is -CH₂-CH=N-O-CH₃:
   (23) X is =O, R is -CH₂-CH=N-O-CH₂-Phenyl;
   (24) X is =O, R is -CH₂-CH=N-N(CH3)₂;
   (25) X is =O, R is -CH₂-CH=N-NH(CH₃);
   (26) X is =O, R is -CH₂-CH=N-(4-Morpholinyl);
   (27) X is =O, R is -CH₂-CH=N-NH(Phenyl); and
   (28) X is =O, R is -CH₂-CH=N-N(Phenyl)₂;
   (29) X=O, R=Phenylpropyl;
   (30) X=O, R is -CH₂CH=CH-(4-methylphenyl);
   (31) X=O, R is -CH₂-CH(OH)-Phenyl;
   (32) X=O, R is -CH₂-CH(Br)-CH₂Br;
   (33) X=O, R is -CH₂CH₂NHCH₂CH₂CH₂-Phenyl;
   (34) X=O, R is -CH₂CH₂NHCH(CH₂Phenyl)CO₂Me;
   (35) X=O, R is -CH₂CH₂NHCH₂CH₂CH₃;
   (36) X=O, R is -CH₂CH₂NHCH₂CO₂CH₂CH₂;
   (37) X=O, R is -CH₂CH₂NHCH₂CH₂-Phenyl;
   (38) X=O, R is -CH₂CH₂NHCH₂CH₂-(4-hydroxyphenyl);
   (39) X=O, R is -CH2CH₂NHCH₂CH₂-(3-hydroxyphenyl);
   (40) X=O, R is -CH₂CH₂NHCH₂CH₂-(3-methoxyphenyl);
   (41) X=O, R is -CH₂CH₂NHCH₂CH₂-(2-methoxyphenyl);
   (42) X=O, R is -CH₂CH₂NHCH₂CH₂-(4-methoxyphenyl);
   (43) X=O, R is -CH₂CH₂NHCH₂-phenyl;
   (45) X=O, R is -CH₂CH₂NHCH₂CH₂-(3-chlorophenyl);
   (46) X=O, R is -CH₂CH₂NHCH₂CH₂-(2-chlorophenyl);
   (47) X=O, R is -CH₂CH₂NHCH₂CH₂-(4-chlorophenyl);
   (48) X=O, R is -CH₂CH₂NHCH₂CH₂-O-phenyl);
   (49) X=O, R is -CH₂CH₂NHCH₂CH₂CH₂-(4-quinolinyl);
   (50) X=O, R is -CH₂CH₂NHCH₂CH₂CH₂-(3-quinolinyl);
   (51) X=O, R is -CH₂CH₂NHCH₂CH₂CH₂CH₂-phenyl;
   (52) X=O, R is -CH₂-CH=N-NH-C(O)-NH₂;
   (53) X=O, R is -CH₂-CH=N-NH-(2-pyridinyl);
   (54) X=O, R is -CH₂-CH=N-(4-methylpiperazinyl);
   (55) X=O, R is -CH₂-CH=N-O-phenyl;
   (56) X=O, R is -CH₂CH(OH)CH₂NHCH₂CH₂-phenyl;
   (57) X=O, R is -CH₂CH(OH)CH₂NHCH₂-(4-pyridinyl;
   (58) X is =O, R is (3-iodophenyl)methyl; and
   (59) X is =O, R is (4-fluorophenyl)methyl.

More preferred compounds are selected from the group consisting of:
Compound of Formula (X): X is =O, R is -CH₂-CH=CH₂-(3-quinolinyl);
Compound of Formula (X): X is =O, R is allyl;
Compound of Formula (X): X is =O, R is 2-hydroxy-3-(benzylamino)propyl;
Compound of Formula (X): X is =O, R is 2-oxopropyl;
Compound of Formula (X): X is =O. R is -CH₂-C≡CH;
Compound of Formula (X): X is =O, R is -CH₂-CH=N-OH;
Compound of Formula (X): X is =O, R is -CH₂-CH₂OH;
Compound of Formula (X): X is =O, R is -CH₂-CH₂NH₂; and
Compound of Formula (X): X is =O, R is -CH₂-CN;
as well as the pharmaceutically acceptable salts thereof.

A process for the preparation of 6-O-substituted macrolide derivatives having the Formula: 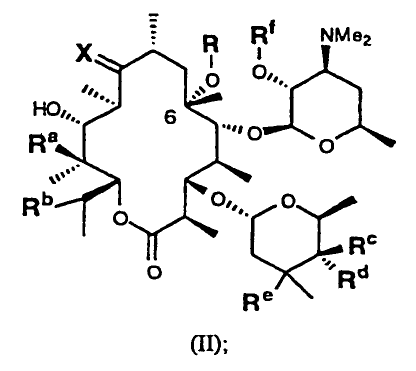 wherein
X is selected from the group consisting of
(1) =O,
   V is =N-O-R¹ where R¹ is selected from the group consisting of
   (a) unsubstituted C₁-C₁₂-alkyl,
   (b) C₁-C₁₂-alkyl substituted with aryl,
   (c) C₁-C₁₂-alkyl substituted with substituted aryl,
   (d) C₁-C₁₂-alkyl substituted with heteroaryl,
   (e) C₁-C₁₂-alkyl substituted with substituted heteroaryl,
   (f) C₃-C₁₂-cycloalkyl,
   (g) -Si-(R²)(R³)(R⁴) wherein R², R³ and R⁴ are each independently selected from C₁-C₁₂-alkyl,
      and
   (h) -Si-(Aryl)₃;
   or
(4) =N-O-C(R⁵)(R⁶)-O-R¹ where R¹ is as defined above and R⁵ and R⁶ are each independently selected from the group consisting of
   (a) hydrogen,
   (b) unsubstituted C₁-C₁₂-alkyl,
   (c) C₁-C₁₂-alkyl substituted with aryl,
   (d) C₁-C₁₂-alkyl substituted with substituted aryl,
   (e) C₁-C₁₂-alkyl substituted with heteroaryl,
      and
   (f) C₁-C₁₂-alkyl substituted with substituted heteroaryl,
   or
   R⁵ and R⁶ taken together with the atom to which they are attached form a C₃-C₁₂-cycloalkyl ring;
R^{a} is hydroxy;
R^{b} is hydrogen
R^{c} is hydrogen and R^{d} is of hydroxy,
R^{e} is methoxy;
R^{f} is hydrogen;
R is selected from the group consisting of
(3) C₂-C₁₀-alkyl substituted with one or more substituents selected from the group consisting of
   (a) halogen,
   (b) hydroxy,
   (c) C₁-C₃-alkoxy,
   (d) C₁-C₃-alkoxy-C₁-C₃-alkoxy.
   (e) oxo,
   (f) -N₃,
   (g) -CHO,
   (h) O-SO₂-(substituted C₁-C₆-alkyl),
   (i) -NR¹⁵R¹⁶ wherein R¹⁵ and R¹⁶ are selected from the group consisting of
      (i) hydrogen,
      (ii) C₁-C₁₂-alkyl,
      (iii) substituted C₁-C₁₂-alkyl.
      (iv) C₁-C₁₂-alkenyl,
      (v) substituted C₁-C₁₂-alkenyl,
      (vi) C₁-C₁₂-alkynyl,
      (vii) substituted C₁-C₁₂-alkynyl,
      (viii) aryl,
      (ix) C₃-C₈-cycloalkyl,
      (x) substituted C₃-C₈-cycloalkyl,
      (xi) substituted aryl,
      (xii) heterocycloalkyl,
      (xiii) substituted heterocycloalkyl,
      (xiv) C₁-C₁₂-alkyl substituted with aryl,
      (xv) C₁-C₁₂-alkyl substituted with substituted aryl,
      (xvi) C₁-C₁₂-alkyl substituted with heterocycloalkyl,
      (xvii) C₁-C₁₂-alkyl substituted with substituted heterocycloalkyl,
      (xviii) C₁-C₁₂-alkyl substituted with C₃-C₈-cycloalkyl,
      (xix) C₁-C₁₂-alkyl substituted with substituted C₃-C₈-cycloalkyl,
      (xx) heteroaryl,
      (xxi) substituted heteroaryl,
      (xxii) C₁-C₁₂-alkyl substituted with heteroaryl,
         and
      (xxiii) C₁-C₁₂-alkyl substituted with substituted heteroaryl,
   or
   R¹⁵ and R¹⁶ taken together with the atom to which they are attached form a morpholinyl;
(3) C₄-C₁₀-alkenyl;
(7) C₃-C₁₀-alkynyl;
   is a method comprising:
   (a) treating a compound having the formulae 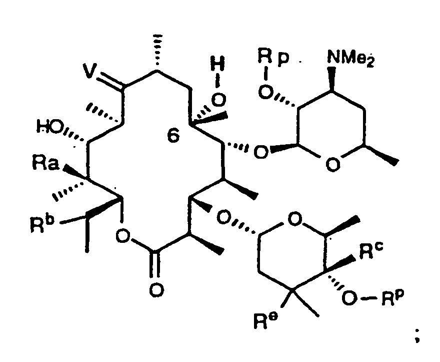 wherein R^{p} is a hydroxy protecting group and V is =N-O-R¹ or =N-O-C(R⁹)(R¹⁰)-O-R¹
      wherein R¹, R⁹ and R¹⁰ are as defined above, with a base in an aprotic solvent then with an alkylating agent to give a compound having the formula 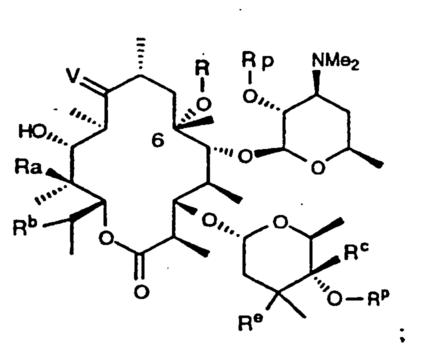 wherein X, R^{a}, R^{b}, R^{c}, R^{d}, R^{e}, R^{f}, are as defined above,
      V is =N-O-R¹ or =N-O-C(R⁵)(R⁶)-O-R¹ wherein R¹, R⁵ and R⁶ are as defined above, and
      R is the "alkyl group" derived from the corresponding alkylating agent;
   (b) deprotecting the 2'- and 4'-hydroxyl groups to give a compound of the formula 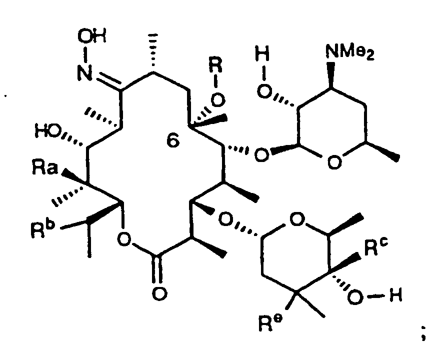 wherein X, R^{a}, R^{b}, R^{c}, R^{d}, R^{e}, R^{f}, are as defined above and R is the "alkyl group" derived from the corresponding alkylating agent;
      and
   (c) deoximation with an inorganic sulfur oxide salt or an inorganic nitrite salt in the presence of acid in a suitable solvent to give the desired products.

A preferred process for the preparation of 6-O-substituted macrolide compounds of the invention is the process immediately above wherein in step (a) the base is selected from the group consisting of potassium hydroxide, cesium hydroxide, tetraalkylammonium hydroxide, sodium hydride, potassium hydride, potassium isopropoxide, potassium tert-butoxide and potassium isobutoxide, the alkylating agent is selected from the group consisting of allyl bromide, propargyl bromide, benzyl bromide, 2-fluoroethyl bromide, 4-nitrobenzyl bromide, 4-chlorobenzyl bromide, 4-methoxybenzyl bromide, α-bromo-p-tolunitrile, cinnamyl bromide, methyl 4-bromocrotonate, crotyl bromide, 1-bromo-2-pentene, 3-bromo-1-propenyl phenyl sulfone. 3-bromo-1-trimethylsilyl-1-propyne, 3-bromo-2-octyne, 1-bromo-2-butyne, 2-picolyl chloride, 3-picolyl chloride, 4-picolyl chloride, 4-bromomethyl quinoline, bromoacetonitrile, epichlorohydrin, bromofluoromethane, bromonitromethane, methyl bromoacetate, methoxymethyl chloride, bromoacetamide, 2-bromoacetophenone, 1-bromo-2-butanone, bromo chloromethane, bromomethyl phenyl sulfone, 1,3-dibromo-1-propene, allyl O-tosylate, 3-phenylpropyl-O-trifluoromethane sulfonate, and n-butyl-O-methanesulfonate, and the reaction is performed at a temperature from about -15 °C to about 50 °C for a period from 0.5 hours to 10 days;

In the preferred process in step (b) deprotection is accomplished by use of acetic acid in water and acetonitrile.

In the preferred process in step (c) the deoximating reagent is an inorganic sulfur oxide compound is selected from the group consisting of sodium hydrogen sulfite, sodium pyrosulfate, sodium thiosulfate, sodium sulfate, sodium sulfite, sodium hydrosulfite, sodium metabisulfite, sodium dithionate, potassium thiosulfate, and potassium metabisulfite, or an inorganic nitrite salt in the presence of acid selected from the group consisting of sodium nitrite and potassium nitrite, and the solvent is selected from the group consisting of water, methanol, ethanol, propanol, isopropanol, trimethylsilanol or a mixture of one or more thereof.

A preferred process of the invention is a process as described above for the preparation of 6-O-substituted macrolide compounds having formula (II) thereof wherein in step (a) the starting compound of has the formula 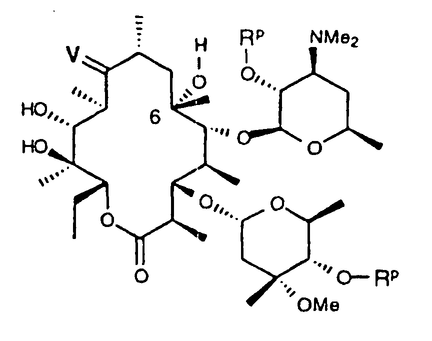 wherein R^{p} is trimethylsilyl and V is a ketone protecting group and it is treated with potassium hydroxide in a mixture of THF and DMSO, in step (b) deprotection of the 2'- and 4'-hydroxyl groups is accomplished using acetic acid in water and acetonitrile to give a compound having the formula

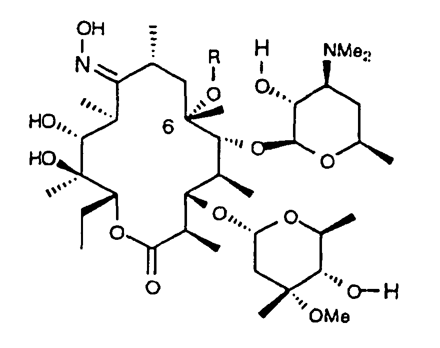

In this preferred process in step (c) the 9-oxime is deoximinated using NaHSO₃ and formic acid in ethanol-water.

In a more preferred process for the preparation of 6-O-substituted macrolide compounds having the formula having formula (II), in step (a) R^{p} is trimethylsilyl and the ketone protecting group is O-(1-isopropoxycyclohexyl) oxime.

### Definitions

The terms "C₁-C₁₂-alkyl" as used herein refer to saturated, straight- or branched-chain hydrocarbon radicals containing between one and twelve carbon atoms. Examples of C₁-C₃ alkyl radicals include methyl, ethyl, propyl and isopropyl, and examples of C₁-C₆-alkyl radicals include, but are not limited to, methyl, ethyl, propyl, isopropyl, *n*-butyl, *tert*-butyl, neopentyl and n-hexyl.

The term "C₁-C₆-alkoxy" as used herein refers to an C₁-C₆-alkyl group, as previously defined, attached to the parent molecular moiety through an oxygen atom. Examples of C₁-C₆-alkoxy, but are not limited to, methoxy, ethoxy, propoxy, isopropoxy, *n*-butoxy, *tert*-butoxy, neopentoxy and n-hexoxy.

The term "acylamino" as used herein refers to a C₁-C₆-alkyl, aryl, or substituted aryl group attached to the amino group via a carbonyl grouping. Examples of acylamino include, but are not limited to acetylamino, trifluoroacetylamino, propanoylamino, benzoylamino, 4-chlorbenzoylamino, and the like.

The term "alkenyl" as used herein refers to a branched or straight hydrocarbon chain comprising two to ten carbon atoms which also comprises one or more carbon-carbon double bonds. Representative alkenyl groups include 2-propenyl (i.e., allyl), 3-methyl-2-butenyl, 3,7-dimethyl-2,6-octadienyl, 4,8-dimethyl-3,7-nonadienyl, 3,7,11-trimethyl-2.6.10-dodecatrienyl and the like.

The term "alkynyl" as used herein refers to a branched or straight hydrocarbon chain comprising two to ten carbon atoms which also comprises one or more carbon-carbon triple bonds. Representative alkynyl groups include ethynyl, 2-propynyl (propargyl), 1-propynyl and the like.

The term "C₁-C₃-alkyl-amino" as used herein refers to one or two C₁-C₃-alkyl groups, as previously defined, attached to the parent molecular moiety through a nitrogen atom. Examples of C₁-C₃-alkyl-amino include, but are not limited to methylamino, dimethylamino, ethylamino, diethylamino, and propylamino.

The term "aprotic solvent" as used herein refers to a solvent that is relatively inert to proton activity, i.e., not acting as a proton-donor. Examples include, but are not limited to, hydrocarbons, such as hexane and toluene, for example, halogenated hydrocarbons, such as, for example, methylene chloride, ethylene chloride, chlorofom, and the like, heteroaryl compounds, such as, for example, tetrahydrofuran and N-methylpyrrolidinone, and ethers such as diethyl ether, bis-methoxymethyl ether. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick *et al.,* Vol. II, in the Techniques of Chemistry Series, John Wiley & Sons, NY, 1986.

The term "aryl" as used herein refers to a mono- or bicyclic carbocyclic ring system having one or two aromatic rings including, but not limited to, phenyl, naphthyl, tetrahydronaphthyl, indanyl, indenyl and the like. Aryl groups (including bicyclic aryl groups) can be unsubstituted or substituted with one, two or three substituents independently selected from loweralkyl, substituted loweralkyl, haloalkyl, alkoxy, thioakoxy, amino, alkylamino, dialkylamino, acylamino, cyano, hydroxy, halo, mercapto, nitro, carboxaldehyde, carboxy, alkoxycarbonyl and carboxamide. In addition, substituted aryl groups include tetrafluorophenyl and pentafluorophenyl.

The term "C₃-C₁₂-cycloalkyl" as used herein refers to carbocyclic groups of 3 to 12 carbons, respectively, for example, cyclopropyl, cyclobutyl, cyclopentyl, cyclohexyl, and cycloheptyl.

The term "C₁-C₃-alkyl-C₃-C₅-cycloalkyl", as used herein refers to a C₃-C₅-cycloalkyl radical, as defined above, attached to a C₁-C₃-alkyl radical by replacement of a hydrogen atom on the latter.

The terms "halo" and "halogen" as used herein refer to an atom selected from fluorine, chlorine, bromine and iodine.

The term "heteroaryl", as used herein, refers to a cyclic aromatic radical having from five to ten ring atoms of which one ring atom is selected from S. O and N; zero, one or two ring atoms are additional heteroatoms independently selected from S. O and N; and the remaining ring atoms are carbon, the radical being joined to the rest of the molecule via any of the ring atoms, such as, for example, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, and the like.

The term "heterocycloalkyl" as used herein, refers to a non-aromatic partially unsaturated or fully saturated 3- to 10-membered ring system, which includes single rings of 3 to 8 atoms in size and bi- or tri-cyclic ring systems which may include aromatic six-membered aryl or heteroaryl rings fused to a non-aromatic ring. These heterocycloalkyl rings include those having from one to three heteroatoms independently selected from oxygen, sulfur and nitrogen, in which the nitrogen and sulfur heteroatoms may optionally be oxidized and the nitrogen heteroatom may optionally be quaternized.

Representative heterocycloalkyl rings include, but are not limited to, oxiranyl, aziranyl, oxetanyl, azetidinyl, pyrrolidinyl, piperidinyl, pyrazolinyl, pyrazolidinyl, piperazinyl, azacycloheptanyl, azacyclooctanyl, 1,4-diazacycloheptanyl, imidazolidinyl, oxazolidinyl, isoxazolidinyl, morpholinyl, thiazolidinyl, isothiazolidinyl, and tetrahydrofuryl.

Specific heterocycloalkyl rings contained in the examples below include: 3-methyl-4-(3-methylphenyl)piperazine, 3-methylpiperidine, 4-(bis-(4-fluorophenyl)methyl)piperazine, 4-(diphenylmethyl)piperazine, 4-(ethoxycarbonyl)piperazine, 4-(ethoxycarbonylmethyl)piperazine, 4-(phenylmethyl)piperazine, 4-(1-phenylethyl)piperazine, 4-(1,1-dimethylethoxycarbonyl)piperazine, 4-(2-(bis-(2-propenyl)amino)ethyl)piperazine, 4-(2-(diethylamino)ethyl)piperazine, 4-(2-chlorophenyl)piperazine, 4-(2-cyanophenyl)piperazine, 4-(2-ethoxyphenyl)piperazine, 4-(2-ethylphenyl)piperazine, 4-(2-fluorophenyl)piperazine, 4-(2-hydroxyethyl)piperazine, 4-(2-methoxyethyl)piperazine, 4-(2-methoxyphenyl)piperazine, 4-(2-methylphenyl)piperazine, 4-(2-methylthiophenyl)piperazine, 4-(2-nitrophenyl)piperazine, 4-(2-nitrophenyl)piperazine, 4-(2-phenylethyl)piperazine, 4-(2-pyridyl)piperazine, 4-(2-pyrimidinyl)piperazine, 4-(2,3-dimethylphenyl)piperazine, 4-(2,4-difluorophenyl)piperazine, 4-(2,4-dimethoxyphenyl)piperazine, 4-(2,4-dimethylphenyl)piperazine, 4-(2,5-dimethylphenyl)piperazine, 4-(2,6-dimethylphenyl)piperazine, 4-(3-chlorophenyl)piperazine, 4-(3-methylphenyl)piperazine, 4-(3-trifluoromethylphenyl)piperazine, 4-(3,4-dichlorophenyl)piperazine, 4-(3,4-dimethoxyphenyl)piperazine, 4-(3,4-dimethylphenyl)piperazine, 4-(3,4-methylenedioxyphenyl)piperazine, 4-(3,4,5-trimethoxyphenyl)piperazine, 4-(3,5-dichlorophenyl)piperazine, 4-(3,5-dimethoxyphenyl)piperazine, 4-(4-(phenylmethoxy)phenyl)piperazine, 4-(4-(1,1-dimethylethyl)phenylmethyl)piperazine, 4-(4-chloro-3-trifluoromethylphenyl)piperazine, 4-(4-chlorophenyl)-3-methylpiperazine, 4-(4-chlorophenyl)piperazine, 4-(4-chlorophenyl)piperazine, 4-(4-chlorophenylmethyl)piperazine, 4-(4-fluorophenyl)piperazine, 4-(4-methoxyphenyl)piperazine, 4-(4-methylphenyl)piperazine, 4-(4-nitrophenyl)piperazine, 4-(4-trifluoromethylphenyl)piperazine, 4-cyclohexylpiperazine, 4-ethylpiperazine, 4-hydroxy-4-(4-chlorophenyl)methylpiperidine, 4-hydroxy-4-phenylpiperidine, 4-hydroxypyrrolidine, 4-methylpiperazine, 4-phenylpiperazine, 4-piperidinylpiperazine, 4-((2-furanyl)carbonyl)piperazine, 4-((1,3-dioxolan-5-yl)methyl)piperazine, 6-fluoro-1,2,3,4-tetrahydro-2-methylquinoline, 1,4-diazacycloheptane, 2,3-dihydroindolyl, 3,3-dimethylpiperidine, 4,4-ethylenedioxypiperidine, 1,2,3,4-tetrahydroisoquinoline, 1,2,3,4-tetrahydroquinoline, azacyclooctane, decahydroquinoline, piperazine, piperidine, pyrrolidine, thiomorpholine, and triazole.

"Hydroxy-protecting group", as used herein, refers to an easily removable group which is known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf, for example, T.H. Greene and P.G.M. Wuts, Protective Groups in Organic Synthesis, 2nd edition, John Wiley & Sons, New York (1991). Examples of hydroxy-protecting groups include, but are not limited to, methylthiomethyl, *tert*-dimethylsilyl, *tert*-butyldiphenylsilyl, acyl substituted with an aromatic group.

The term "ketone protecting group", as used herein, refers to an easily removable group which is known in the art to protect a ketone group against undesirable reactions during synthetic procedures and to be selectively removable. The use of ketone-protecting groups is well known in the art for protecting groups against undesirable reactions during a synthetic procedure and many such protecting groups are known, cf, for example, T.H. Greene and P.G.M. Wuts, Protective Groups in Organic Synthesis, 2nd edition, John Wiley & Sons, New York (1991). Examples of ketone-protecting groups include, but are not limited to, ketals, oximes, O-substituted oximes for example O-benzyl oxime, O-phenylthiomethyl oxime, 1-isopropoxycyclohexyl oxime.

A the term "protected-hydroxy" refers to a hydroxy group protected with a hydroxy protecting group, as defined above, including benzoyl, acetyl, trimethylsilyl, triethylsilyl, methoxymethyl groups, for example.

The term "protogenic organic solvent" as used herein refers to a solvent that tends to provide protons, such as an alcohol, for example, methanol, ethanol, propanol, isopropanol, butanol, t-butanol, and the like. Such solvents are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of protogenic solvents may be found in organic chemistry textbooks or in specialized monographs, for example: Organic Solvents Physical Properties and Methods of Purification, 4th ed., edited by John A. Riddick *et al.,* Vol. II, in the Techniques of Chemistry Series. John Wiley & Sons, NY, 1986.

The term "substituted aryl" as used herein refers to an aryl group as defined herein substituted by independent replacement of one, two or three of the hydrogen atoms thereon with Cl. Br, F, I, OH, CN, C₁-C₃-alkyl, C₁-C₆-alkoxy, C₁-C₆-alkoxy substituted with aryl, substituted aryl, heteroaryl, or substituted heteroaryl, methoxymethoxy, amino, C₁-C₃-alkyl-amino, or (C₁-C₃-alkyl)₂-amino, acylamino; in addition, any one substitutent may be an aryl, heteroary, or heterocycloalkyl group.

The term "substituted heteroaryl" as used herein refers to a heteroaryl group as defined herein substituted by independent replacement of one, two or three of the hydrogen atoms thereon with Cl, Br. F. I. OH, CN, C₁-C₃-alkyl, C₁-C₆-alkoxy, C₁-C₆-alkoxy substituted with aryl, substituted aryl, heteroaryl, or substituted heteroaryl, methoxymethoxy, amino, C₁-C₃-alkyl-amino, or (C₁-C₃-alkyl)₂- amino, acylamino; in addition, any one substitutent may be an aryl, heteroary, or heterocycloalkyl group.

The term "substituted heterocycloalkyl" as used herein, refers to a heterocycloalkyl group, as defined above, substituted by independent replacement of one, two or three of the hydrogen atoms thereon with Cl, Br, F, I, OH, CN, C₁-C₃-alkyl, C₁-C₆-alkoxy, C₁-C₆-alkoxy substituted with aryl, substituted aryl, heteroaryl, or substituted heteroaryl, methoxymethoxy, amino, C₁-C₃-alkyl-amino, or (C₁-C₃-alkyl)₂-amino, acylamino; in addition, any one substitutent may be an aryl, heteroary, or heterocycloalkyl group.

Numerous asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereo-orientations or an individual isomer of assigned or unassigned orientation may be present.

As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al.* describe pharmaceutically acceptable salts in detail in J. Pharmaceutical Sciences, 66: 1-19 (1977).

The salts can be prepared *in situ* during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptalble salts include adipate, alginate, ascorbate, aspartate, benzenesulfonate, benzoate, bisulface, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyates, acrylates and ethylsuccinates.

The term "pharmaceutically acceptable prodrugs" as used herein refers to those prodrugs of the compounds of the present invention which are, within the scope of sound medical judgement, suitable for use in contact with with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminun hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgement of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol. 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the use of a compound of the present invention for manufacturing a medicament, bacterial infections are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of this invention per day in single or multiple doses.

### Abbreviations

Abbreviations which have been used in the descriptions of the scheme and the examples that follow are: AIBN for azobisisobutyronitrile; Bu₃SnH for tributyltin hydride; CDI for carbonyldiimidazole; DBU for 1,8-diazabicyclo[5.4.0]undec-7-ene; DEAD for diethylazodicarboxylate; DMF for dimethylformamide; DMSO for dimethylsulfoxide; DPPA for diphenylphosphoryl azide; Et₃N for triethylamine; EtOAc for ethyl acetate; Et₂O for diethyl ether; EtOH for ethanol; HOAc for acetic acid; MeOH for methanol; NaN(TMS)₂ for sodium bis(trimethylsilyl)amide; NMMO for N-methylmorpholine N-oxide; TEA for triethylamine; THF for tetrahydrofuran; and TPP for triphenylphosphine.

### Synthetic Methods

The compounds and processes of the present invention will be better understood in connection with the following synthetic schemes I-VI (to be found following the text describing the schemes) which illustrate the methods by which the compounds of the invention may be prepared. The groups X, R, R^{a}, R^{b}, R^{c}, R^{d}, R^{e}, R^{f}, are as defined above unless otherwise noted below.

Scheme I illustrates the preparation of the starting material derived from erythromycin A, a compound of formula (XII). The preparation of protected erythromycin A is described in the following United States patents, US 4,990,602; US 4,331,803, US 4,680,368, US 4,670,549 and by European Patent Application EP 260,938. In general, the 9-ketone of compound 1 is protected, for example as an oxime (V is =N-O-OR¹ or =N-O-C(R⁹)(R¹⁰)-O-R¹ where R¹, R⁹ and R¹⁰ are as defined above), and then either as a separate step or in the same pot, the 2'- and 4"-hydroxyls are protected.

The 9-ketone of compound (1) is protected to give compound (2) where V is =N-OR¹ where R¹ is as defined above or =N-O-C(R⁹)(R¹⁰)-O-R¹ where R¹, R⁹ and R¹⁰ are as defined above. In a preferred embodiment of the process, V is O-(1-isopropoxycyclohexyl) oxime.

The 2'- and 4"-hydroxy groups of erythromycin A (2) are protected by reaction with a suitable hydroxy protecting reagent, such as those described by T.W.Greene and P.G.M. Wuts in Protective Groups in Organic Synthesis, 2nd ed., John Wiley & Son, Inc., 1991, for example, acetic anhydride, benzoic anhydride, benzyl chloroformate or a trialkylsilyl chloride in an aprotic solvent. Examples of aprotic solvents are dichloromethane, chloroform, DMF, tetrahydrofuran (THF), N-methyl pyrrolidinone, dimethylsulfoxide, diethylsulfoxide, N,N-dimethylformamide, NN-dimethylacetamide, N-methyl-2-pymolidone, hexamethylphosphoric triamide, a mixture thereof or a mixture of one of these solvents with ether, tetrahydrofuran, 1,2-dimethoxyethane, acetonitrile, ethyl acetate, acetone and the like. Aprotic solvents do not adversely affect the reaction, and are preferably dichloromethane, chloroform, DMF, tetrahydrofuran (THF), N-methyl pyrrolidinone or a mixture thereof. Protection of 2'- and 4"-hydroxy groups of erythromycin A thus affords compound (3) where R^{p} is a hydroxy protecting group. In a preferred embodiment of the process, R^{p} is trimethylsilyl.

Scheme II illustrates the general preparation of the compounds of the invention derived from erythromycin A. The alkylation of the 6-hydroxy group of compound (3) can be carried out with an alkylating agent in a solvent in the presence of a base at a temperature from about -15 °C to about 50 °C to give compound (4). Alkylating agents include alkyl chlorides, bromides, iodides or alkyl sulfonates. Specific examples of alkylating agents include allyl bromide, propargyl bromide, benzyl bromide, 2-fluoroethyl bromide, 4-nitrobenzyl bromide, 4-chlorobenzyl bromide, 4-methoxybenzyl bromide, α-bromo-p-tolunitrile, cinnamyl bromide, methyl 4-bromocrotonate, crotyl bromide, 1-bromo-2-pentene, 3-bromo-1-propenyl phenyl sulfone, 3-bromo-1-trimethylsilyl-1-propyne, 3-broino-2-octyne, 1-bromo-2-butyne, 2-picolyl chloride, 3-picolyl chloride, 4-picolyl chloride, 4-bromomethyl quinoline, bromoacetonitrile, epichlorohydrin, bromofluoromethane, bromonitromethane, methyl bromoacetate, methoxymethyl chloride, bromoacetamide, 2-bromoacetophenone, 1-bromo-2-butanone, bromo chloromethane, bromomethyl phenyl sulfone, 1,3-dibromo-1-propene, and the like. Examples of alkyl sulfonates are: allyl O-tosylate, 3-phenylpropyl-O-trifluoromethane sulfonate, n-butyl -O-methanesulfonate and the like. It is sufficient to use 1 to 4 mole equivalents of alkylating agents relative to compound (3). Examples of the solvents used are aprotic solvents such as dimethylsulfoxide, diethylsulfoxide, N,N-dimethylformamide, N,N-dimethylacetamide, N-methyl-2-pyrrolidone, hexamethylphosphoric triamide, a mixture thereof or a mixture of one of these solvents with ether, tetrahydrofuran, 1,2-dimethoxyethane, acetonitrile, ethyl acetate, acetone and the like. Examples of the base which can be used include potassium hydroxide, cesium hydroxide, tetraalkylammonium hydroxide, sodium hydride, potassium hydride, potassium isopropoxide, potassium tert-butoxide, potassium isobutoxide and the like. The amount of base used is usually 1 to 4 equivalents relative to compound (3).

The deprotection of the 2'- and 4'-hydroxyl groups is carried out according to methods described in literature, for example, by T.W.Greene and P.G.M. Wuts in Protective Groups in Organic Synthesis, 2nd ed., John Wiley & Son, Inc., 1991. The conditions used for the deprotection of the 2'- and 4'-hydroxyl groups usually results in the conversion of X to =N-OH. (For example, using acetic acid in acetonitrile and water results in the deprotection of the 2'- and 4'-hydroxyl groups and the conversion of X from =N-O-OR¹ or =N-O-C(R⁹)(R¹⁰)-O-R¹ where R¹, R⁹ and R¹⁰ are as defined above to =N-OH.) If this is not the case, the conversion is carried out in a separate step.

The deoximation reaction can be carried out according to the methods described in the literature, for example by Greene and Wuts (*op. cit.*) and others. Examples of the deoximating agent are inorganic sulfur oxide compounds such as sodium hydrogen sulfite, sodium pyrosulfate, sodium thiosulfate, sodium sulfate, sodium sulfite, sodium hydrosulfite, sodium metabisulfite, sodium dithionate, potassium thiosulfate, potassium metabisulfite and the like. Deoximation may also be accomplished by treatment with an inorganic nitrite salt, for example sodium nitrite or potassium nitrite, in the presence of acid. Examples of the solvents used are protic solvents such as water, methanol, ethanol, propanol, isopropanol, trimethylsilanol or a mixture of one or more of the mentioned solvents and the like. The deoximation reaction is more conveniently carried out in the presence of an organic acid such as formic acid, acetic acid and trifluoroacetic acid, but may be accomplished with hydrochloric acid also. The amount of acid used is from about I to about 10 equivalents of the amount of compound 5 used. In a preferred embodiment, the deoximination is carried out using an organic acid such as formic acid in ethanol and water to give the desired product (6).

The desired 6-O-"alkylated" compound may be prepared directly as described above or obtained from chemical modification of an initially prepared 6-O-"alkylated" compound. Representative examples of further elaboration of the 6-position are shown in Scheme III and Scheme IV. For example, compound (6A), which is a compound of formula (6)
wherein R is -CH₂CH=CH₂ (prepared with allyl bromide as the alkylating reagent) and
wherein M represents the macrolide ring system, can be further derivatized. The double bond of the allyl compound can be (a) reduced to give the 6-O-propyl compound (7); (b) treated with osmium tetroxide to give the 2,3-dihydroxypropyl compound (8); (c) oxidized with 3-chloroperoxybenzoic acid to give the epoxy methyl compound (9), which can be opened with nucleophiles such as amines or N-containing heterocyclic compounds, for example, to give compounds with N-containing side chains (10); (d) oxidized under Wacker conditions (*cf.,* Tsuji, *in* "Organic Synthesis with Palladium Compounds", NY, Springer-Verlan, **1980**, pp. 6-12) to give the 6-O-CH₂-C(O)-CH₃ compound (11); (e) brominated with hydrogen bromide perbromide to give (12); (f) reacted with aryl halides under Heck conditions (R.F. Heck, Org. React., 1982, 27, 345-390) to give (13); (g) oxidized with ozone to give the 6-O-CH₂-CHO compound (14) which can in turn be (i) converted to oxime (17) by reaction with H₂NOR', (ii) converted to hydrazone (15) by reaction with H₂NNR'R", and (iii) reductively aminated with primary amines, H₂NR', in the presence of NaCNBH₃ to give (16). Reaction of oxime (17), where R'=H, with diisopropylcarbodiimide in the presence of CuCl gives nitrile (18).

In Scheme IV, the propargylic compound (19), which is a compound of formula (6) of Scheme II wherein R is CH₂CCH (prepared with propargyl bromide as the alkylating reagent) can also be further derivatized. The triple bond can be coupled to aryl halides using Pd (II) or Pd(0) catalysts in amine solvents in the presence of co-catalytic CuI (Sonogashira *et al., Tetrahedron Lett.,* **1975**, 50, 4467-4470.) to give the aryl substituted alkyne compound (20); brominated with N-bromosuccinimide in the presence of silver nitrate (Weichert, R., *Angew. Chem. Int. Ed. Engl.,* 1984, 23, 727-728) to provide the brominated alkyne (25) ; hydroborated with 9-BBN to give the boronated compound which is then treated with an aryl or alkenyl halide or sulfonate in the presence of Pd(0) catalysts according to the method of Suzuki (*Pure Appl. Chem.,* **1985,** 57, 1749-1758) to give the aryl sustituted olefin or a conjugated diene compound (22); coupled to terminal alkynes in the presence of cupric salts under conditions of the Eglinton Reaction (Eglinton and McCrae, *Adv. Org. Chem.* 4, 225-328, 1963.) to generate diynes (24); or coupled with acyl halides utilizing cocatalytic Pd(II)/Cu(I) in amine solvents (Sonogashira, K.; Hagihara, N.; Tohda, Y. *Synthesis,* 1977, 777-778.) to give the alkynyl ketone (23). Compound (20) can be selectively reduced to the corresponding cis olefin (21) by catalytic hydrogenation in EtOH at atmospheric pressure in the presence of 5% Pd/BaSO₄ and quinoline.

It is understood that the foregoing chemistry is merely illustrative and is not to be taken as a limitation upon the scope of the invention. Various changes and modifications to the disclosed embodiments will be apparent to those skilled in the art, and may be made without departing from the spirit and scope thereof. The foregoing chemistry is directed primarily to the preparation of compounds of formula II.

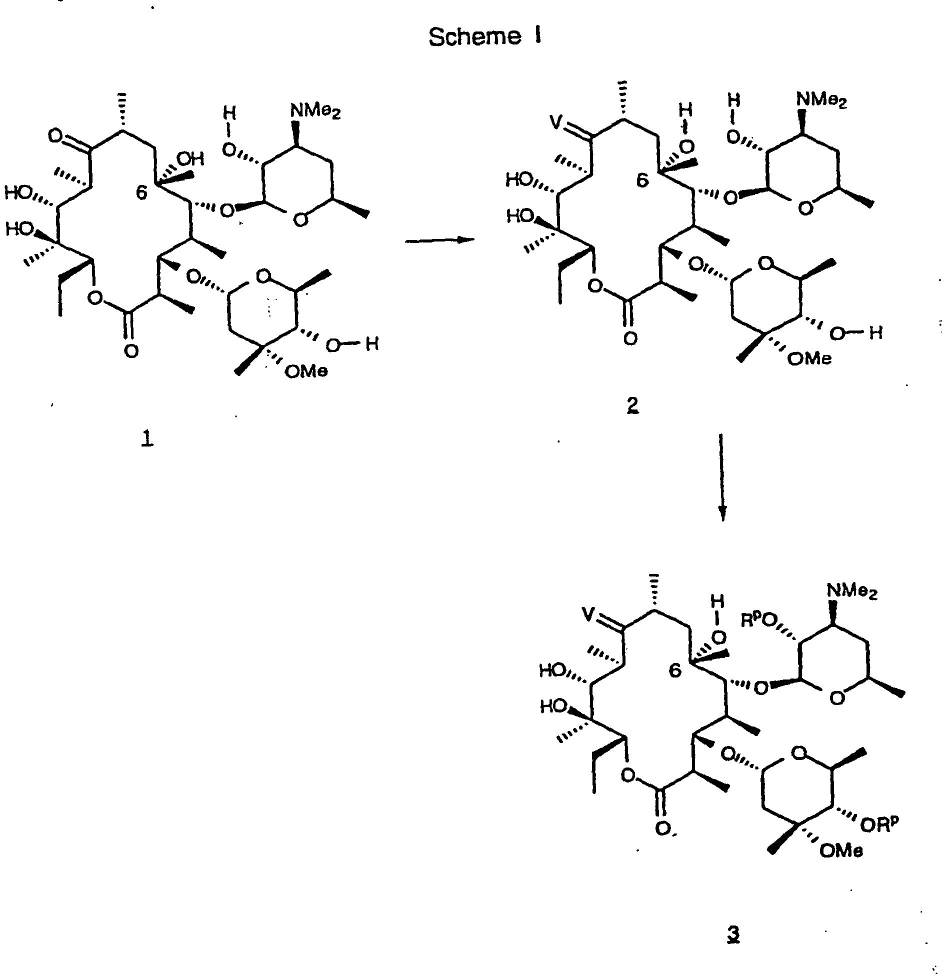

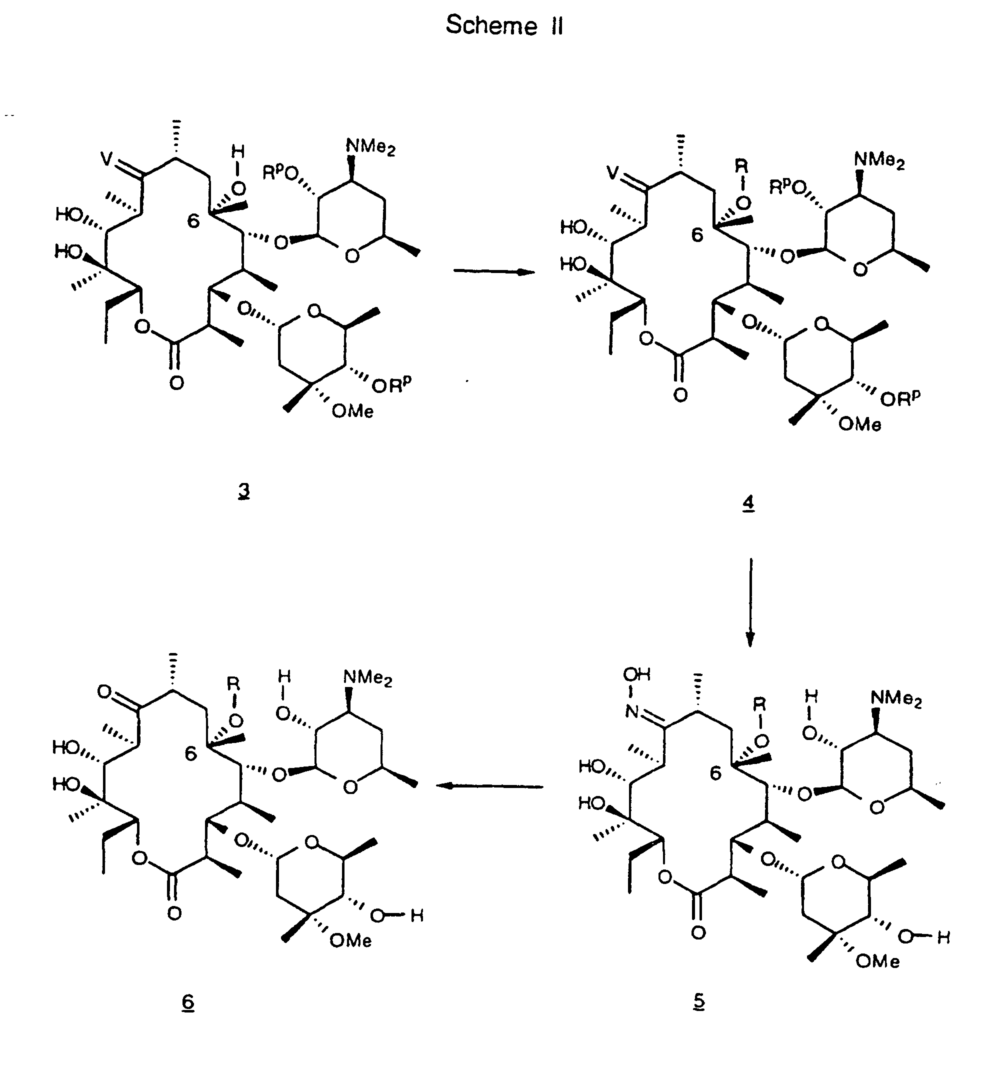

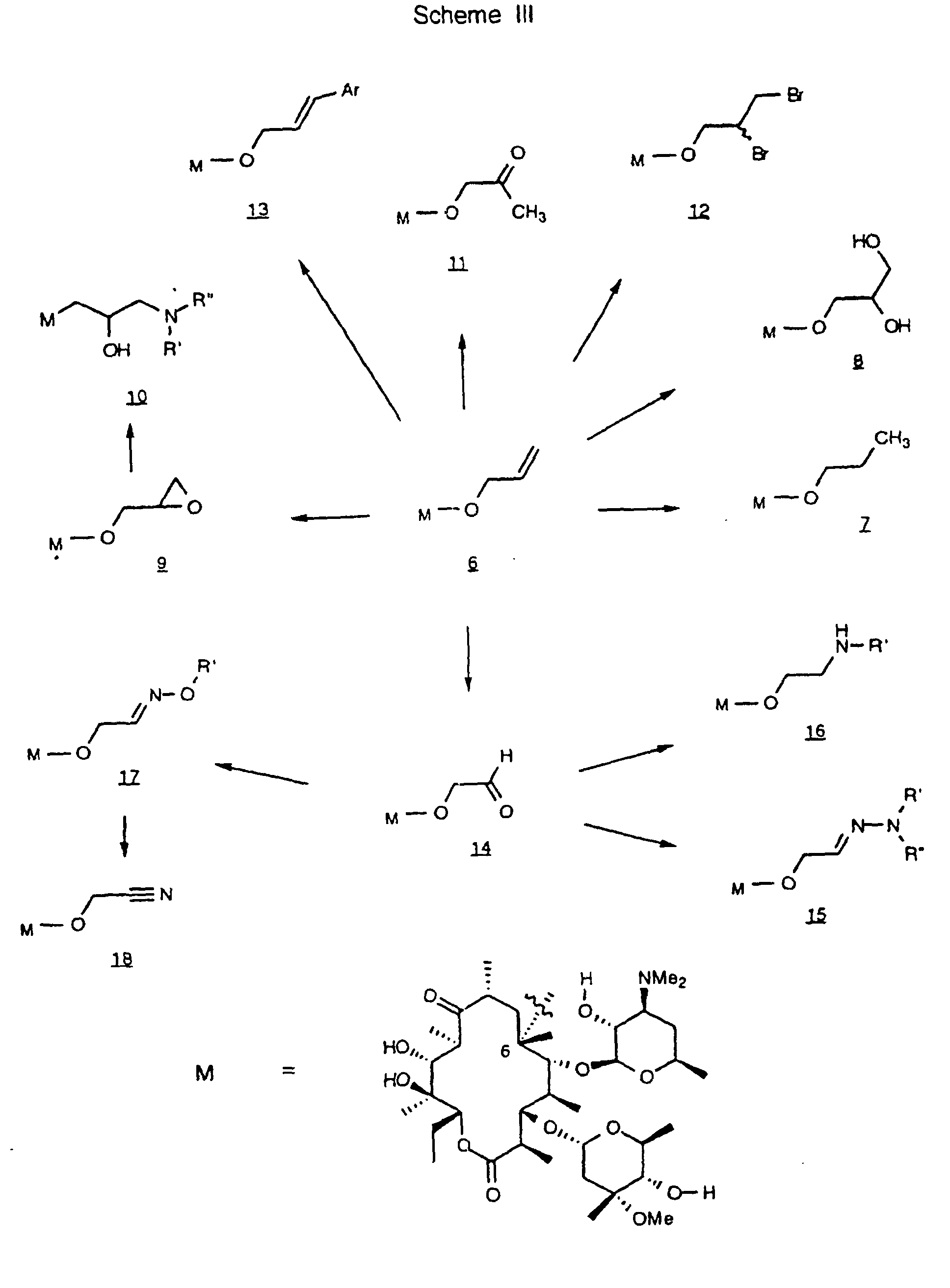

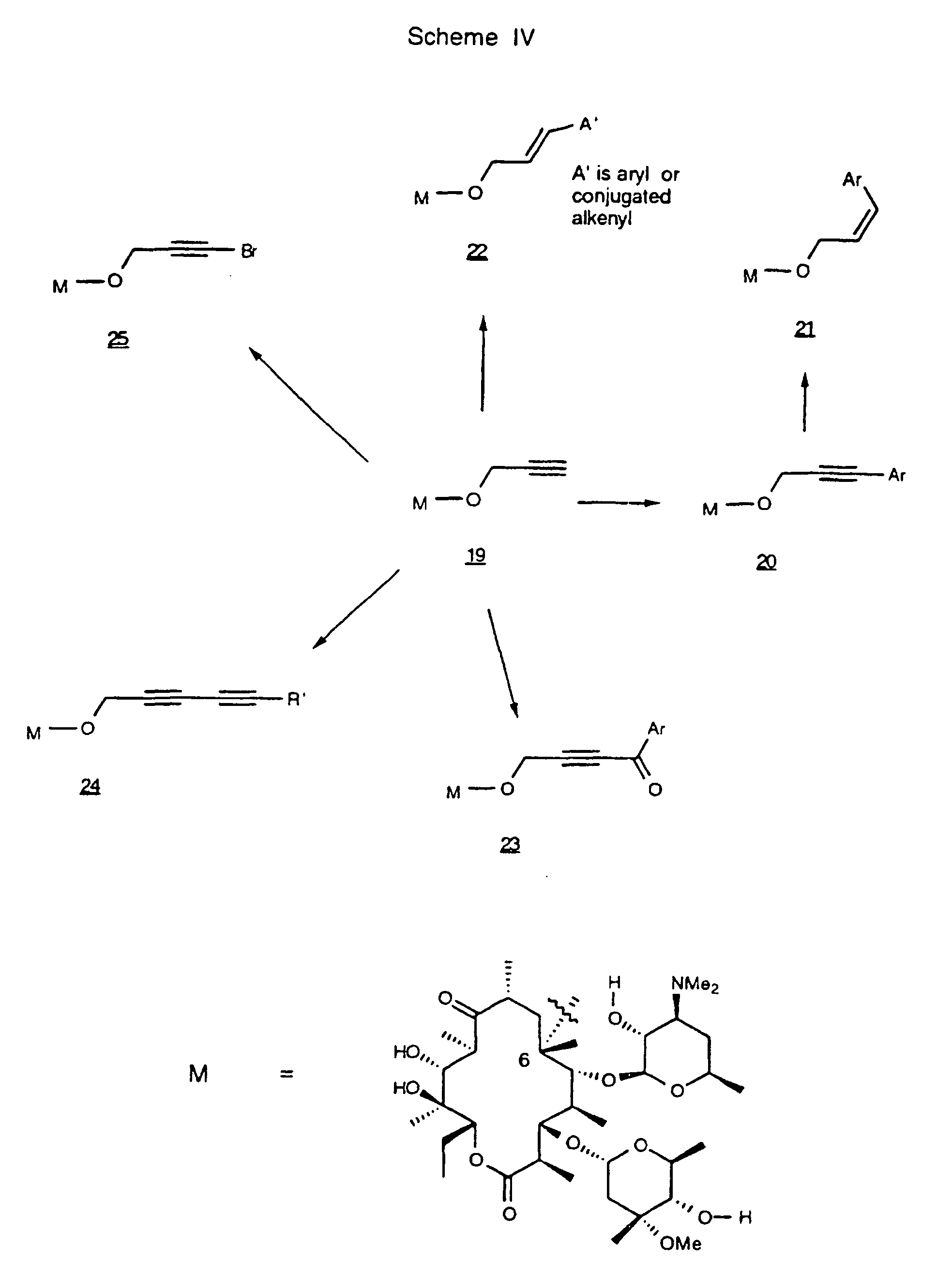

The compounds and processes of the present invention will be better understood in connection with the following examples, which are intended as an illustration of and not a limitation upon the scope of the invention.

### Example 1

### Compound of Formula (X): X is =O, R is allyl

### Example 1A

### Compound of Formula (XII): X is =N-O-(1-isopropoxycyclohexyl), R is allyl, R^{p} is Trimethylsilyl

To a 0 °C solution of 5 g of the compound of Formula XII where X is =N-O-(1-isopropoxycyclohexyl) and R^{p} is trimethylsilyl in 15 mL of DMSO and 20 mL of THF was added 1.23 mL of freshly distilled allyl bromide. After approximately 10 minutes, a solution prepared by warming and stirring (556 mg. 2.05 equivalents) of powdered KOH in 25 mL of 1:1 THF-DMSO at 50 °C for 20-30 minutes was added dropwise over 5 minutes. After about 1 hour, the chilled reaction mixture was treated with 200 mL of EtOAc followed by 762 µL of allyl amine followed by 60 mL of water. The organic layer was washed with water followed by brine, dried over MgSO₄, filtered and concentrated *in vacuo* to afford 5.3 g of crude title compound. Purification by silica gel chromatography eluting with 5% acetone in hexanes containing 0.25% triethylamine afforded 2.35 g (45%) of the title compound.

### Example 1B

### Alternate preparation of Compound of Formula (XII): X is-=N-O-(1-isopropoxycyclohexyl), R is allyl, R^{p} is trimethylsilyl

To a solution of 103.2 g (0.100 mmol) of the compound of Formula XII where X is =N-O-(1-isopropoxycyclohexyl) and R^{p} is trimethylsilyl in 500 mL of DMSO and 500 mL of THF cooled to 0 °C and flushed with nitrogen was added freshly distilled allyl bromide (17.3 mL) over 10 minutes. A solution of potassium t-butoxide (120 mL 1M THF solution, 0.120 mmol) in THF (100 mL) and DMSO (230 mL) was added dropwise over 3.5 hours at 0 °C, and the mixture was stirred at 0 °C under nitrogen for 2 hours. Additional potassium t-butoxide solution was added (50 mL 1M THF solution, 0.05 mmol, in 50 mL of DMSO) dropwise over 1 hour, and the mixture was stirred for 2 hours. The mixture was cooled and taken up in ethyl acetate (1.5 L). This solution was washed with water and brine, then dried over Na₂SO₄. Removal of the solvent under vacuum gave the title compound (125.1 g).

### Example 1C

### Compound of Formula (X): X is =N-OH, R is allyl

To a solution of the compound resulting from Example 1A (1.7 g) in 17 mL of acetonitrile and 8.5 mL of water was added 9 mL of HOAc at ambient temperature. After several hours at ambient temperature, the reaction mixture was diluted with 200 mL of toluene and concentrated *in vacuo.* The residue obtained was found to contain unreacted starting material, so additional acetonitrile (15 mL), water (70 mL) and HOAc (2 mL) was added. After 2 hours, an additional 1 mL of HOAc was added. After approximately three more hours, the reaction mixture was placed in the freezer overnight. The reaction mixture was allowed to warm to ambient temperature, diluted with 200 mL of toluene and concentrated *in vacuo.* The residue was chased two times with toluene and dried to constant weight (1.524 g).

### Example 1D

### Compound of Formula (X): X is =O. R is allyl

The compound resulting from Example 1C (1.225 g) in 16 mL of 1:1 EtOH-water was treated with NaHSO₃ (700 mg) and formic acid (141 µL) and warmed at 86 °C for 2.5 hours. After approximately three hours, the reaction mixture was allowed to cool to ambient temperature, diluted with 5-6 mL of water, basified with 1 N NaOH to pH 9-10 and extracted with EtOAc. The combined organic extracts were washed with brine (2x), dried over MgSO4, filtered and concentrated *in vacuo.* The crude material was purified by column chromatography eluting with 1% MeOH in dichloromethane containing 1% ammonium hydroxide to give 686 mp (57%) of the title compound. ¹³C NMR (CDCl₃) δ 219.3 (C-9), 174.8 (C-1), 135.5, 116.3, 101.9 (C-1'), 95.9 (C-1"). 79.7 (C-5), 78.8 (C-6), 78.5 (C-3), 74.1 (C-12), 72.4 (C-3"), 70.6 (C-11), 68.1 (C-5'), 65.5 (allylic methylene), 65.1 (C2'), 49.0 (C-3" O-CH₃), 45.0 (C-2), 44.1 (C-8), 39.7 (NMe₂), 37.9 (C-4), 37.1 (C-10), 34.6 (C-2"), 28.4 (C-4'), 21.0. 20.6 (C-3" CH₃, C-6' CH₃), 20.8 (C-14), 18.3 (C-6"), 18.1 (C-8 CH₃), 15.7, 15.6 (C-2 CH₃, C-6 CH₃), 11.9 (C-10 CH₃), 10.1 (C-15), 8.9 (C-4 CH₃). MS (FAB)+ m/e 774 (M+H)⁺, 812 (M+K)+.

### Example 1E

### Alternate preparation of Compound of Formula (X): X is =O, R is allyl

A sample of the compound prepared according to Example 1B (76.2 g) was dissolved in H₂O (120 mL) and EtOH (120 mL), then pellets of NaNO₂ (33.5 g, 0.485 mol, 5 equiv) were added to the solution and the reaction mixture was stirred until the NaNO₂ was dissolved. Hydrochloric acid (4N, 121 mL, 0.484 mol) was then added dropwise over a 10 minutes with rapid stirring. The reaction mixture was then heated to 70 °C and stirred for 2 hours. The reaction mixture was cooled, and solid NaHCO₃ was added slowly until the solution was saturated. The mixture was then concentrated to approximately half its volume under reduced pressure and extracted with ethyl acetate (3 ×). The organic extract was washed with brine and dried with Na₂SO₄. The solvent was removed under reduced pressure, and the residue was crystallized from acetonitrile to give 29.2 g of desired product. A second crop of crystals gave an additional 7.02 g of material. Analytical data was as in Example 1D above.

### Example 2: Comparative example

### Compound of Formula (X): X is =O, R is propyl

The compound resulting from Example 1 (100 mg) was catalytically hydrogenated in MeOH (10 mL) using a palladium on carbon catalyst and hydrogen. The catalyst was removed by filtration, and the filtrate was concentrated *in vacuo.* The crude product (93 mg) was purified by column chromatography on silica gel eluting with 1% MeOH in dichloromethane containing 1% ammonium hydroxide to afford 38 mg (38%) of the title compound. ¹³C NMR (CDCl₃) δ 220.5 (C-9), 175.1 (C-1), 102.2 (C-1'), 96.4 (C-1"), 79.8 (C-5).79.0 (C-3), 78.7 (C-6), 77.8 (C-4"), 76.4 (C-13), 74.4 (C-12), 72.8 (C-3"), 71.1 (C-11), 68.8, 68.5, 66.0, 65.9, 65.6, 49.4 (C-3" -OMe), 45.3 (C-2), 44.5, 40.2 (-NMe₂), 38.4 (C-7), 38.2 (C-4), 37.4 (C-10), 35.1, 28.6, 21.8, 21.5 (C-14), 21.3, 21.0 (C-3" Me, 6 Me), (18.7, 18.6 (C-6" Me, 8 Me)), (16.2, 16.1 (C-2 Me, 6 Me)), 12.3 (C-10 Me), 10.5 (C-15), 10.1, 9.4 (C-4 Me). MS (DCI/NH₃) m/z 776 (M+H)⁺.

### Example 3

### Compound of Formula (X): X is =O, R is 2.3-dihydroxypropyl

To an ambient temperature solution of the compound resulting from Example 1 (100 mg) in 6 mL of THF was added N-methylmorpholine N-oxide (98 mg) followed by 32 µL of 4% by weight osmium tetroxide in water. The reaction mixture was stirred overnight and then quenched by the addition of 3 equivalents of NaHSO₃. After stirring at ambient temperature for 10 minutes, the reaction mixture was filtered through a silica gel plug eluting with 5% MeOH in dichloromethane containing 1% ammonium hydroxide to afford, after concentration at reduced pressure, the title compound (81 mg, 77%) as a mixture of epimers. ¹³C NMR (CDCl₃) δ 222.6, 221.6 (C-9), 176.9, 176.0 (C-1), 102.2 (C-1'), 102.1, 96.5 (C-1"), 96.4, 80.0, 79.9, 79.8, 78.8. 78.7, 77.6, 77.5, 77.2, 77.0, 76.7, 74.8, 74.7 (C-12), 72.8 (C-3''), 71.0, 71.0, 70.9 (C-11), 70.9, 68.9, 68.9, 68.5, 66.8, 66.5, 66.3, 66.2, 65.8, 65.6, 63.3, 63.0 (C-18), 55.3, 49.3 (-OCH₃), 45.6, 45.4 (C-2), 44.7 (C-8), 40.2 (-NMe₂), 38.4, 38.2, 38.2, 37.9, 37.6, 35.1 (C-2"), 35.0, 28.5, 28.5 (C-4'), 21.7, 21.5, 21.5, 21.4, 21.0, 20.9, 18.8, 18.6, 18.5, 16.2, 16.2, 16.0, 11.9 (C-10 CH₃), 10.4 (C-15), 10.4, 9.4 (C-4 CH₃), 9.3. MS m/z 808 (M+H)⁺.

### Example 4

### Compound of Formula (X): X is =O, R is 2,3-epoxypropyl

To an ambient temperature solution of the compound resulting from Example 1 (100 mg) in 1.5 mL of dichloromethane was added ∼170 mg of m-chloroperoxybenzoic acid. The reaction mixture was stirred at ambient temperature overnight and concentrated *in vacuo.* The residue obtained was taken up in EtOAc and washed with saturated sodium bicarbonate solution (2x) followed by brine, dried over MgSO₄ and concentrated *in vacuo* to afford 93 mg of crude product. The crude product was redissolved in EtOAc and washed with 1 M NaHSO₃ followed by NaHCO₃ solution and brine, dried over MgSO₄ and concentrated *in vacuo.* The residue obtained was chromatographed on silica gel eluting with 5% MeOH in dichloromethane containing 1% NH₄OH to afford the title compound as a mixture of epimers. ¹³C NMR (CDCl₃) δ 219.8, 219.0 (C-9), 175.5, 175.2 (C-1), 102.2, 102.2 (C-1'). 96.3, 96.2 (C-1"), 80.2, 79.9, 79.6, 79.0, 78.8, 77.8, 77.7, 76.6 (C-6,5,3,4",13)). 74.6 (C-12), 72.7 (C-3"), 71.0 (C-11), 68.8, 68.8, 68.5 (C-5'). (66.2, 66.0 (C-16)), (66.1, 65.6 (C-5",3')), 50.3, 49.8, 49.3 (3" OMe), 46.6, 45.5, (45.3, 45.2 (C-2)), 44.6, 44.6 (C-8), 40.2 (-NMe₂), 38.4 (C-7), 38.2, 38.2, (37.6, 37.5 (C-10)), 35.1 (C-2"), 35.0, 28.8 (C-4'), (21.4, 21.2, 21.1, 20.9 (C-3" Me, 6' Me, C-14), (18.7, 18.6, 18.5 (C-6" Me, 8 Me)), (16.1, 16.0, 15.9 (C-2 Me, 6 Me)), 12.2 (C-10 Me), 12.2, 10.5 (C-15). 9.3 (C-4 Me), 9.2. MS (FAB) m/e 790 (M+H)⁺. 812 (M+Na)⁺, 828 (M+K)⁺. High Resolution Mass Spec m/z Calcd for C₄₀H₇₁NO₁₄K: 828.4512. Found: 828.4516.

### Example 5

### Compound of Formula (X): X is =O, R is 2-hydroxy-3-(imidazol-1-yl)propyl

To an ambient temperature solution of the compound resulting from Example 4 (100 mg) in ~1 mL of chloroform was added 17 mg of imidazole. The reaction vessel was sealed and stirring was continued at ambient temperature for 1 hour. Two additional equivalents of imidazole were added, and stirring was continued for several days. The solvent was removed under reduced pressure, and the crude material obtained was purified by column chromatography eluting with 5% MeOH in dichloromethane containing 1% NH₄OH to afford 44 mg (41%) of the title compound. ¹³C NMR (CDCl₃) mixture of epimers δ 223.3, 221.2 (C-9), 176.4, 175.9 (C-1), 137.9, 128.7, 120.1, 119.9, 102.2, 102.1 (C-1'), 96.5 (C-1"), 80.3, 80.0, 79.7, 79.2, 78.9, 77.6, 77.6, 77.4, 77.2, 77.0, 77.0, 76.7, 74.7, 74.6, 72.7, 72.7, 71.0, 69.7, 69.4, 69.0, 69.0, 68.5. 68.5, 66.8 (C-16), 66.0, 65.5, 65.4, 50.2, 49.3 (C-18), 49.3 (C-3" -OMe), 45.5 (C-2), 45.2 (C-2), 44.7 (C-8), 40.1 (-NMe₂), 38.2 (C-7), 38.1 (C-7). 37.8, 37.5, 35.0, 34.9 (C-2"), 28.4 (C-4'), 21.6, 21.4, 21.3, 21.3, 21.2, 20.9, 20.8, 18.9, 18.8, 18.7, 18.6, 16.2, 16.1 16.0 (C-2 Me, C-6 Me), 11.9 (C-10 Me), 10.4 (C-15), 9.3 (C-4 Me). MS m/z 858 (M+H)⁺. High Resolution Mass Spec m/z Calcd for C₄₃H₇₆N₃O₁₄: 858.5327. Found: 858.5320.

### Example 6

### Comnound of Formula (X): X is =O, R is 2-hydroxy-3-(morpholin-4-yl)propyl

To an ambient temperature solution of the compound resulting from Example 4 (100 mg) in 1 mL of chloroform was added 22 µL of morpholine. The reaction vessel was sealed and stirring was continued at ambient temperature for 1 hour. Two additional equivalents of morpholine was added and stirring was continued for several days. The solvent was removed under reduced pressure, and the crude material obtained was chromatographed on silica gel eluting with 3% MeOH in dichloromethane containing 1% NH₄OH to give 35 mg (33%) of the title compound. ¹³C NMR (CDCI₃) mixture of epimers δ 220.3, 219.1 (C-9), 176.1. 175.5 (C-1), 102.1, 102.1 (C-1'), 96.2, 96.1 (C-1"), 80.0, 79.8, 79.7, 79.1, 78.6, 78.5. 77.8, 77.7, 77.1, 76.6, 75.0, 75.0, 72.8, 71.0, 68.5, 68.2. 67.8, 67.0, 66.9, 66.4, 66.0, 65.9, 65.5. 65.5, 61.4, 60.8, 53.9, 53.8, 49.3, 49.3 (-OMe), 45.5, 45.4 (C-2), 44.8, 44.7 (C-8), 40.2 (-NMe₂), 38.3 (C-10,4), 38.2, 38.1, 37.9. 37.7 (C-7), 35.0. 28.5 (C-4'), 21.7, 21.5, 21.4, 21.4, 21.3, 21.3 (C-14, 3"-Me, 6'-Me), 21.1, 19.0, 18.7, 18.6, (C-8 Me, C-6" Me), 16.3, 16.2, 16.0 (C-2 Me, 6 Me), 12.1, 12.0 (C-10 Me), 10.6, 10.5 (C-15), 9.3 (C-4 Me). MS (FAB) m/e 877 (M+H)⁺, 915 (M+K)⁺.

### Example 7

### Compound of Formula (X): X is =O, R is 2-hydroxy-3-(benzylamino)propyl

To an ambient temperature solution of the compound resulting from Example 4 (140 mg) in 1.5 mL of chloroform was added 3 equivalents (58 µL) of benzylamine. The reaction mixture was stirred overnight at ambient temperature and then warmed at 62 °C for approximately 3 hours and then stirred overnight at ambient temperature. The reaction mixture was then heated at 70 °C for 2 hours and then concentrated *in vacuo.* The residue was chased two times with toluene to afford 170 mg of crude title compound. Crude product was purified by silica gel chromatography eluting with 2% MeOH in dichloromethane containing 1% NH₄OH to give the title compound as a mixture of epimers. 13^{C} NMR (CDCl₃) δ 221.7, 220.1 (C-9), 176.4 (C-1), 175.7, 140.7. 128.5, 128.2, 128.1 , 128.1, 126.7, 126.6, 126.5, 102.2 (C-11), 96.2 (C-1"), 79.6, 79.3, 78.8, 78.7, 77.6, 77.6, 76.8. 76.7, 74.9, 72.7, 72.7, 71.0, 69.7. 69.1, 68.7, 68.5, 67.9, 67.8 (C-16), 66.0, 65.6, 53.8. 53.6, 51.6, 51.3, 49.3 (-OCH₃), 45.5 (C-2), 45.4, 44.7, 44.6 (C-8), 40.2 (-NMe₂), 38.2, 38.2 (C-7), 38.0, 37.9 (C-10, 4), 35.0, 34.8 (C-2"), 28.5 (C-4'), 21.7, 21.4, 21.4, 21.1, 21.1, 18.9, 18.6, 18.6, 16.2, 16.2. 16.0, 15.9, 12.1, 12.0 (C-10 CH₃), 10.5 (C-15), 9.3, 9.3 (C-4 CH₃). MS m/z 897 (M+H)⁺.

### Example 8

### Compound of Formula (X): X is =O, R is 2-oxoethyl

Method A. To a solution of the compound from Example 3 (275 mg) in 6.5 mL of a 20% aqueous THFwas added 87 mg of NaIO₄. The reaction was stirred at ambient temperature for 2 hours and then 0.5 equivalents of NaIO₄ was added. After 2 additional hours, another 0.5 equivalent of NaIO₄ was added. After two more hours, the reaction mixture was filtered through a silica gel plug eluting with 4% MeOH in dichloromethane containing 1% NH₄OH to afford 195 mg (65%) of the title compound. ¹³C NMR (CDCl₃) δ 221.0 (C-9), 203.2 (CHO), 175.5 (C-1), 102.4 (C-1'), 96.3 (C-1"), 80.5. 79.8, 78.8, 77.7, 76.7, 74.5 (C-12), 72.7 (C-3"), 71.0, 70.3, 68.9, 68.7, 66.1, 65.6, 49.4 (C-3" OMe), 45.3, 44.7, 40.2 (NMe₂), 38.4 (C-4), 38.2 (C-7), 37.4 (C-10), 34.9 (C-2"), 28.5 (C-4'), (21.5, 21.4, 21.1 (C-3" Me, 6 Me)), 21.0, (18.8, 18.5 (C-8 Me, 6" Me)), 16.0 (C-2 Me), 12.2 (C-10 Me), 10.4 (C-15), 9.2 (C-4 Me). Mass Spec m/z 776 (M+H)⁺.
Method B. A solution of the compound from Example 3 (8 g) in 350 dichloromethane (8 mL) was cooled to -78°C, ozone was bubbled into it until a blue color persisted, and nitrogen was then bubbled through it until the blue color disappeared. Methyl sulfide (6 mL) was added, the solution was warmed to 0 °C and stirred for 30 minutes. The solvent was removed under vacuum, the residue was redissolved in THF (90 mL), and triphenylphosphine (7.5 g) was added. The mixture was stirred at 55 °C for 4 5 hours, then concentrated *in vacuo* and dried under high vacuum to give the crude product (16.5 g) Flash chromatography on silica gel (10:1) eluting with acetone/hexanes/triethylamine (75:25:0.35) gave of the title compound (4.9 g. 61%) . Mass Spec m/z 776 (M+H)⁺.

### Example 9

### Compound of Formula (X); X is =O. R is 2-oxopropyl

A mixture of 1.5 mL of 7:1 DMF-H₂O, 5 mg PdCl₂ and 21 mg of CuCl was stirred under an oxygen atmosphere for ∼1.33 hours. To this mixture was added a solution of the compound resulting from Example 1 (150 mg) in 1.5 mL of 7:1 DMF-H₂O dropwise over 10 minutes. The reaction mixture was warmed to 50 °C and maintained at that temperature for approximately 1 hour and at ambient temperature overnight. Additional portions of PdCl₂ (5 mg) and CuCl (21 mg) were added. The reaction mixture was warmed to 54 °C and maintained at that temperature for about 3 hours. The reaction mixture was allowed to cool to ambient temperature and then O₂ was bubbled through the reaction mixture which was stirred overnight at ambient temperature. Additional PdCl₂ (10 mg) and CuCl (42 mg) were added, and the O₂ was continued. The reaction mixture was warmed to 40 °C for about 3 hours and then stirred over the weekend at ambient temperature. The reaction mixture was diluted with EtOAc and washed two times with 30% aqueous ammonium hydroxide solution and two times with brine, dried over MgSO₄ and concentrated *in vacuo* to afford 85 mg of crude title compound. Purification by eluting through a silica gel plug with 1% MeOH in dichloromethane containing 1% ammonium hydroxide afforded 47 mg of the title compound. ¹³C NMR (CDCl₃) δ 217.9, 205.3, 175.0, 102.4, 96.5, 80.6, 79.0, 78.9, 77.7, 76.7, 75.1, 72.7, 71.0, 69.6, 68.7, 68.3, 66.1, 65.6, 49.4, 44.8, 40.2, 38.4, 37.6, 35.0, 28.5, 26.5. 21.4. 21.3, 19.2, 18.7, 16.2, 15.9, 12.2, 10.6, 9.3. High Resolution Mass Spec (FAB) Calcd for m/z (M+H)⁺ C₄₀H₇₂NO₁₂: 790.4953. Found: m/z 790.4932.

### Example 10

### Compound of Formula (X): X is =O, R is -CH₂-C≡CH

### Example 10A

### Compound of Formula (X): X is =N-O-(1-isopropoxyclohexyl), R is -CH₂-C≡CH

A solution of 1.14 g of powdered KOH in 30 mL of anhydrous DMSO and 30 mL of anhydrous freshly distilled THF was added via addition funnel to a 0 °C solution of the compound of Formula XII where X is =N-O-(1-isopropoxycyclohexyl) and R^{p} is trimethylsilyl (10 g) in 60 mL of 1:1 DMSO-THF. This was followed by a solution containing 2.38 mL of propargyl bromide (80%) in toluene added over 10-15 minutes. The reaction mixture was stirred at 0 °C for about one hour and then 2 additional equivalents of propargyl bromide was added at 0 °C. After 2 hours, 2 equivalents of powdered KOH (~ 1 g) was added at 0 °C, and the reaction mixture was placed in the refrigerator overnight. The next day an additional 4 mL of propargyl bromide was added at 0 °C. When tlc indicated that the reaction was complete, the reaction was quenched with 10 equivalents of allylamine at 0 °C and stirred for 5 minutes. The mixture was diluted with H₂O and EtOAc, and the organic layer was washed with water and brine, then dried over MgSO4 and concentrated under reduced pressure to afford 11.5 g of crude title compound. Filtration through a silica gel plug eluting with 10% acetone in hexane containing 0.25% Et₃N afforded 9.3 of purified title compound.

### Example 10B

### Compound of Formula (X): R is -CH₂-C≡H, X is =N-O-H

To the compound resulting from Example 10A (9.3 g) in 50 mL of acetonitrile and 35 mL of water was added 50 mL of HOAc. The reaction mixture was stirred at ambient temperature for 2 hours and placed in the refrigerator overnight. The reaction mixture was allowed to warm to ambient temperature, diluted with toluene and concentrated *in vacuo.* The residue obtained was used without further purification.

### Example 10C

### Compound of Formula (X): X is =O, R is -CH₂-C≡CH

The compound resulting from Example 10B (8.16 g) in 1:1 EtOH-H₂O (140 ml) was treated with 4 equivalents of NaHSO₃ and formic acid (960 µL, 2.4 equivalents) and warmed to ∼82°C. After approximately 2.5 hours, the reaction mixture was allowed to cool to ambient temperature, basified to pH 10 with 1 N NaOH solution and extracted with EtOAc. The combined organic extracts were washed, dried and concentrated *in vacuo.* The crude product obtained was chromatographed on silica gel eluting with 1% MeOH in dichloromethane containing 1% ammonium hydroxide to afford 2.9 g (40%) of the title compound which was recrystallized from acetonitrile. ¹³C NMR (CDCI₃) δ 219.7 (C-9), 175.2 (C-1), 102.6 (C-1'), 96.2 (C-1"), 80.7 (C-5), 80.3 (C-6), 78.8 (C-3), 77.9 (C-4"), 76.6, 74.5, 73.9 (C-12), 72.7, 71.0 (C-2'), 68.7 (C-5'). 65.8, 65.6, 51.8 (C-16), 49.4 (C-3' OMe), 45.2 (C-2), 44.8 (C-8), 40.2 (NMe₂), 38.6 (C-7), 38.5 (C-4), 37.5 (C-10), 35.0 (C-2"), 28.6 (C-4'), 21.5 (C-3" Me, 6' Me), 21.2 (C-14), 21.0 (C-6 Me), (18.7, 18.4 (C-2 Me. 6 Me)), 16.1, 16.0, 12.3 (C-10 Me). 10.6 (C-15). 9.2 (C-4 Me). MS (FAB) m/e 772 (M+H)⁺. 810 (M+K)⁺.

### Example 11

### Compound of Formula (X): X is =O, R is -CH₂-CHOH-CH₂-N₃

To an ambient temperature solution of the compound resulting from Example 4 (100 mg) in 0.75 mL of DMF was added 12 mg of NaN₃. The reaction mixture was stirred at ambient temperature approximately 5.5 hours and then an additional 8 mg of NaN₃ was added. The reaction mixture was stirred at ambient temperature overnight, heated at 70 °C-90 °C for 3 hours and then treated with an additional 14 mg of NaN₃. The reaction mixture was heated at 60 °C overnight. Four drops of water were added, and the reaction mixture was heated at 80 °C for 4 hours. One equivalent of ammonium chloride was added and heating was continued at 80 °C for 2 hours. The reaction mixture was allowed to cool to ambient temperature, diluted with ethyl acetate and washed with 0.5 N NaOH solution and brine, dried and concentrated *in vacuo.* The residue obtained was filtered through a silica gel plug eluting with 4% MeOH in dichloromethane containing 1 % ammonium hydroxide to give 47 mg (50%) of the title compound containing trace amounts of DMF. The DMF was removed by dissolving the compound in 1:1 EtOAc-Et₂O, washing with water followed by brine, drying over magnesium sulfate and concentrating *in vacuo* to give 45 mg of title compound which was further purified by filtering through silica gel eluting with 4% MeOH in dichloromethane containing 1% ammonium hydroxide to give the title compound. ¹³C NMR (CDCl₃) mixture of epimers δ 222.8, 221.2 (C-9), 176.6, 175.8 (C-1), (102.2, 102.1 (C-1')), (96.4, 96.3 (C-1")), 80.2, 80.1, 79.4, 78.9, 78.7, 77.6, 77.5, 77.4, 77.0, 76.6, 74.8. 74.7. 72.8, 71.0 (C-11), 70.0, 68.9, 68.6, 67.3, 66.8, 66.2. 65.6, 53.8. 53.2 (C-18), 49.3 (C-3" OMe), 45.6 (C-2), 45.4, 44.7 (C-8), 40.2 (-NMe₂), 38.4, 38.2, 37.9. 37.6, (35.1. 35.0 (C-2")), 28.5 (C-4'), 21.8, 21.5, 21.2, (21.1, 21.0 (C-14)), 20.9, 18.8, 18.2, 16.1, 12.0 (C-10 Me), 12.0, 10.4 (C-15), 9.3, 9.3 (C-4 Me). MS (FAB) m/e 833 (M+H)⁺, 871 (M+K)⁺. High Resolution Mass Spec m/z (M+H)+ Calcd for C₄₀H₇₃N₄O₁₄: 833.5123. Found: 833.5137.

### Example 12

### Compound of Formula (X): X is =O, R is -CH₂-CH=N-OH

To a solution of the compound resulting from Example 8 (600 mg) in 5 mL of MeOH was added a solution of 80 mg of hydroxylamine hydrochloride and 255 µL of N-methylmorpholine in 2 mL of MeOH. The reaction mixture was stirred at ambient temperature for 5 hours and then concentrated *in vacuo.* The residue obtained was purified by eluting from silica gel using 4% MeOH in dichloromethane containing 1% ammonium hydroxide to give the title compound as a 1:1 mixture of oxime isomers. ¹³C NMR (CDCl₃) δ 220.8, 219.9 (C-9). 175.4, 175.3 (C-1), 151.7 (CH=N), 149.2 (CH=N). 102.4. 102.3 (C-1'), 96.3 (C-1"), 80.0, 80.0. 79.9. 79.8. 78.8. 78.8. 77.7. 77.3. 77.0, 76.7. 76.5. 76.4. 74.5. 74.4 (C-12). 72.7 (C-3"), 72.7. 71.1. 68.8, 68.7. 68.5. 69.5. 66.0, 65.9, 65.4. 61.4, 58.6. 49.4, 49.3 (3" -OCH₃), 45.2. 45.2, 44.6, 40.2 (-NMe₂), (38.4, 38.3 (C-4)), 38.2 (C-7), 37.4, 37.2 (C-10), 35.0 (C-2"), 28.6 (C-4'), 21.4, 21.4, 21.2. 21.1, 21.0, 20.9. 20.5, 18.7, 18.6, (18.4, 18.4 (C-8 Me, 6" Me)), 16.1, 16.0, 16.0 (C-2 Me), 15.9, 12.2 (C-10 Me), 10.4 (C-15), 9.2 (C-4 Me). MS m/z 791 (M+H)⁺.

### Example 13

### Compound of Formula (X): X is =O, R is -CH₂-CH₂OH

To a -78 °C solution of the compound resulting from Example 8 (75 mg) in 1 mL of anhydrous THF was added 1.1 equivalents of L-Selectride dropwise over 2 minutes. The reaction mixture was stirred at -78 °C for approximately one hour and then quenched at -78 °C with an aqueous solution of tris-hydroxymethylaminomethane followed by EtOAc. The organic phase was washed twice with brine, dried over magnesium sulfate and concentrated *in vacuo.* The crude material (76 mg) was chromatographed on silica gel eluting with 3% MeOH in dichloromethane containing 1% ammonium hydroxide to afford 20 mg of the desired title compound. ¹³C NMR (CDCl₃) δ 222.1 (C-9), 175.9 (C-1), 102.1, 96.3, 83.8, 80.2, 79.6, 78.8, 77.7, 77.0, 74.8, 72.8, 71.1, 68.8, 68.5, 66.1, 66.0, 65.6, 62.0, 49.4, 45.6, 44.7, 40.2 (NMe₂), 38.6, 38.1, 37.8, 35.1 (C-2"), 28.5 (C-4'), 21.6, 21.5, 21.4, 21.0, 18.7, 18.6, 16.2, 12.1, 10.5, 9.4. MS m/e 778 (M+H)⁺.

### Example 14

### Compound of Formula (X): X is =O. R is -CH₂-CH₂NH₂

The compound resulting from Example 12 (160 mg) was subjected to catalytic hydrogenation using a Raney nickel catalyst under 4 atmospheres of hydrogen over 20 hours. The catalyst was removed by filtration, and the filtrate was concentrated under reduced pressure to afford 159 mg of crude title compound. Purification by column chromatography on silica gel eluting with 7% MeOH in dichloromethane containing 1% ammonium hydroxide afforded 87 mg (55%) of the title compound. ¹³C NMR (CDCI₃) δ 219.9 (C-9), 175.5 (C-1), 101.6 (C-1'), 96.1 (C-1"), 79.3 (C-5), 78.9 (C-3), 78.4 (C-6), 77.3 (C-4"), 76.3 (C-13), 74.8 (C-12), 72.5 (C-3"), 71.1 (C-11), (68.0, 67.7, 65.6, 64.4, 64.2 (C-2',3',5",5',16)), 48.8 (C-3" OMe), 45.0 (C-2), 44.4, 40.6, 39.6 (NMe₂), (37.9, 37.8 (C-4, 10)), 37.5 (C-7), 34.8 (C-2"), 29.0 (C-4'), 21.0. 20.9. 20.8 (C-17), 20.7, (18.4, 18.1 (C-8 Me, 6" Me), (15.9, 15.4 (C-2 Me, 6 Me), 11.4 (C-10 Me), 10.0 (C-15), 9.0 (C-4 Me). MS m/z 777 (M+H)⁺.

### Example 15

### Compound of Formula (X): X is =O, R is -CH₂-CN

To a solution of the compound resulting from Example 12 (165 mg) in 5 mL of freshly distilled THF was added 2 equivalents of diisopropylcarbodiimide (65 µL) followed by a catalytic amount of CuCl. After stirring approximately 2 hours at ambient temperature, two additional aliquots of diisopropylcarbodiimide (65 µL) were added plus additional CuCl. After 3 more hours, the reaction was complete and the solvent was removed *in vacuo* to afford the title compound. Mass Spec m/z 773 (M+H)⁺.

### Example 16

### Compound of Formula (X): X is =O, R is -CH₂-Phenyl

### Example 16A

### Compound of Formula (XII): X is =-N-O-(1-isopropoxycyclohexyl), R is -CH₂-Phenyl, R^{p} is Trimethylsilyl

To a 0 °C solution of 30 mL of a 1:1 solution of THF and DMSO containing 5 g of the compound of Formula (X) wherein X is =N-O-(1-isopropoxycyaohexyl) and R is hydrogen was added 1.2 mL of benzyl bromide. A second solution of 30 mL of 1:1 DMSO and THF containing 560 mg of powdered KOH was added in portions over 45 minutes at 0 °C with good stirring. Upon completion of the addition, stir at 0 °C under nitrogen for 1 hour and then allylamine (700 µL) and ethyl acetate were added. The solution was washed wtih water and brine (2x), dried over magnesium sulfate, filtered and concentrated *in vacuo* to afford 6 g of the title compound.

### Example 16B

### Compound of Formula (XII): X is =N-O-(1-isopropoxycyclohexyl), R is -CH₂-Phenyl, R^{p} is H

To a room temperature solution of 6 g of the compound resulting from Example 16A in 65 mL of anhydrous THF was added 14.5 mL of 1 M tetrabutylammonium fluoride. After several hours, the solvent was removed under reduced pressure and the residue was dried to constant weight. Purification by column chromatography eluting with 4% methanol in dichloromethane containing 1 % ammonium hydroxide afforded 2.8 g of the title compound.

### Example 16C

### Compound of Formula (XII): X is =N-OH, R is -CH₂-Phenyl, R^{p} is H

To the compound resulting from Example 16B (2.8 g) in 26 mL of acetonitrile was added 14 mL of water followed by 14 mL of acetic acid. The reaction mixture was stirred for ∼4 hours at ambient temperature and then placed in the freezer overnight. The volatiles were removed *in vacuo,* and the residue was chased twice with toluene and dried to constant weight to afford 2.73 g of the title compound.

### Example 16D

### Compound of Formula (X): X is =O, R is -CH₂-phenyl

To the compound resulting from Example 16C (2.7 g) in 23 mL of EtOH and water (23 mL) was added 1.4 g of NaHSO₃ This was followed by 292 µL of formic acid, and the reaction mixture was warmed to 80 °C. After approximately 90 minutes, the reaction mixture was allowed to cool to ambient temperature, basified to pH ∼10-11 with 2 N NaOH solution and extracted with ethyl acetate. The combined organic extracts were washed with water followed by brine, dried over magnesium sulfate, filtered and concentrated *in vacuo.* The crude material (1.95 g) was chromatographed on silica gel eluting with 1% methanol in dichloromethane containing 1% ammonium hydroxide followed by 2% methanol in dichloromethane containing 1% ammonium hydroxide to afford 715 mg of the title compound. Further purification was effected by chromatography on silica gel eluting with 2% ammonium hydroxide in dichloromethane followed by 2% methanol in dichloromethane containing 1% ammonium hydroxide to afford 435 mg of the title compound. ¹³C NMR (CDCl₃) δ 219.28. 174.69, 139.20, 128.51, 127.95, 127.12, 102.20, 96.42, 80.14, 79.80, 78.96, 77.79, 77.42, 77.00, 76.56, 74.77, 72.84, 71.11, 68.75, 68.56, 66.39, 66.21, 65.61, 49.41, 45.15, 44.62, 40.27, 38.02, 37.91, 35.19, 28.54, 21.95, 21.56, 21.53, 21.28, 19.2, 18.82, 16.25, 16.09, 12.24, 10.61, 9.56. MS (FAB) m/e 824 (M+H)⁺.

### Example 17

### Compound of Formula (X): X is =O. R is -CH₂-CH=CH-Phenyl-

The title compound was prepared by the procedures described in Example 16 substituting 3-phenyl allyl bromide for benzyl bromide. For conversion of the oxime to the ketone, the reaction mixture was heated about 3 hours and then placed in the freezer overnight. Chromatography on silica gel eluting with 1% methanol in dichloromethane containing 1% ammonium hydroxide afforded ∼700 mg (17% yield for three steps) of the title compound. H. Res. MS: 850.5338.

### Example 18

### Compound of Formula (X): X is =O, R is -CH₂-CH=N-O-CH₃

To a room temperature solution of 150 mg of the compound resulting from Example 8 in 1 mL of methanol was added a solution of 14 mg of methoxylamine hydrochloride in 0.5 mL methanol containing 64 µL of N-methyl morpholine in one portion. The reaction mixture was stirred at ambient temperature under a nitrogen atmosphere for about 6 hours and then treated with 0.75 equivalents (12 mg) of methoxylamine hydrochloride. The reaction mixture was stirred at ambient temperature for 1 hour and then placed in the refrigerator overnight. The volatiles were removed under reduced pressure to afford 215 mg of crude title compound. Purification by column chromatography on silica gel eluting with 1% methanol in dichloromethane containing 1% ammonium hydroxide afforded 120 mg (78%) of the title compound as a 1:1 syn:anti mixture. ¹³C NMR (COCl₃) δ 219.3, 218.4, 173.8, 173.7, 157.9, 149.5, 146.8, 100.8, 100.7, 94.7, 94.5, 93.0. 78.4, 78.2, 77.3, 77.1, 76.2, 75.9, 75.4, 75.0, 74.9, 74.8, 72.9, 72.7, 71.2, 71.1, 69.4. 67.2, 67.1, 67.0, 65.3, 64.5, 64.4, 64.0, 59.9, 57.3, 47.8, 47.8, 44.8, 43.7, 43.6, 43.0, 38.6, 36.8, 36.7, 36.7, 35.7, 33.4, 26.9, 19.9, 19.8, 19.4, 18.9, 17.1, 16.9, 16.8, 14.4, 14.4, 10.6, 8.9, 8.8, 7.6, 7.5. MS (FAB) m/e 805 (M+H)⁺.

### Example 19

### Compound of Formula (X): X is =O, R is -CH₂-CH=N-O-CH₂-Phenyl

The title compound was prepared as a 1:1 syn:anti mixture by the procedures described in Example 18 substituting O-benzyl hydroxylamine hydrochloride for methoxylamine hydrochloride to afford 118 mg (70%). ¹³C NMR (CDCl₃) δ 219.3, 218.5, 173.8, 173.7, 149.8, 147.6, 136.6, 126.5, 126.5, 126.2, 125.8, 125.8, 100.8, 100.8, 94.7, 94.5, 93.0, 78.5, 78.4, 78.2, 77.2, 76.2, 76.2, 75.8, 75.4, 75.0, 74.9, 74.2, 74.0, 72.8, 72.7, 71.2. 71.1, 69.4, 67.2, 67.1, 67.0, 65.3, 64.4, 64.4, 60.1, 57.5, 53.8, 47.8, 47.8, 44.8, 43.6, 43.0, 38.6, 36.8, 36.8, 36.7, 36.7, 35.6, 33.4, 33.4, 26.9, 19.9, 19.8, 19.8, 19.5, 19.4. 19.0. 17.1, 17.0, 16.8, 16.8, 14.4, 14.4, 10.6, 10.6, 8.9, 7.6. MS (FAB) m/e 881 (M+H)⁺.

### Example 20

### Compound of Formula (X): X is =O, R is -CH₂-CH=N-N(CH₃)₂

The title compound was prepared by the procedures described in Example 18 substituting N,N-dimethyl hydrazine for methoxylamine hydrochloride. Purification by column chromatography eluting with 2% methanol in dichloromethane containing 1% ammonium hydroxide afforded 115 mg (73%) of the title compound as a single (syn or anti) isomer. ¹³C NMR (CDCI₃) δ 217.8, 173.5, 132.4, 100.5, 94.5, 92.7, 78.1, 77.4, 76.0, 75.6, 75.2, 74.7, 74.6, 72.6, 70.9, 69.2, 66.7, 64.1, 63.7, 63.0, 47.5, 43.4, 42.7, 41.0, 38.4, 36.6, 36.5, 35.4, 33.1, 26.7, 19.6, 19.6, 19.4, 19.2, 16.8, 16.6, 14.3, 14.0, 10.4, 8.8, 7.4. MS (FAB) m/e 818 (M+H)⁺.

### Example 21

### Compound of Formula (X): X is =O, R is -CH₂-CH=N-NH(CH₃)

The title compound was prepared by the procedures described in Example 18 substituting N-methyl hydrazine for methoxylamine hydrochloride. Purification by column chromatography eluting with 2% methanol in dichloromethane containing 1% ammonium hydroxide afforded 89 mg (58%) of the title compound as a single pure isomer of unknown stereochemistry. ¹³C NMR (CDCI₃) δ 219.7, 175.5, 136.2, 102.5. 96.5, 80.1, 79.4, 79.1, 77.9, 77.2, 77.0, 76.7, 76.5, 74.5, 72.8, 71.1, 68.7, 66.1, 65.7, 64.4, 49.4, 45.3, 44.6, 40.3, 38.6, 38.5, 37.4, 35.1, 34.8, 28.6, 21.5, 21.5, 21.3, 21.0, 18.8, 18.5, 16.2, 15.9, 12.3, 10.7, 9.2. MS (FAB) m/e 804 (M+H)⁺.

### Example 22

### Compound of Formula (X): X is =O, R is -CH₂-CH=N-(4-Morpholinyl)

The title compound was prepared by the procedures described in Example 18 substituting N-amino morpholine for methoxylamine hydrochloride. Purification by column chromatography eluting with 5% methanol in dichloromethane containing 1% ammonium hydroxide followed by re-chromatography eluting with 2% methanol in dichloromethane containing 1% ammonium hydroxide afforded 125 mg (75%) of the title compound as a single pure isomer of unknown stereochemistry. Diagnostic peaks ¹H NMR (CDCl₃, 500 MHz) δ 0.84 (t, 3H), 2.29 (s, 6H), 3.12 (m, 4H), 3.34 (s, 3H), 3.85 (t, 4H), 4.50 (d. 1H), 4.92 (d, 1H), 5.06 (d of d, 1H). MS (FAB) m/e 860 (M+H)⁺.

### Example 23

### Compound of Formula (X): X is =O, R is -CH₂-CH-N-NH(Phenyl)

The title compound was prepared by the procedures described in Example 18 substituting N-phenyl hydrazine for methoxylamine hydrochloride. Purification by column chromatography eluting with 1% methanol in dichloromethane containing 1% ammonium hydroxide afforded 50 mg of the title compound as a single pure isomer of unknown stereochemistry. ¹MS (FAB) m/e 866 (M+H)⁺.

### Example 24

### Compound of Formula (X): X is =O, R is -CH₂-CH=N-N(Phenyl)₂

The title compound was prepared by the procedures described in Example 18 substituting N,N-diphenyl hydrazine for methoxylamine hydrochloride. Purification by column chromatography eluting with 2% methanol in dichloromethane containing 1% ammonium hydroxide afforded 156 mg of the title compound as a single pure isomer of unknown stereochemistry. Diagnostic peaks ¹H NMR (CDCl₃, 300 MHz) δ 7.07-7.39 (m, 10H), (m, 6.46 (t, 1H). MS (FAB) m/e 942 (M+H)⁺.

### Examples 26-105 as comparative examples

### Compounds having Formula (X) wherein X is O, R is CH₂-CH(OH)-CH₂-R^{V}, and R^{V} is as shown

In accordance with the procedures of Examples 5, 6, 7 or 210, except replacing the reagent amines from those examples with the appropriate amine, the compounds of Examples 26-105, which are compounds having Formula (X) wherein X is O, R is CH₂-CH(OH)-CH₂-R^{V}, and R^{V} is as shown in Table 1 below, were prepared.

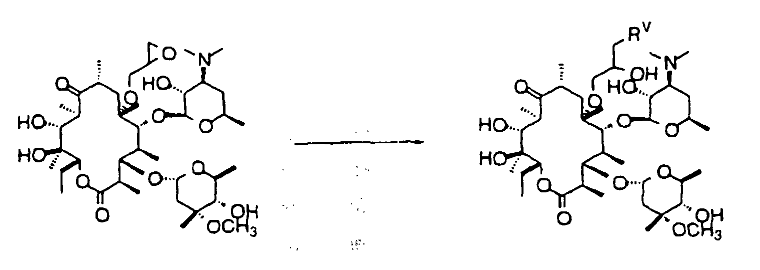

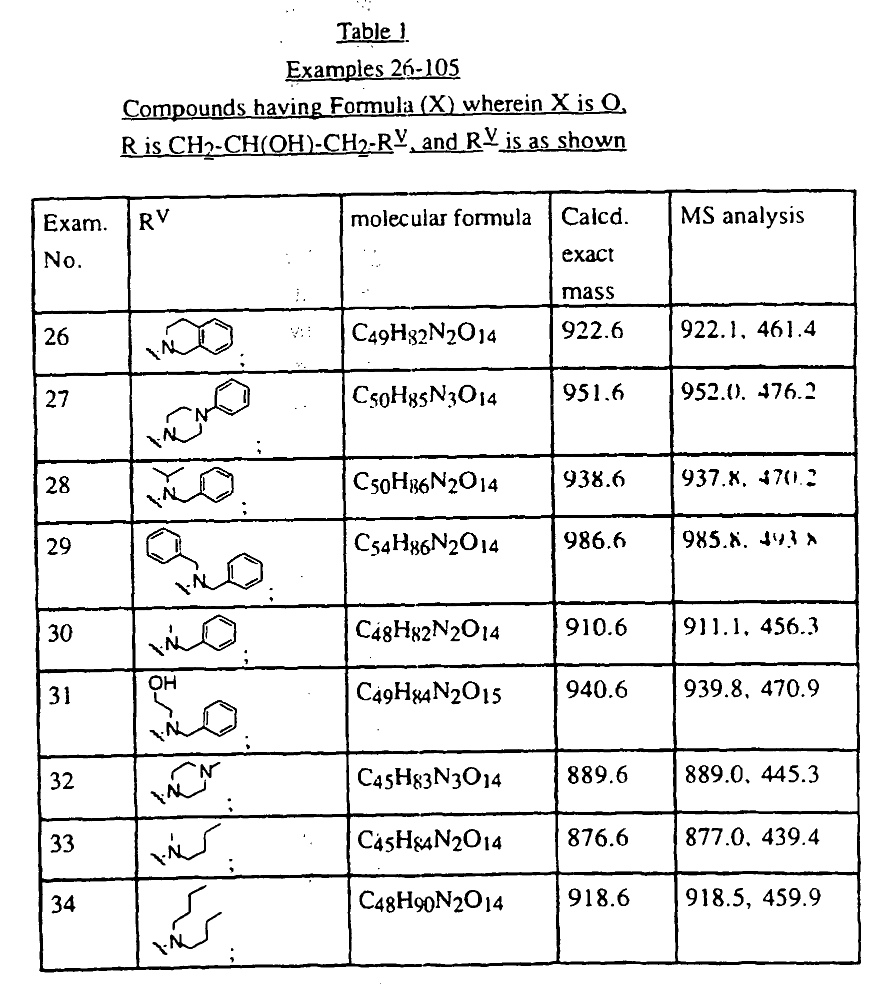

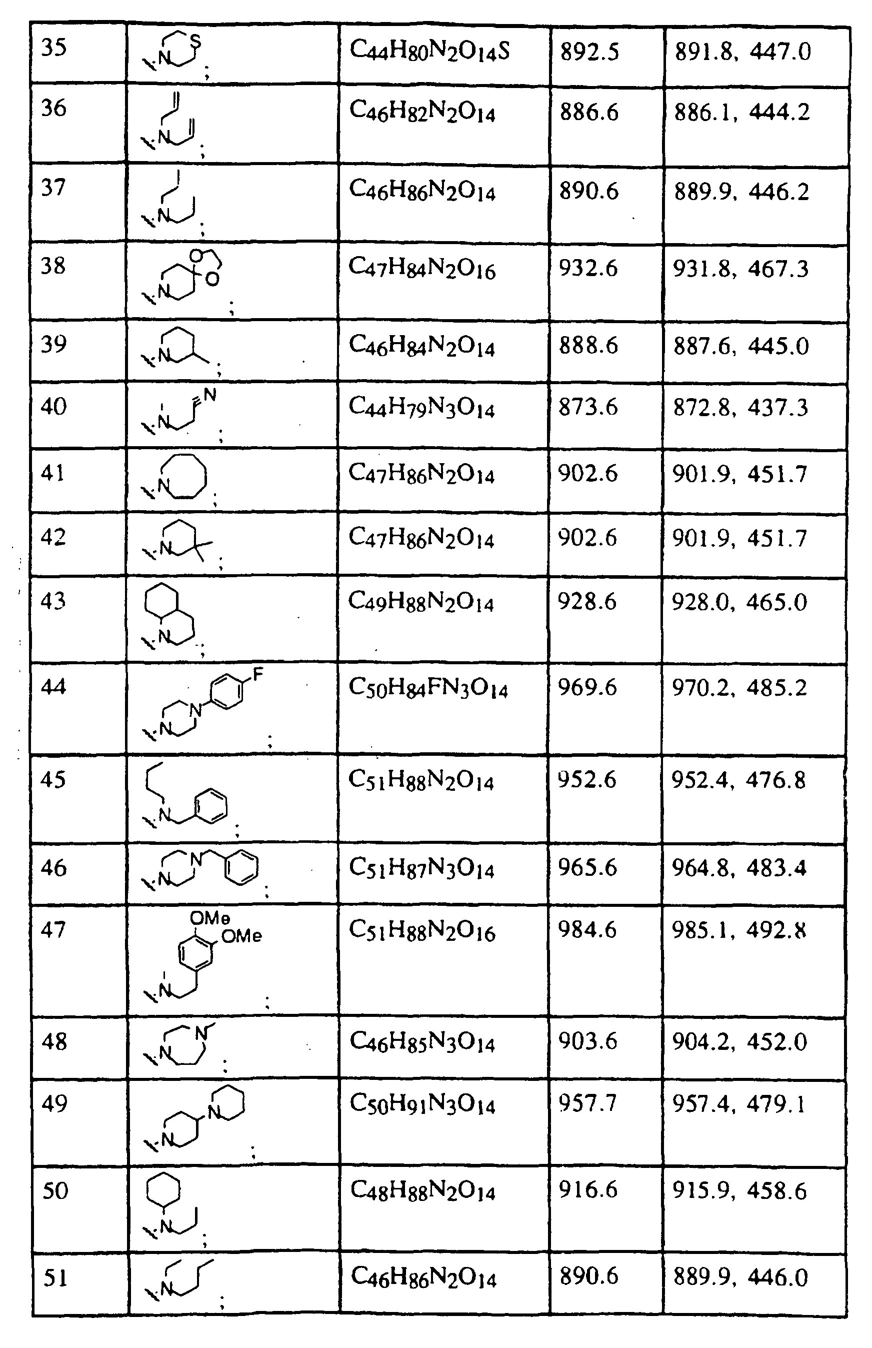

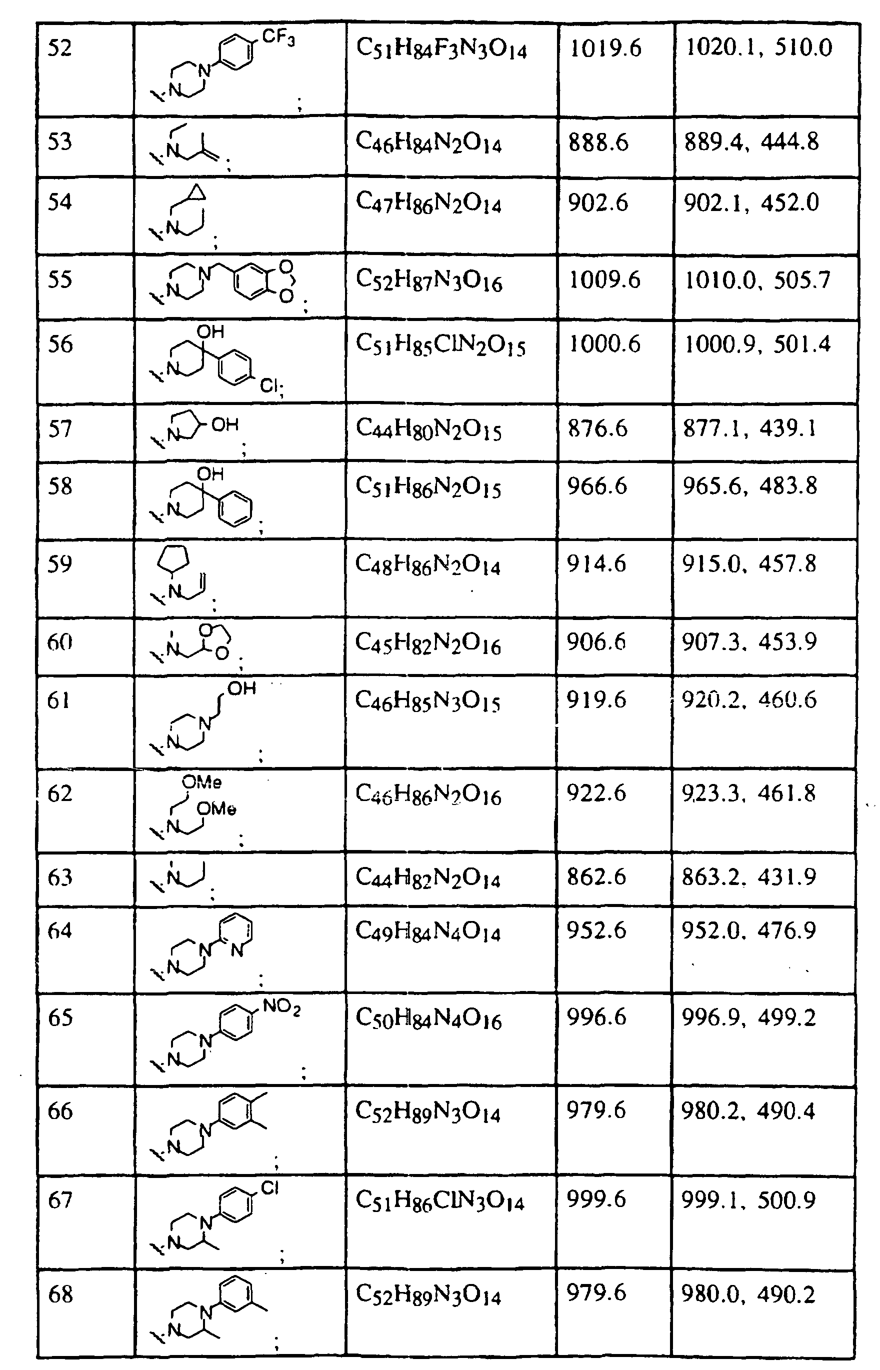

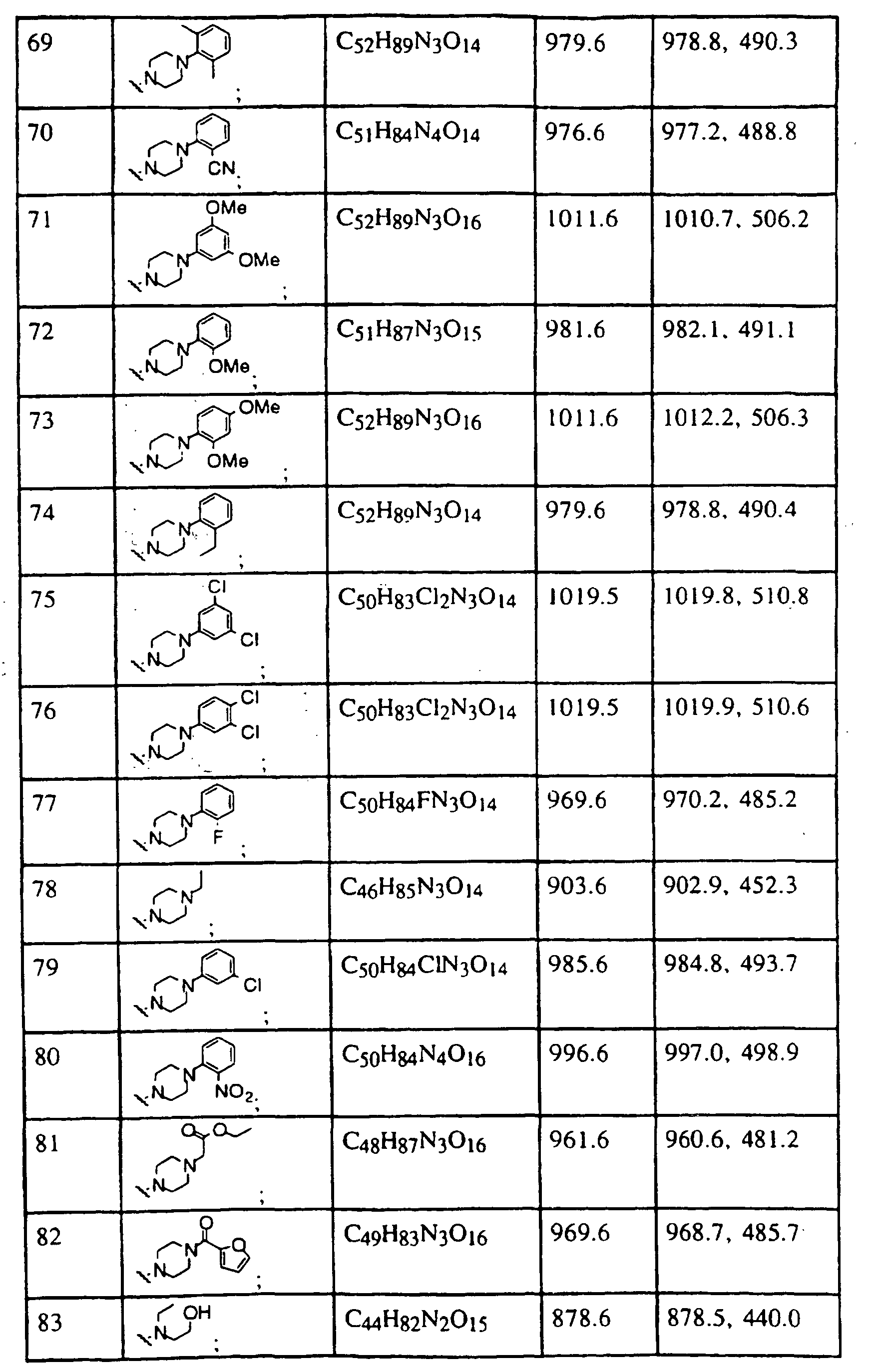

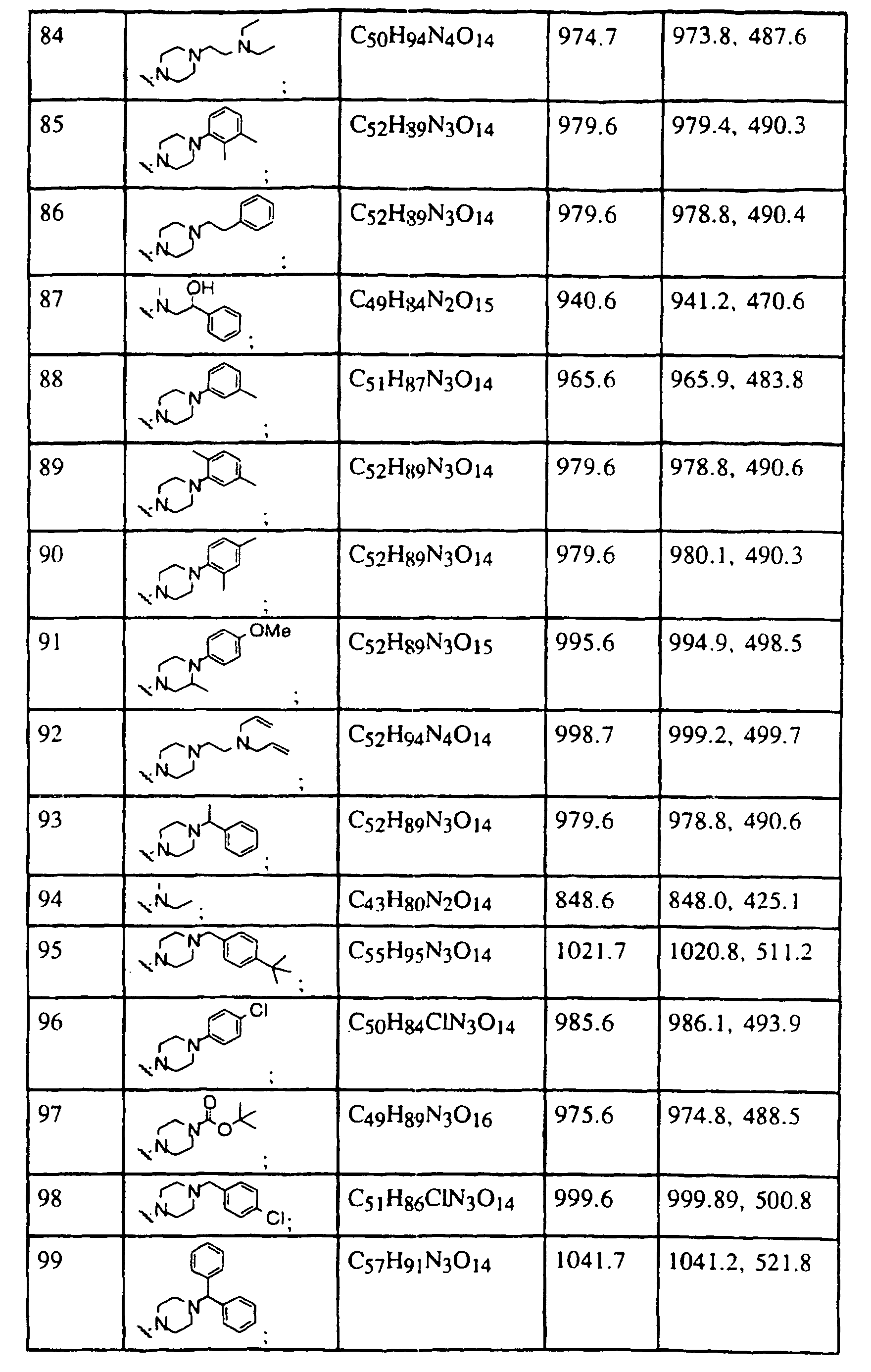

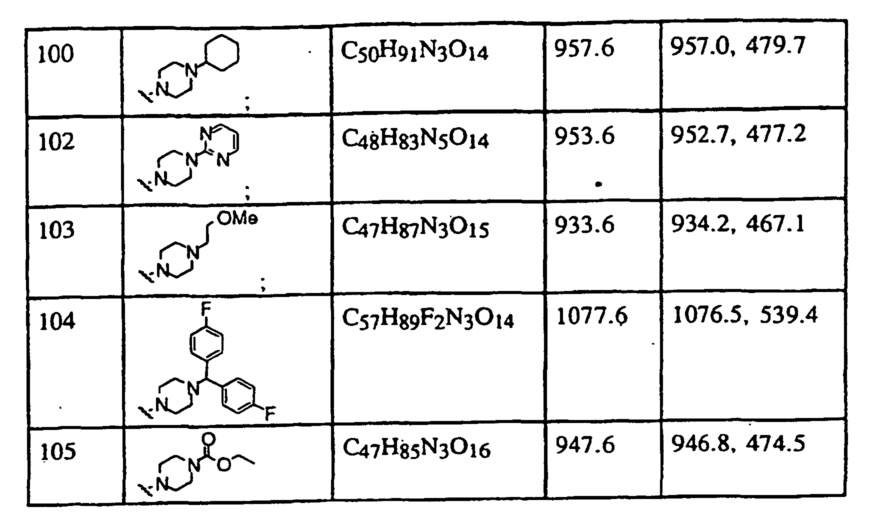

### Examples 106-153 as comparative examples

### Compounds having Formula (X) wherein X is O; R is CH2-CH2-R^{W}; and R^{W} is as shown

Treating the compound of Example 8 with an appropriate reagent amine in the presence of sodium cyanoborohydride in methanol and acetic acid, the compounds of Examples 106-153, which are compounds having Formula (X) wherein X is O, R is CH₂-CH₂-R^{W}; and R^{W} is as shown in Table 2 below, were prepared.

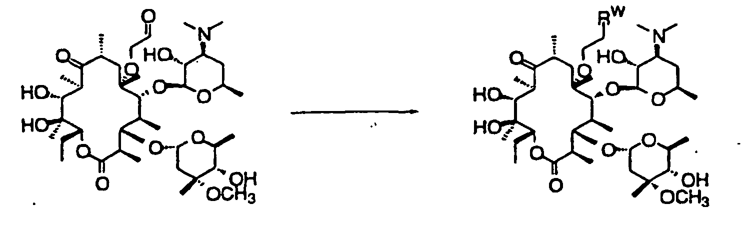

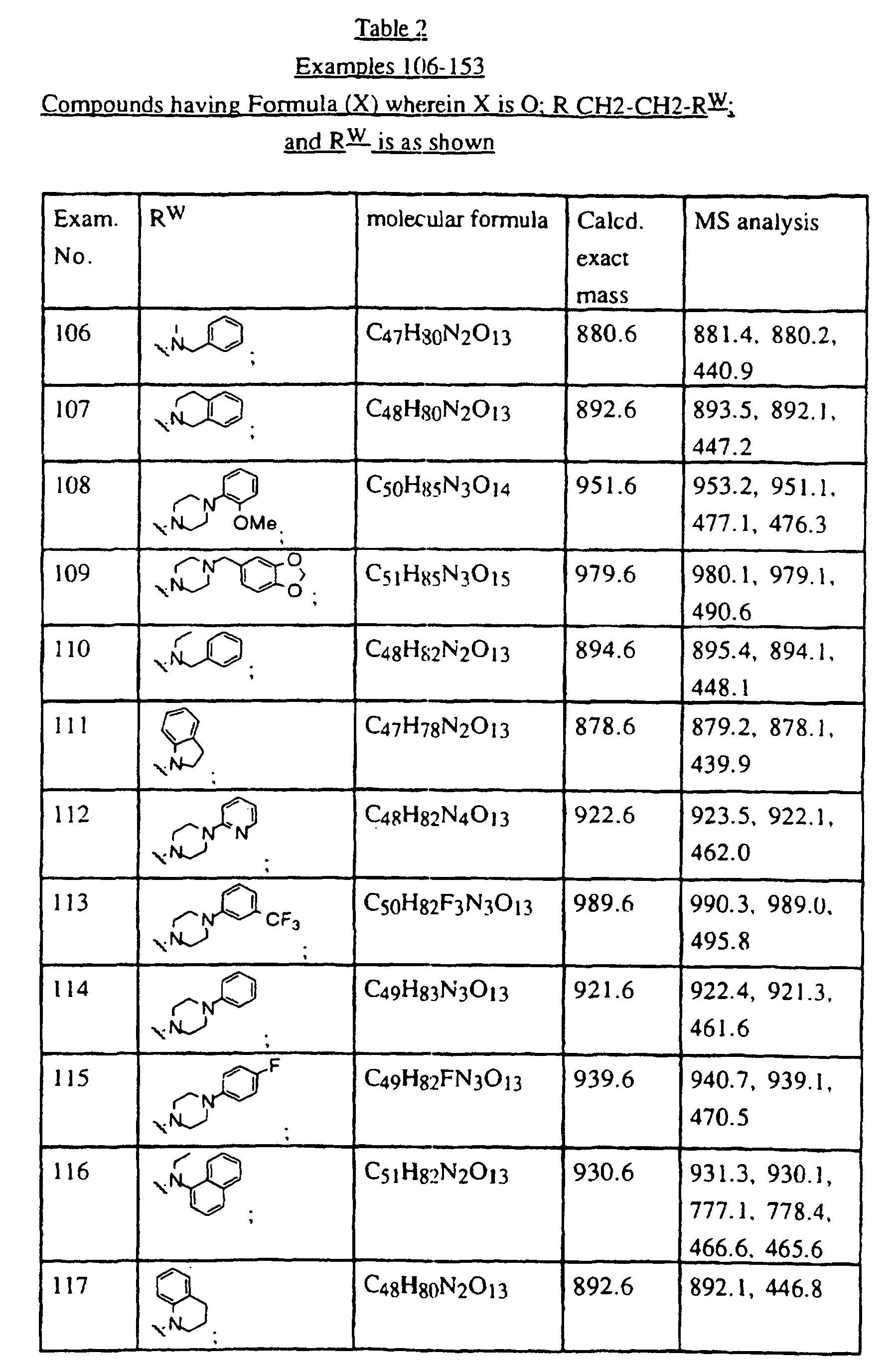

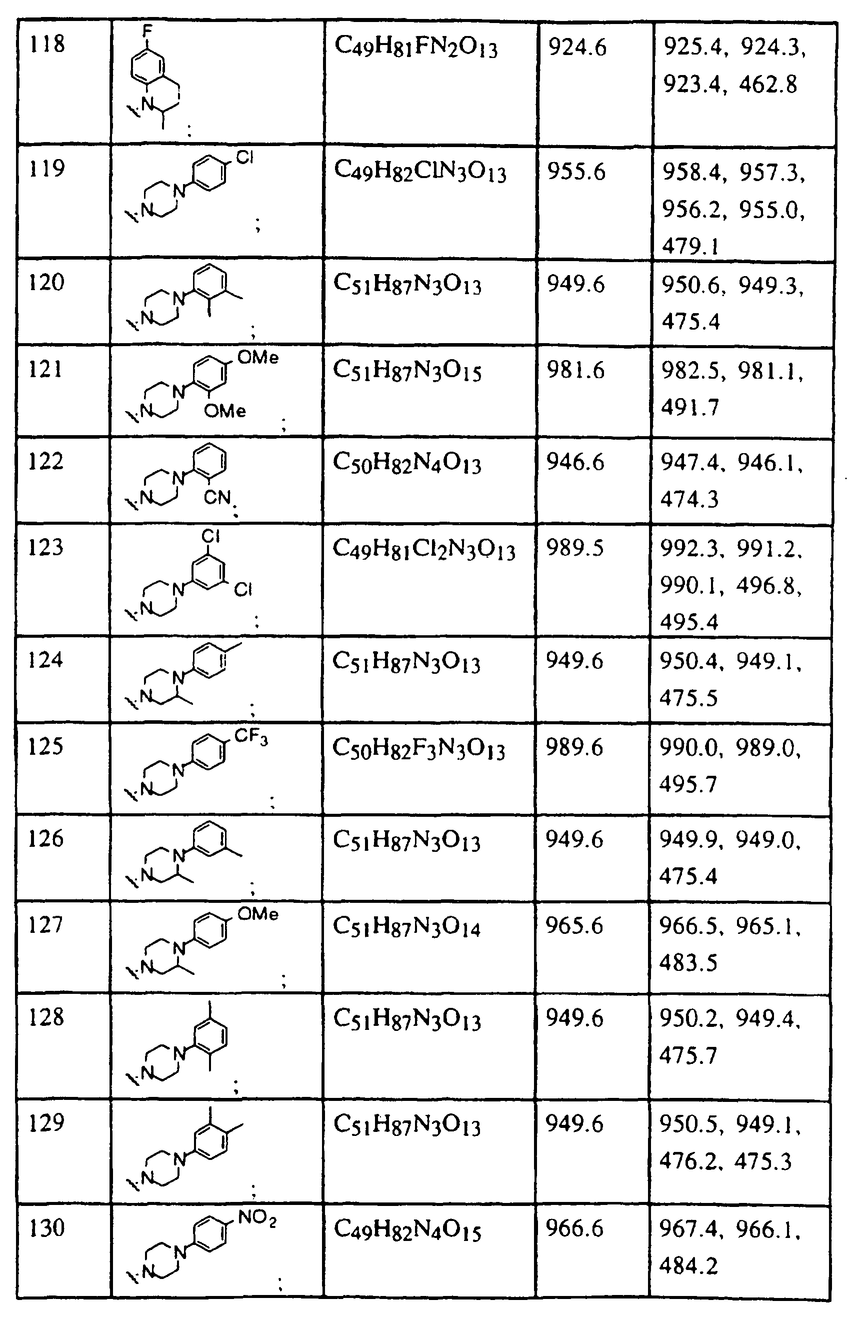

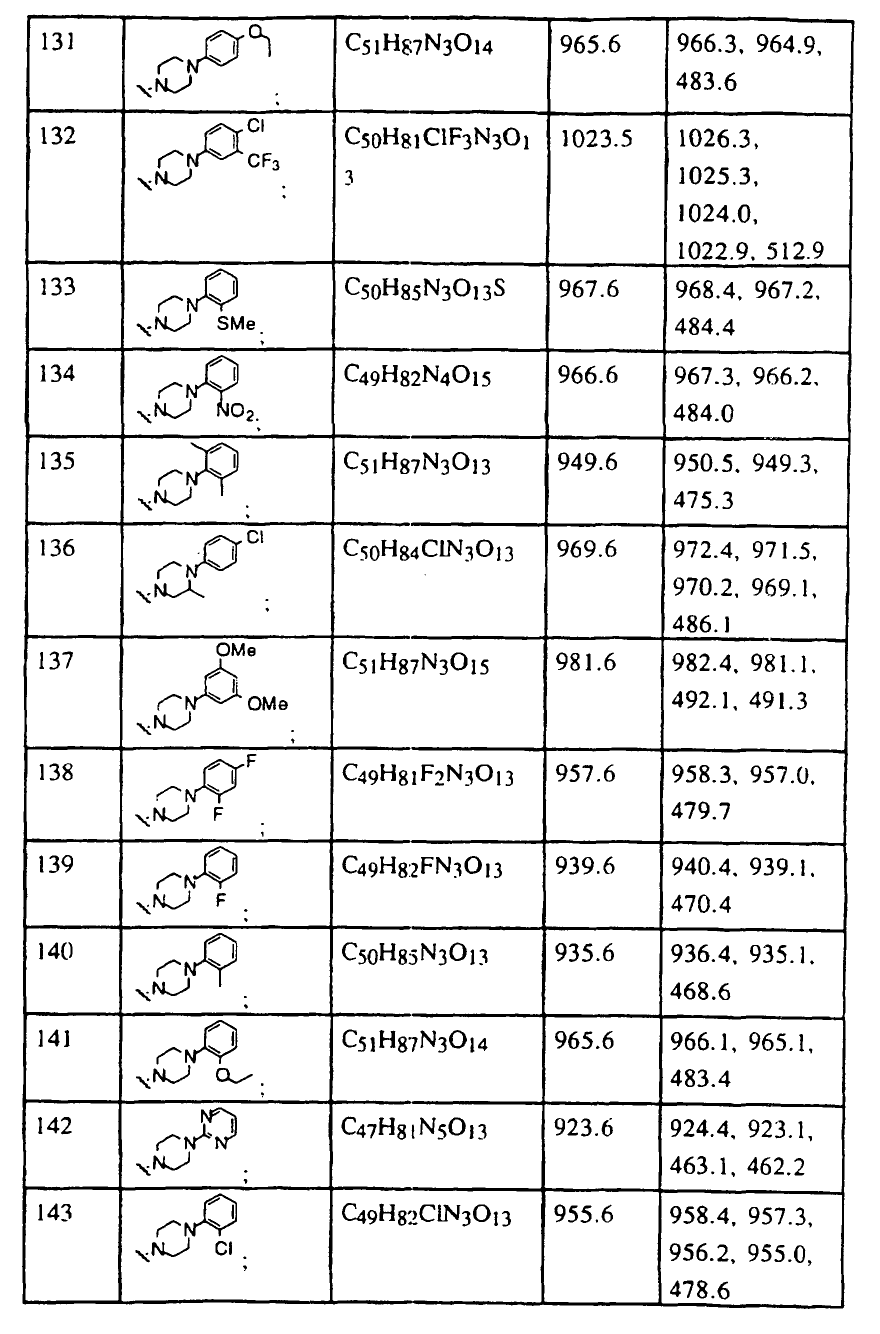

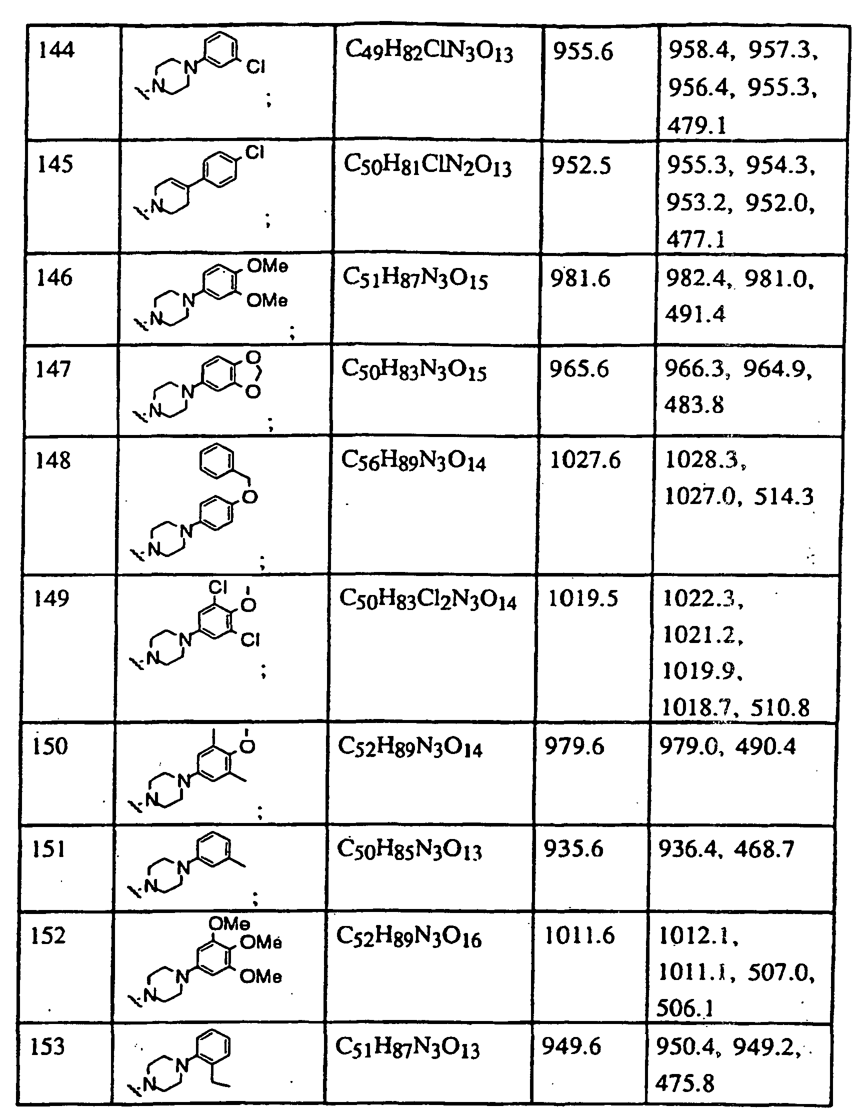

### Examples 154-164 as comparative examples

### Compounds having Formula (X) wherein X is O, R is CH2-CH=N-R^{X}, and R^{X} is as shown

Treating the compound of Example 8 according to the procedures of Example 12, except substituting the appropriate substituted hydroxyl amine reagent for the unsubstituted hydroxylamine reagent of Example 12; the compounds of Examples 154-164, which are compounds having Formula (X) wherein X is O, R is CH2-CH=N-R^{X} and R^{X} is as shown in Table 3 below were prepared.

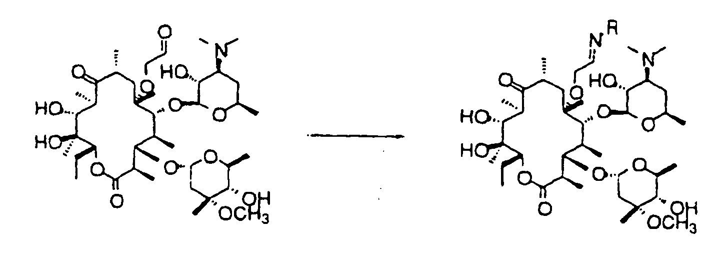

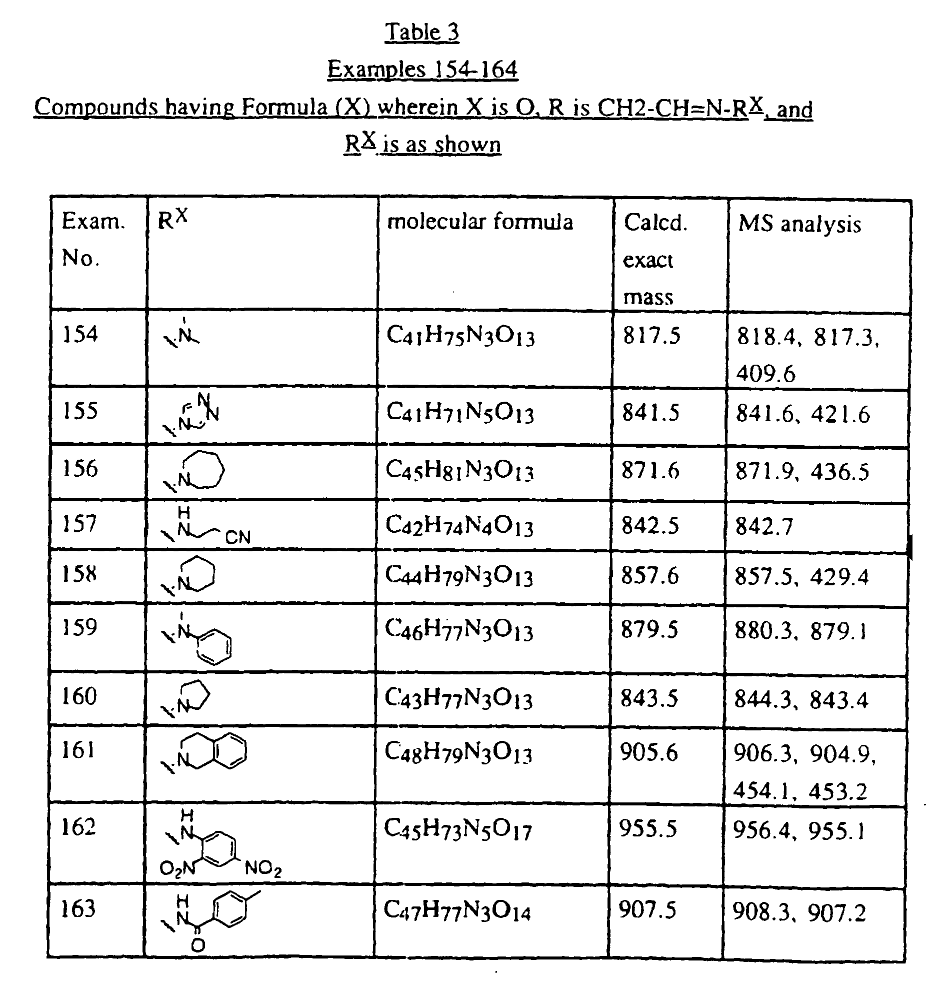

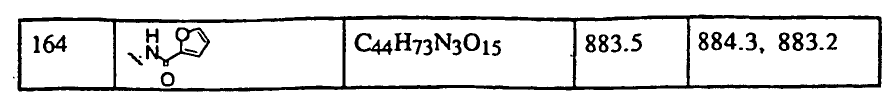

### Examples 165 - 182 are comparative examples

### Example 165

### Compound of Formula (V): R^{b} is H; R^{c} is H; R^{d} is phenylmethyloxycarbonyl; R^{e} is methoxy; R^{f} is H; R is -CH₂-CH=CH₂

### Step 165a. 2'-acetyl-6-O-allyl erythromycin A

A solution of 6-O-allyl erythromycin A (10 g, the compound of Formula (X) wherein X is =O and R is -CH₂-C=CH₂, from Example 10 step c above) and triethylamine (2.25 mL) in dichloromethane (50 mL) was cooled to 0 °C and flushed with nitrogen. Acetic anhydride (2.4 mL) was added, the solution was stirred at 0 °C for 5 minutes, then the ice bath was removed and the mixture was stirred for 5 hours at ambient temperature. The mixture was quenched by addition of 1.5 M aqueous KH₂PO₄ (50 mL), then extracted with ethyl acetate. The organic phase was washed with water and brine, then dried over MgSO₄. The solvent was removed under vacuum, and the residue was dried. The residue was crystallized from acetonitrile to give the title compound (5.66 g).

### Step 165b. 2'-acetyl-4'-(phenylmethyloxycarbonyl)-6-O-allyl erythromycin A

To a solution of 2'-acetyl-6-O-allyl erythromycin A (5 g, from step 165a) in dry dichloromethane (50 mL) was added DMAP (3 g), and the solution was cooled to -40 °C. The solution was flushed with nitrogen, and benzyl chloroformate (3 mL) waa added over 45 minutes. Then additional DMAP (585 mg) and benzyl chloroformate (0.585 mL over 45 minutes) was added. The mixture was stirred under nitrogen for 0.5 hours at -40 °C and at -20 °C for 40 hours, then diluted with ethyl acetate and quenched with saturated aqueous sodium bicarbonate. The organic phase was separated, washed with water and brine, then dried over MgSO₄. The solvent was removed, the residue (6.5 g) was triturated with ethyl acetate, and the solvent was washed, dried and removed under vacuum. The residue was crystallized from acetonitrile, then purified by chromatography on mixed alumina and silica gel to give the title compound (4.6 g).

### Step 165c. Compound of Formula (V): R^{b} is H; R^{c} is H; R^{d} is phenylmethyloxycarbonyl; R^{e} is methoxy; R^{f} is acetyl; R is -CH₂-CH=CH₂

To a solution of 2'-acetyl-4'-(phenylmethyloxycarbonyl)-6-O-allyl erythromycin A (4.5 g, from step 165b) in THF (90 mL) at -40 °C was added 1 M sodium bis(trimethylsilyl)amine (5.25 mL), and the mixture was stirred for 10 minutes. To this was added carbonyldimidazole (2.7g) in THF over 45 minutes, then the ice bath was removed and the mixture was stirred at ambient temperature for 40 minutes. The mixture was cooled to 0 °C, quenched with 1 M aqueous KH₂PO₄, then extracted with ethyl acetate. The organic phase was washed with water and brine, then dried over MgSO₄. The solvent was removed under vacuum, and the residue was dried. The residue was crystallized from acetonitrile, then purified by chromatography on silica gel, eluting with 25-50% acetone/hexane to give the title compound (2.65 g).

### Step 165d. Compound of Formula (V): R^{b} is H; R^{c} is H; R^{d} is phenylmethyloxycarbonyl; R^{e} is methoxy; R^{f} is H; R is -CH₂-CH=CH₂

A solution of the compound from step 165c (400 mg) in methanol (30 mL) was stirred at room temperature for 20 hours and at 50 °C for 6 hours. The solvent was removed to give the title compound. H. Res. MS 934.5176 (M+H)⁺.

### Example 166

### Compound of Formula (V): R^{b} is H; R^{c} is H; R^{d} is hydroxy, R^{e}, is methoxy; R^{f} is H; R is -CH₂-CH=CH₂

To a sample of the compound from Example 165 in methanol was added 10% Pd/C (450 mg), and the mixture was shaken under 1 atm of hydrogen for 2.5 hours. The mixture was filtered, and the solvent was removed. The residue was purified by chromatography on silica gel, eluting with 25-50% acetone/hexane to give the title compound. H. Res. MS 802.4941 (M+H)⁺.

### Example 167

### Compound of Formula (VI): R^{b} is H; R^{c} is H; R^{d} is phenylmethyloxycarbonyl; R^{e} is methoxy; R^{f} is acetyl; R is -CH₂-CH=CH₂

A solution of the compound of Example 165 step c (3.6 g) and DBU (4.4 mL) in benzene (136 mL) was heated at reflux for 8 hours. The solvent was removed under vacuum, and the residue was dissolved in ethyl acetate. This solution was extracted with 1M NaH₂PO₄ and washed with brine, then dried over MgSO₄. The solvent was removed, and the residue was purified by chromatography on silica gel, eluting with 25% acetone/hexane to give the title compound (3.3 g).

### Example 168

### Compound of Formula (VII): W is -NH-; R^{b} is H; R^{c} is H: R^{d} is phenylmethyloxycarbonyl; R^{e} is methoxyl;R^{f} is H; R^{g} is H; R is propyl

### Step 168a. Compound (28) of Scheme V: 2'-R^{p} is acetyl, 4"-R^{p} is phenylmethyloxycarbonyl; R is -CH₂-CH=CH₂

To a -10 °C solution of the compound of Example 167 (3.3 g) in THF (60 mL) flushed with nitrogen was added 60% NaH (284 mg), and the mixture was stirred at -10 °C for 15 minutes. The mixture was warmed to 0 °C, and a solution of CDI (1.7 g) in THF (30 mL) was added over 15 minutes. The mixture was then stirred at room temperature under nitrogen for 2.5 hours. The mixture was cooled to 0 °C, quenched with ethyl acetate, and saturated aqueous sodium bicarbonate solution was added. The organic phase was separated, washed with brine and dried over MgSO₄. The solvent was removed, and the residue was purified by chromatography on silica gel, eluting with 35% acetone/hexane to give the title compound (3.2 g).

### Step 168b. Compound of Formula (VII): W is -NH-; R^{b} is H; R^{c} is H; R^{d} is phenylmethyloxycarbonyl; R^{e} is methoxy; R^{f} is H; R^{g} is H; R is propyl

To a sample of the compound from step 168a (100 mg) in DMF (1 mL) was added hydrazine (0.048 mL), and the mixture was stirred at 60 °C under nitrogen for 43 hours. The mixture was diluted with ethyl acetate, then washed with aqueous sodium bicarbonate and dried over MgSO₄. The solvent was removed, and the treatment with hydrazine was repeated, heating for 24 hours at 60 °C. The mixture was diluted with ethyl acetate, then washed with aqueous sodium bicarbonate and dried over MgSO₄. The solvent was removed, and the residue was purified by chromatography on silica gel, eluting with 1% methanol/dichloromethane containing 0.5% NH₄OH to give the title compound (136 mg). H. Res. MS 950.5594 (M+H)⁺.

### Example 169

### Compound of Formula (VII): W is -NH-; R^{b} is H; R^{c} is H; R^{d} is H; R^{e} is methoxy; R^{f} is H; R^{g} is H; R is propyl

To a sample of the compound from Example 168 (73 mg) in methanol (2 mL) was added 10% Pd/C (60 mg), and the mixture was shaken under 1 atm of hydrogen for 2 hours. The mixture was filtered, and the solvent was removed to give the title compound (56 mg). H. Res. MS 816.5198 (M+H)⁺.

### Example 170

### Compound of Formula (VII): W is -NH-; Rb is H; R^{c} is H; R^{d} is phenylmethyloxycarbonyl; R^{e} is methoxy; R^{f} is H; R^{g} is H; R is -CH₂-CH=CH₂

To a solution of the compound of Example 168 step a (3.3 g) in acetonitrile (26 mL) was added hydrazine (0.206 mL), and the mixture was stirred at room temperature for 22 hours. The mixture was diluted with ethyl acetate, and this solution was washed with water and brine and dried over MgSO₄. The solvent was removed, and the residue was dissolved in methanol and stirred under nitrogen at room temperature for 66 hours. The solvent was removed to give the title compound containing a mixture (2.20 g) of the C10 epimers. This material was dissolved in methanol (15 mL) and stirred at ambient temperature in a sealed tube with 2 M NH₃ in methanol (15 mL) for 5 days. The solvent was removed, and the residue (2.18 g) was purified by chromatography on silica gel, eluting with 7% methanol/dichloromethane containing 3% NH₄OH to give the title compound (1.86 g). H. Res. MS 948.5440 (M+H)⁺.

### Example 171

### Compound of Formula (VII): W is absent; R^{b} is H; R^{c} is H; R^{d} is phenylmethyloxycarbonyl; R^{e} is methoxy; R^{f} is acetyl; R^{g} is 4-phenylbutyl; R is -CH₂-CH=CH₂

To a solution of the compound of Example 168 step a (2.0 g) in acetonitrile (22 mL) was added 4-phenylbutylamine (2.5 mL), and the mixture was stirred at room temperature for 66 hours. The solvent was removed, and the residue was purified by chromatography on silica gel, eluting with 1% ethyl acetae/hexane containing 1% NH₄OH to give the title compound (2.14 g).

### Example 172

### Compound of Formula (VII): W is absent; R^{b} is H; R^{c} is H; R^{d} is phenylmethyloxycarbonyl; R^{e} is methoxy; R^{f} is H; R^{g} is 4-phenylbutyl; R is -CH₂-CH=CH₂

A solution of the compound of Example 17 (400 mg) in methanol (15 mL) was stirred under nitrogen at room temperature for 4 days. The solvent was removed, and the residue (2.18 g) was purified by chromatography on silica gel, eluting with 40% acetone/hexane to give the title compound (355 mg). MS 1065 (M+H)⁺.

### Example 173

### Compound of Formula (VII): W is absent; R^{b} is H; R^{c} is H: R^{d} is H; R^{e} is methoxy; R^{f} is H; R^{g} is 4-phenylbutyl; R is propyl

To a sample of the compound from Example 172 (220 mg) in methanol (4 mL) was added 10% Pd/C (200 mg), and the mixture was shaken under 1 atm of hydrogen for 3 hours. The mixture was filtered, and the solvent was removed to give a compound which was purified by chromatography on silica gel, eluting with 1% methanol/dichloromethane containing 10% NH₄OH to give the title compound (148 mg). Anal. Calcd. for C₅₁H₈₄N₂O₁₃: C, 65.63: H, 9.07; N, 3.00; Found: C, 65.25; H, 9.00; N, 2.87. H. Res. MS 933.6059 (M+H)⁺.

### Example 174

### Compound of Formula (VII): W is absent: R^{b} is H; R^{c} is H; R^{d} is phenylmethyloxycarbonyl; R^{e} is methoxy; R^{f} is acetyl; R^{g} is 4-phenylbutyl; R is -CH₂-CH=CH₂-(3-quinolinyl)

To a sample of the compound from Example 171 (500 mg, 0.452 mmol) in acetonitrile (5 mL) were added 3-bromoquinoline (188 mg, 0.904 mmol), palladium acetate (21 mg, 0.094 mmol), tri-(o-tolyl)phosphine (55 mg, 0.181 mmol) and triethylamine (0.126 mL, 0.904 mmol). The mixture was cooled to -78°C, degassed, sealed in a tube under nitrogen, and heated at 50 °C for 2 hours, and at 80 °C for 14 hours. Additional palladium acetate (20 mg. 0.094 mmol) and tri-(o-tolyl)phosphine (20 mg) were added, and the mixture was heated at 100 °C for 12 hours. Additional 3-bromoquinoline (0.046 mL) and triethylamine (0.065) were added, and the mixture was heated at 100 °C for 24 hours. The mixture was cooled and diluted with ethyl acetate, then washed with saturated brine, 1M tris(hydroxymethyl)aminomethane, brine and dried over MgSO₄. The solvent was removed to give a compound which was purified by chromatography on silica gel, eluting with 25-40% acetone/hexane to give the title compound (444 mg).

### Example 175

### Compound of Formula (VII): W is absent; R^{b} is H; R^{c} is H; R^{d} is phenylmethytoxycarbonyl; R^{e} is methoxy; R^{f} is H; R^{g} is 4-phenylbutyl; R is -CH₂-CH=CH₂-(3-quinolinyl)

A solution of the compound of Example 174 (444 mg) in methanol (20 mL) was stirred under nitrogen at 50 °C for 7 hours and at room temperature for 16 hours. The solvent was removed, and the residue (420 mg) was purified by chromatography on silica gel, eluting with 1% methanol/dichloromethane to give the title compound (170 mg). H. Res. MS 1192.6678 (M+H)⁺.

### Example 176

### Compound of Formula (VII): W is absent; R^{b} is H; R^{c} is H; R^{d} is H; R^{e} is methoxy; Rf is H; R^{g} is 4-phenylbutyl; R is -CH₂-CH₂-CH₂(3-quinolinyl)

To a sample of the compound from Example 175 (130 mg) in methanol (6 mL) was added 10% Pd/C (100 mg), and the mixture was shaken under 1 atm of hydrogen for 17 hours. The mixture was filtered, and the solvent was removed to give a compound which was purified by chromatography on silica gel, eluting with 2% methanol/dichloromethane containing 1% NH₄OH to give the title compound (41 mg). H. Res. MS 1060.6453 (M+H)⁺.

### Example 177

### Compound of Formula (VI): R^{a} is hydroxy; R^{b} is H; R^{c} is H; R^{d} is H; R^{e} is methoxy; R^{f} is H; R is -CH₂-CH=CH₂

A mixture of 6-O-allyl erythromycin A (30 g. prepared according to Example 10 step c above), trethylamine (70 mL) and ethylene carbonate (24 g) was heated at 95 °C under nitrogen with stirring for 66 hours. The mixture was cooled and diluted with ethyl acetate and extracted with water. The organic phase was washed with water and brine and dried over MgSO₄. The solvent was removed under vacuum, and the residue was purified by chromatography on silica gel, eluting with 2% methanol/dichloromethane containing 1% NH₄OH to give the title compound (20.9 g). The compound was crystallized from acetonitrile with a yield of 14.6 g in the first crop MS 756 (M+H)⁺.

### Example 178

### Compound of Formula (VII): W is absent; R^{b} is H; R^{c} is H; R^{d} is H; R^{e} is methoxy; R^{f} is H; R^{g} is H; R is -CH₂-CH=CH₂

### Step 178a. Compound of Formula (VI): R^{a} is hydroxy; R^{b} is H; R^{c} is H; R^{d} is trimethylsilyl; R^{e} is methoxy; R^{f} is trimethylsilyl; R is -CH₂-CH=CH₂

To a solution of the compound from Example 177 (12.2 g) in acetonitrile (90 mL), THF (20 mL) and dichloromethane (6 mL) was added hexamethyldisilazane (10.1 mL), and the reaction was stirred at room temperature for 44 hours. The solvents were then removed under vacuum to give the title compound (15.1 g).

### Step 178b. Compound (28) of Scheme V: 2'-R^{p} is trimethylsilyl; 4"-R^{p} is trimethylsilyl; R is -CH₂-CH=CH₂

To a -10°C solution of the compound of step 178a (15.1 g) in freshly distilled THF (200 mL) was added 60% NaH (1.3 g), and the mixture was stirred for 10 minutes then warmed to 0 °C. To this solution was added carbodiimidazole (6.5 g) in THF (100 mL) over 15 minutes, then the mixture was stirred at room temperature for 2 hours. The mixture was cooled to 0 °C, diluted with ethyl acetate and quenched with 5% aqueous sodium bicarbonate solution. This mixture was extracted with ethyl acetate, and the organic phase was washed with water and brine, then dried over MgSO₄. The solvent was removed to give the title compound.

### Step 178c. Compound of Formula (VII): W is absent; R^{b} is H; R^{c} is H; R^{d} is trimethylsilyl; R^{e} is methoxy; R^{f} is trimethylsilyl; R^{g} is H; R is -CH₂-CH=CH₂

A solution of the compound from step 178b (17.5 g) in acetonitrile (250 mL) and liquid ammonia (250 mL) at -78 °C was sealed in a tube and stirred at room temperature for 24 hours. After cooling to -78 °C the seal was broken, and the solution was stirred at room temperature to release the excess ammonia. The solvent was then removed under vacuum to give the title compound.

### Step 178d. Compound of Formula (VII): W is absent; R^{b} is H; R^{c} is H; R^{d} is H; R^{e} is methoxy; R^{f} is H; R^{g} is H; R is -CH₂-CH=CH₂

To a solution of the compound of step 178c (17 g) in acetonitrile (200 mL) at 0 °C was added a solution of HF (48%, 2.3 mL) in acetonitrile (10 mL) over one minute. The mixture was stirred at room temperature for 1 hour, then cooled to 0 °C. Solid sodium bicarbonate (9 g) was added, and the mixture was stirred for 30 minutes. The solution was diluted with ether (350 mL) and water (200 mL), and the phases were separated. The organic phase was washed with water and brine, then dried over MgSO₄. The solvent was removed, and the residue was purified by chromatography on silica gel, eluting with 1% methanol/dichloromethane containing 1% NH₄OH to give the title compound (12.3 g). H. Res. MS 799.4962 (M+H)⁺.

### Example 179

### Compound of Formula (VII): W is absent; R^{b} is H; R^{c} is H; R^{d} is H; R^{e} is methoxy; R^{f} is H; R^{g} is H; R is propyl

To a sample of the title compound of Example 178 (150 mg) in methanol (3 mL) was added 10% Pd/C (120 mg), and the mixture was treated with 1 atm of hydrogen at room temperature for 5.5 hours. The catalyst and the solvent were removed, and the residue was purified by chromatography on silica gel, eluting with 1% methanol/dichloromethane containing 1% NH₄OH to give the title compound (84 mg). H. Res. MS 801.5110 (M+H)⁺.

### Example 180

### Compound of Formula (VII): W is absent; R^{b} is H; R^{c} is H; R^{d} is H; R^{e} is methoxy; Rf is H; R^{g} is H; R is -CH₂-C(O)-H

A sample of the title compound (6 g) from Example 178 was treated with ozone according to the procedure of Example 8b to give the title compound (4.5 g).

### Example 181

### Compound of Formula (VII): W is absent; R^{b} is H; R^{c} is H; R^{d} is H; R^{e} is methoxy; R^{f} is H; R^{g} is H; R is -CH₂-C=N-O-CH₂-phenyl

To a sample of the title compound (200 mg) from Example 180 in methanol (1 mL) was added a solution of O-benzylhydroxylamine hydrochloride (76 mg) in methanol (1 mL) and 0.082 mL of N-methylmorpholine. The mixture was stirred at room temperature for 16 hours, then diluted with ethyl acetate, washed with water and brine, and dried over MgSO₄. The solvent was removed, and the residue was purified by chromatography on silica gel, eluting with 1% methanol/dichloromethane containing 1% NH₄OH to give the title compound (125 mg, 56%) as a mixture of syn/anti isomers. H. Res. MS 906.5314 (M+H)⁺.

### Example 182

### Compound of Formula (VII): W is absent; R^{b} is H; R^{c} is H; R^{d} is H; R^{e} is methoxy; R^{f} is H; R^{g} is H; R is -CH₂-CH₂-NH-CH₂-phenyl

To a sample of the title compound (150 mg) from Example 180 in methanol (2 mL) at 0 °C was added benzylamine (0.024 mL), acetic acid (0.034 mL) and sodium cyanoborohydride (82 mg, in three portions). The mixture was stirred at room temperature for 2 hours, then quenched by addition of 5% aqueous sodium bicarbonate and extracted into ethyl acetate. The organic phase was washed with water and brine, and dried over MgSO₄. The solvent was removed, and the residue was purified by chromatography on silica gel, eluting with 2% methanol/dichloromethane containing 1% NH₄OH to give the title compound (35 mg). H. Res. MS 892.5526 (M+H)⁺.

### Example 183

### Compound of formula (X): X=O, R=Phenylpropyl

A sample of the compound prepared according to Example 17 (70 mg) was catalytically hydrogenated by procedures described in Example 2. The crude product (64 mg) was purified by column chromatography on silica gel eluting with 98:1:1 dichloromethane/methanol/ammonium hydroxide to afford the title compound (44 mg, 44 %). MS m/z 852 (M+H)⁺.

### Example 184

### Compound of formula (X): X=O, R is -CH₇CH=CH-(4-methylphenyl)

To a mixture of NaH (60%, 19 mg) in THF (2 mL) at 0 °C was added tris(4-methylphenyl)phosphonium chloride (172 mg) over 3 minutes. The mixture was stirred at ambient temperature for 40 minutes, then re-ecooled to 0 °C. To this mixture was added a THF solution (2 mL) containing a sample of the compound of Example 8 (300 mg). The reaction was sealed under nitrogen and stirred at ambient temperature for 30 hours. The reaction mixture was taken up in EtOAc and washed successively with water, aqueous 5% sodium bicarbonate and brine. The organic layers were dried over MgSO₄ and concentrated *in vacuo.* The crude product (428 mg) was purified by column chromatography on silica gel eluting with 8% methanol in dichloromethane to afford the title compound (120 mg, 42%). High Res. MS: Calculated m/z for (M+H)⁺: C₄₇H₇₈NO₁₃: 864.5473: Found: 864.5444.

### Example 185

### Compound of formula (X): X=O, R is -CH₂-CH(OH)-Phenyl

To a solution of the compound of Example 8 (500 mg) in THF (15 mL) at -10 °C was added phenylmagnesium bromide (6.6 mL. 1 molar solution in THF) over 20 minutes under a nitrogen atmosphere. The reaction was stirred at ambient temperature for 2 hours, then cooled to 0°C. Water (1 mL) was added, the mixture was stirred for 3 minutes, then extracted with EtOAc. The organic layers were washed with brine, dried over MgSO₄ and concentrated *in vacuo.* Chromatography on silica gel eluting with dichioromethane/MeOH/ammonium hydroxide (98:1:1) gave the title compound (300 mg). High Res. MS: calculated m/z for (M+H)⁺: C₄₅H₇₆NO₁₄: 854.5266; Found: 854.5264.

### Example 186

### Comgound of formula (X): X=O, R is -CH₂-CH(Br)-CH₂Br

To a solution of the compound of Example 1D (200 mg) in dichloromethane (5 mL) 0 °C was added acetic acid (0.073 mL) and pyridinium bromide perbromide (134 mg). After 1 hour at 0 ° the reaction was warmed to ambient temperature and stirred for 16 hours. The reaction mixture was concentrated *in vacuo,* the residue was dissolved in EtOAc which was washed with water (2X) and brine, then dried over MgSO₄ and concentrated *in vacuo.* Chromatography on silica gel eluting with 8% MeOH in dichloromethane gave 155 mg (65%) of the title compound. High resolution Mass Spec: calculated m/z for (M+H)⁺: C₄₀H₇₂NO₁₃⁷⁹Br₂:932.3370; Found: 932.3376.

### Example 187

### Compound of formula (X): X=O, R is -CH₂CH₂NHCH₂CH₂CH₂-Phenyl

To a solution of the compound of Example 8 (150 mg) and 3-phenylpropylamine (0.033 mL) in ethanol (2 mL) was added acetic acid (0.044 mL). After stirring at ambient temperature for 30 minutes, Pd/C (10 %, 125 mg) was added under a nitrogen atmosphere. The reaction was stirred for 16 hours under 1 atm hydrogen, then the mixture was filtered and concentrated. The residue was dissolved in EtOAc and washed with 5% sodium bicarbonate and brine. After drying over MgSO₄ and concentrating *in vacuo,* the crude residue was purified with column chromatography on silica gel eluting with dichloromethane/MeOH/ammonium hydroxide (97:2:1) to afford of the title compound (90 mg, 55%). High resolution Mass Spec: calculated m/z for (M+H)⁺: C₄₈H₈₃N₂O₁₃:895.5895: Found: 895.5905.

### Example 188

### Compound of formula (X): X=O, R is -CH₂CH₂NHCH(CH₂Phenyl)CO₂Me

To a solution of the compound of Example 8 (150 mg). L-phenylalanine methyl ester hydrochloride (250 mg), acetic acid (0.066 mL) in methanol (2 mL) was added sodium cyanoborohydride (120 mg in 3 portions) over 5 minutes under nitrogen. The reaction was stirred at ambient temperature for 4.5 hours, then was quenched with aqueous 5% sodium bicarbonate. Following an EtOAc extraction and brine wash, the organic layer was dried over MgSO₄. The EtOAc extracts were filtered and concentrated *in vacuo.* Purification was performed with column chromatography on silica gel eluting with dichloromethane/methanol/ammonium hydroxide (98:1:1) to give the title compound (127 mg. 70%) . High resolution Mass Spec: calculated m/z for (M+H)⁺: C₄₉H₈₃N₂O₁₅: 939.5793: Found:939.5798.

### Example 189

### Compound of formula (X): X=O, R is -CH₂CH₂NHCH₂CH₂CH₃

The title compound was prepared by procedures described in Example 187 except substituting propylamine for phenylpropylamine to give the title compound (90 mg, 57%). H. Res. MS 819.5583 (M+H)⁺.

### Example 190

### Compound of formula (X): X=O, R is -CH₂CH₂NHCH₂CO₂CH₂CH₂

The title compound was prepared by procedures described in Example 187 except substituting glycine ethyl ester for phenylpropylamine to give the title compound (72 mg, 46%). H. Res. MS 863.5472 (M+H)⁺.

### Example 191

### Compound of formula (X): X=O, R is -CH₂CH₂NHCH₂CH₂-Phenyl

The title compound was prepared by procedures described in Example 187 except substituting phenethylamine for phenylpropylamine to give of the title compound (55 mg, 34%). H. Res. MS 881.5762 (M+H)⁺.

### Example 192

### Compound of formula (X): X=O, R is -CH₂CH₂NHCH₂CH₂-(4-hydroxyphenyl)

The title compound was prepared by procedures described in Example 187 except substituting 4-hydroxyphenethylamine for phenylpropylamine to give of the title compound (80 mg, 48%). H. Res. MS 897.5674 (M+H)⁺.

### Example 193

### Compound of formula (X): X=O, R is -CH₂CH₂NHCH₂CH₂-(3-hydroxyphenyl)

The title compound was prepared by procedures described in Example 187 except substituting 3-hydroxyphenethylamine for phenylpropylamine to give the title compound (69 mg, 40%). MS 897 (M+H)⁺.

### Example 194

### Compound of formula (X): X=O, R is -CH₂CH₂NHCH₂CH₂-(3-methoxyphenyl)

The title compound was prepared by procedures described in Example 187 except substituting 3-methoxyphenethylamine for phenylpropylamine to give the title compound (76 mg, 43%). H. Res. MS 911.5829 (M+H)⁺.

### Example 195

### Compound of formula (X): X=O, R is -CH₂CH₂NHCH₂CH₂-(2-methoxyphenyl)

The title compound was prepared by procedures described in Example 187 except substituting 2-methoxyphenethylamine for phenylpropylamine to give the title compound (36 mg, 20%). H. Res. MS 911.5833 (M+H)⁺.

### Example 196

### Compound of formula (X): X=O, R is -CH₂CH₂NHCH₂CH₂(4-methoxyphenyl)

The title compound was prepared by procedures described in Example 187 except substituting 4-methoxyphenethylamine for phenylpropylamine to give the title compound (78 mg, 44%). H. Res. MS (M+H)⁺.

### Example 197

### Compound of formula (X): X=O, R is -CH₂CH₂NHCH₂-phenyl

The title compound was prepared by procedures described in Example 188 except substituting benzyl amine for L-phenylalanine methyl ester hydrochloride to give the title compound (64 mg, 38%). H. Res. MS 911.5833 (M+H)⁺.

### Example 198: comparative example

### Compound of Formula (X): X is =N-O-(1-isopropoxycyclohexyl), R is fluoromethyl

### Step 198a. Compound of Formula (XII): X is =N-O-(1-isopropoxycyclohexyl), R is fluoromethyl, R^{p} is Trimethylsilyl

To a 0 °C solution of 15 g of the compound of Formula XII where X is =N-O-(1-isopropoxycyclohexyl) and R^{p} is trimethylsilyl in 150 mL of DMSO and 150 mL of THF was added 113.1 g of bromofluoromethane. A solution of potassium t-butoxide (1 M in THF. 25.4 mL) was added dropwise, over 6 hours. The reaction was quenched by addition of allyl amine with stirring for 10 minutes, followed by dilution with water. Ethyl acetate was added, and the organic layer was separated and washed with water and brine, then dried over MgSO₄, filtered and concentrated *in vacuo* to afford 16.5 g of title compound.

### Step 198b. Compound of Formula (XII): X is =N-O-(1-isopropoxyclohexyl), R is fluoromethyl, R^{p} is H

To a room temperature solution of 14.5 g of the compound resulting from step xxxx in 150 mL of anhydrous THF was added 41 mL of 1 M tetrabtitylammonium fluoride. After two hours, the solvent was removed under reduced pressure and the residue was dried to constant weight. Purification by column chromatography eluting with 2% methanol in dichloromethane containing 1% ammonium hydroxide afforded 10.24 g of the title compound.

### Example 199

### Compound of formula (X): X=O, R is -CH₂CH₂NHCH₂CH₂-(3-chlorophenyl)

The tide compound was prepared by procedures described in Example 188 except substituting 3-chlorophenethylamine for L-phenylalanine methyl ester hydrochloride to give the title compound (94 mg, 53%). H. Res. MS 915.5320 (M+H)⁺.

### Example 200

### Compound of formula (X): X=O, R is -CH₂CH₂NH₂CH₂CH₂-(2-chlorophenyl)

The title compound was prepared by procedures described in Example 188 except substituting 2-chlorophenethylamine for L-phenylalanine methyl ester hydrochloride to give the title compound (88 mg, 50%). H. Res. MS 915.5340 (M+H)⁺.

### Example 201

### Compound of formula (X): X=O, R is -CH₂CH₂NHCH₂CH₂-(4-chlorophenyl)

The title compound was prepared by procedures described in Example 188 except substituting 4-chlorophenethylamine for L-phenylalanine methyl ester hydrochloride to give the title compound (84 mg. 47%). H. Res. MS 915.5338 (M+H)⁺.

### Example 202

### Compound of formula (X): X=O, R is -CH₂CH₂NHCH₂CH₂-O-phenyl)

The title compound was prepared by procedures described in Example 188 except substituting 2-phenoxyethylamine for L-phenylalanine methyl ester hydrochloride to give the title compound (71 mg, 41%). H. Res. MS 897.5654 (M+H)⁺.

### Example 203

### Compound of formula (X): X=O, R is -CH₂CH₂NHCH₂CH₂CH₂-(4-quinolinyl)

The title compound was prepared by procedures described in Example 188 except substituting 4-(propylamino)quinoline for L-phenylalanine methyl ester hydrochloride to give the title compound (60 mg. 33%). H. Res. MS 946.5967 (M+H)⁺.

### Example 204

### Compound of formula (X): X=O, R is -CH₂CH₂NHCH₂CH₂CH₂-(3-quinolinyl)

The title compound was prepared by procedures described in Example 188 except substituting 3-(propylamino)quinoline for L-phenylalanine methyl ester hydrochloride to give the title compound. H. Res. MS 946.6022 (M+H)⁺.

### Example 205

### Compound of formula (X): X=O, R is -CH₂CH₂NHCH₂CH₂CH₂CH₂-phenyl

The title compound was prepared by procedures described in Example 188 except substituting 4-phenylbutylamine for L-phenylalanine methyl ester hydrochloride to give the title compound. H. Res. MS 909.6046 (M+H)⁺.

### Example 206

### Compound of formula (X): X=O, R is -CH₂-CH=N-NH-C(O)-NH₂

The title compound was prepared by procedures described in Example 18 except substituting semicarbazide hydrochloride for methoxylamine hydrochloride to give the title compound as a 10:1 anti/syn mixture of isomers. H. Res. MS 833.5153 (M+H)⁺.

### Example 207

### Compound of formula (X): X=O, R is -CH₂-CH=N-NH-(2-pyridinyl)

The title compound was prepared by procedures described in Example 18 except substituting 2-hydrazinopyridine for methoxylamine hydrochloride to give the title compound as a 1:1 anti/syn mixture of isomers. H. Res. MS 867.5351 (M+H)⁺.

### Example 208

### Compound of formula (X): X=O, R is -CH₂-CH=N-(4-methylpiperazinyl)

The title compound was prepared by procedures described in Example 18 except substituting 1-amino-4-methylpiperazine for methoxylamine hydrochloride to give the title compound as a trans isomer. H. Res. MS 873.5765 (M+H)⁺.

### Example 209

### Compound of formula (X): X=O, R is -CH₂-CH=N-O-phenyl

The title compound was prepared by procedures described in Example 18 except substituting O-phenylhydroxylamine hydrochloride for methoxylamine hydrochloride to give the title compound as a 1:1 anti/syn mixture of isomers. H. Res. MS 867.5198 (M+H)⁺.

### Example 210

### Compound of formula (X): X=O, R is -CH₂CH(OH)CH₂NHCH₂CH₂-phenyl

To a solution of the compound from Example 4 (200 mg) in DMF (1 mL) was added benzylamine (0.160 mL), and the reaction was heated at 65 °C for 20 hours. The mixture was diluted with EtOAC (30 mL) and washed successively with water, 5% sodium bicarbonate and brine. The EtOAc layers were then dried over MgSO₄ and concentrated *in vacuo.* The residue was chromatographed on silica gel eluting with dichloromethane/methanol/ammonium hydroxide (96:3:1) to afford of the title compound (109 mg) as a mixture of diastereomers. H. Res. MS 911.5853 (M+H)⁺.

### Example 211

### Compound of formula (X): X=O, R is -CH₂CH(OH)CH₂NHCH₂-(4-pyridinyl

The title compound was prepared by procedures described in Example 210 except substituting 4-(aminoethyl)pyridine for benzylamine to give the title compound (50 mg, 34%). H. Res. MS 898.5635 (M+H)⁺.

### Examples 212 - 221 are comparative examples

### Example 212

### Compound of Formula (VII): W is absent; R^{b} is H; R^{c} is H; R^{d} is H; R^{e} is methoxy; R^{f} is H; R^{g} is H; R is -CH₂-C=N-O-H (anti-isomer)

The title compound was prepared by procedures described in Example 181 substituting hydroxylamine hydrochloride for O-benzylhydroxylamine hydrochloride. The crude product was obtained as a mixture of syn/anti isomers. After chromatography on silica gel eluting with dichloromethane/methanol/ammonium hydroxide (96:3:1) the title compound was obtained as a single anti isomer. H. Res. MS 816.4835 (M+H)⁺.

### Example 213

### Compound of Formula (VII): W is absent; R^{b} is H; R^{c} is H; R^{d} is H; R^{e} is methoxy; R^{f} is H; R^{g} is H; R is -CH₂-C=N-O-H (syn-isomer)

After chromatography of the mixture of syn and anti isomers from Example 212 on silica gel eluting with dichloromethana/methanol/ammonium hydroxide (96:3:1) the title compound was obtained as a single syn isomer. H. Res. MS 816.4835 (M+H)⁺.

### Example 214

### Compound of Formula (VII): W is absent; R^{b} is H; R^{c} is H; R^{d} is H; R^{e} is methoxy; R^{f} is H; R^{g} is H; R is -CH₂-C=N-O-phenyl

The title compound was prepared by procedures described in Example 181 substituting O-phenylhydroxylamine hydrochloride for O-benzylhydroxylamine hydrochloride. The title compound was obtained as a mixture of syn/anti isomers. H. Res. MS 892.5151 (M+H)⁺.

### Example 215

### Compound of Formula (VII): W is absent; R^{b} is H: R^{c} is H; Rd is H; R^{e} is methoxy; Rf is H; R^{g} is H; R is -CH₂-C=N-O-CH₂-(4-nitrophenyl)

The title compound was prepared by procedures described in Example 181 substituting O-(4-nitrobenzyl)hydroxylamine hydrochloride for O-benzylhydroxylamine hydrochloride. The title compound was obtained as a mixture of syn/anti isomers. H. Res. MS 951.5197 (M+H)⁺.

### Example 216

### Compound of Formula (VII): W is absent; R^{b} is H; R^{c} is H; R^{d} is H; R^{e} is methoxy; R^{f} is H; R^{g} is H; R is -CH₂-C=N-O-CH₂(4-quinolinyl)

To a solution of 130 mgs of the title compound of Example 180 in 2 mL MeOH was added 100 mg O-(4-quinolyl)hydroxylamine and catalytic p-toluenesulfonic acid. The reaction was heated at 60°C for 16 hours. The reaction was concentrated *in vacuo.* The residue obtained was chromatographed on silica gel eluting with dichloromethane/methanol/ammonium hydroxide (98:1:1) to afford 85 mg (62%) of the title compound as a mixture of syn/anti isomers. H. Res. MS 957.5443 (M+H)⁺.

### Example 217

### Compound of Formula (VII): W is absent; R^{b} is H; R^{c} is H; R^{d} is H; R^{e} is methoxy; R^{f} is H; R^{g} is H; R is -CH₂-C=N-O-C(phenyl)₃

The title compound was prepared by procedures described in Example 216 except substituting O-tritylhydroxylamine for O-(4-quinolyl)hydroxylamine. The title compound was obtained as a mixture of syn/anti isomers. MS 1058 (M+H)⁺.

### Example 218

### Compound of Formula (VII): W is absent; R^{b} is H; R^{c} is H; R^{d} is H; R^{e} is methoxy; Rf is H; R^{g} is H; R is -CH₂-CH₂-NH₂

A 150 mg sample of the title compound from Example 180 was dissolved in 2 mL of dichloromethane and 24 microliters of benzylamine and 50 mg MgSO₄ were added. The reaction was stirred at ambient tremperature for 16 hours, filtered and concentrated *in vacuo.* The residue obtained was dissolved in EtOH and 120 mg 10% Pd/C was added under a nitrogen atmosphere. The reaction was then placed under 1 atm hydrogen and stirred for 16 hours. The reaction was filtered and concentrated *in vacuo.* The residue obtained was purified with column chromatography on silica gel eluting with dichloromethane/MeOH/ammonium hydroxide (97:2:1) to afford 40 mg of the title compound. MS 802 (M+H)⁺.

### Example 219

### Compound of Formula (VII): W is absent; R^{b} is H; R^{c} is H; R^{d} is H; R^{e} is methoxy; R^{f} is H; R^{g} is H; R is -CH₂-CH₂-NH-CH₂-phenyl

To a solution of 150 mg of the title compound from Example 180, 24 microliters of benzylamine and 34 microliters of acetic acid in 2 mL of MeOH was added 82 mg sodium cyanoborohydride in 3 portions over 5 minutes under a nitrogen atmosphere. The reaction was stirred at ambient temperature for 4.5 hours and was quenched with aqueous 5% sodium bicarbonate. Following an EtOAc extraction and brine wash the organic layer was dried over MgS0₄. The EtOAc extracts were filtered and concentrated *in vacuo.* Purification was performed with column chromatography on silica gel eluting with dichloromethane/methanol/ammonium hydroxide (97:2:1) to give 35 mg (21%) of the title compound. H. Res. MS 892.5526 (M+H)⁺.

### Example 220

### Compound of Formula (VII): W is absent; R^{b} is H; R^{c} is H; R^{d} is H; R^{e} is methoxy; Rf is H; R^{g} is H; R is -CH₂-CH₂-NH-CH₂-CH₂-phenyl

A 200 mg sample of the title compound from Example 180 was dissolved in 2 mL of dichloromethane, and 36 microliters of phenethylamine and 50 mgs of MgSO₄ were added. The reaction was stirred at ambient tremperature for 16 hours, then filtered and concentrated *in vacuo.* The residue obtained was dissolved in EtOH, and 150 mg 10% Pd/C was added under a nitrogen atmosphere. The reaction was then placed under 1 atm hydrogen and stirred for 20 hours. The reaction mixture was filtered and concentrated *in vacuo,* and the residue obtained was purified with column chromatography on silica gel eluting with dichloromethane/MeOH/ammonium hydroxide (97:2:1) to afford 104 mg (46%) of the title compound. H. Res. MS 906.5713 (M+H)⁺.

### Example 221

### Compound of Formula (VII): W is absent; R^{b} is H; R^{c} is H; R^{d} is H; R^{e} is methoxy; R^{f} is H; R^{g} is H; R is -CH₂-CH₂-NH-CH₂-CH₂-CH₂-phenyl

The title compound was prepared by the procedures described in Example 220 except substituting phenylpropylamine for phenethylamine. H. Res. MS 920.5863 (M+H)⁺.

### Example 222

### Compound of Formula (X): X is =O, R is (3-iodophenyl)methyl

### Step 222a. Compound of Formula (XII): X is =N-O-(1-isopropoxycyclohexyl), R is (3-iodophenyl)methyl, R^{p} is Trimethylsilyl

To a 0 °C solution flushed with nitrogen of 33.5 g (0.032 mmol) of the compound of Formula XII where X is =N-O-(1-isopropoxycyclohexyl) and R^{p} is trimethylsilyl in 120 mL of DMSO and 120 mL of THF was added 24 g (0.081 mmol) of 3-iodobenzyl bromide over 10 minutes. A solution of potassium t-butoxide (1 M in THF, 65 mL, 0.065 mmol) was added at 0 °C over 6 hours. The reaction was stirred for an additional hour, then taken up inn ethyl acetate. The organic layer was separated, washed with water and brine, then dried over MgSO₄, filtered and concentrated *in vacuo* to afford 40 g of title compound MS 1249 (M+H)⁺.

### Step 222b. Compound of Formula (XII): X is =N-O-(1-isopropoxycyclohexyl), R is (3-iodophenyl)methyl, R^{p} is H

The compound from step 222a was suspended in acetonitrile (130 mL), and water (65 mL) and acetic acid (65 mL) were added to provide a clear solution. The reaction mixture was stirred at room temperature for 20 hours, and the solvent was removed under reduced pressureto give the title compound (32 g). MS 965 (M+H)⁺.

### Step 222c. Compound of Formula (X): X is =O, R is (3-iodophenyl)methyl

The compound from step 222b (32 g, 0.032 mol) in 500 mL of 1:1 EtOH-water was treated with NaHSO₃ (67.39 g, 0.65 mol) and formic acid (6.11 mL) and warmed at 80°C under nitrogen for 1 hour. The ethanol was removed under vacuum, and the resulting solution was adjusted to pH 10 with sodium carbonate (27.5 g, 0.259 mol) and extracted with EtOAc. The organic phase was washed with water and brine (2x), dried over MgSO4, filtered and concentrated *in vacuo**.* The crude material (9.1 g) was purified by column chromatography eluting with 1% MeOH in dichloromethane containing 1% ammonium hydroxide to give the pure title compound. MS 950 (M+H)⁺.

### Example 223

### Compound of Formula (X): X is =O, R is (4-fluorophenyl)methyl

Following the procedures of Example 222, except substituting 4-fluorobenzyl bromide for the 3-iodobenzyl bromide thereof, the title compound was prepared. MS 842 (M+H)⁺.

### Example 224

### Compound of Formula (X): X is =O, R is -CH₂-CH=CH₂-(3-quinolinyl)

To a sample of the compound from Example 1 (3.09 g, 4 mmol) in acetonitrile (70 mL) were added 3-bromoquinoline (1.08 mL, 8.0 mmol), palladium acetate (180 mg, 0.8 mmol), tri-(o-tolyl)phosphine (365 mg, 1.2 mmol) and triethylamine (1.40 mL, 10 mmol). The mixture was degassed by bubbling N₂ through it for 30 minutes, sealed in a tube under nitrogen, and heated at 60 °C for 1 hour and at 100 °C for 14 hours. The mixture was cooled to room temperature and diluted with ethyl acetate. The organic phase was separated, washed with saturated NaHCO₃ and brine, and dried over MgSO₄. The solvent was removed to give crude product which was purified by chromatography on silica gel, eluting with 40-60% acetone/hexane to give the title compound, (2.73g). MS 901 (M+H)⁺.

### Examples 225 - 283 are comparative examples

### Example 225

### Compound of Formula (II): X is =O, R^{b} is H; R^{c} is H; R^{d} is acetyl; R^{e} is methoxy; R^{f} is acetyl; R is -CH₂-CH=CH₂

To a solution of the compound of Example 1 (80 g, 103 mmol and DMAP (4.0 g, 32.7 mmol) in dichloromethane (200 mL) was added acetic anhydride (40 mL, 400 mmol). The solution was stirred for 5 hours at ambient temperature. The mixture was diluted with dichloromethane (800 mL). The organic phase was washed with 5% Na₂CO₃, saturated NaHCO₃ and brine and dried over MgSO₄. The solvent was removed under vacuum, and the residue was dried. The residue was crystallized from acetonitrile to give the title compound (60.0 g).

### Example 226

### Compound of Formula (VII): W is absent; R^{b} is H; R^{c} is H; R^{d} is acetyl; R^{e} is methoxy; Rf is acetyl; R^{g} is H; R is -CH₂-CH=CH₂

### Step 226a. (Compound (28) of Scheme V: 2'-R^{p} is acetyl; 4"-R^{p} is acetyl; R is allyl

To a solution of the compound of Example 225 (19.5 g, 22.75 mmol) in THF (125 mL) cooled to -48 °C in a dry ice-acetonitrile bath was added sodium bis(trimethylsilyl)amide (30.0 mL, 1 M in THF, 30.0 mmol) over 30 min. After 45 min, a solution of 15.0 g (91.0 mmol) of carbonyldiimidazole in 75 mL of THF and 50 mL of DMF was added. The mixture was stirred for 2.5 hrs at -48 °C and 18 hrs at room temperature. The reaction was quenched by adding a solution of 0.5 M NaH₂PO₄ (200 mL). The product was isolated by extraction of the reaction mixture with ethyl acetate. The extract was dried with MgSO4 and concentrated to give the crude product, which was purified by flash chromatography using 40-60% acetone/hexanes, yielding 19.66 g (92%) of the title compound.

### Step 226b. Compound (29) of Scheme V--which is also a Compound of Formula (VII): W is absent; R^{b} is H; R^{c} is H; R^{d} is acetyl; R^{e} is methoxy; R^{f} is acetyl; R^{g} is H; R is -CH₂-CH=CH₂

To a solution of compound from step 226a (40.0 g, 42.9 mmol) in acetonitrile (1000 mL) and THF (100 mL) was added concentrated ammonium hydroxide (28-30%, 120 mL). The mixture was stirred at room temperature for 7 days. Solvents were removed *in vacuo,* and the residue was taked up in ethyl acetate. The organic layers were washed with brine, dried over MgSO₄ and concentrated *in vacuo.* The residue was chromatographed on silica gel eluting with dichloromethane/MeOH/ammonium hydroxide (10:1:0.05) to give the title compound (23.07 g). HRMS: calculated m/z for (M+H)+: C45H74N2O15: 883.5164; Found: 883.5162.

### Example 227

### Compound of Formula (VII): W is absent; R^{b} is H; R^{c} is H; R^{d}is acetyl; R^{e} is methoxy; R^{f} is acetyl; R^{g} is H; R is -CH₂-CH=CH₂-(3-quinolinyl)

To a sample of the title compound from Example 226 (20.5 g, 23.2 mmol) in acetonitrile (200 mL) were added 3-bromoquinoline (6.47 mL. 31.1 mmol), palladium acetate (1.07 g, 4.76 mmol), tri-(o-tolyl)phosphine (2.43 g, 7.97 mmol) and triethylamine (9.13 mL, 65.5 mmol). The mixture was degassed by bubbling N₂ through for 30 minutes, sealed in a tube under nitrogen, and heated at 60 °C for 1 hour and 14 hours at 100 °C. The mixture was cooled and diluted with ethyl acetate, which was separated and washed with saturated NaHCO₃ and brine, then dried over MgSO₄. The solvents were removed and the crude product was purified by chromatography on silica gel eluting with 40-60% acetone/hexane to give the title compound (21.0 g). MS: [M+H]+ at m/z 883.

### Example 228

### Compound of Formula (VII): W is absent; R^{b} is H; R^{c} is H; R^{d} is acetyl; R^{e} is methoxy; R^{f} is H; R^{g} is H; R is -CH₂CH=CH₂-(3-quinolinyl)

A sample of compound from Example 227 (109 mg, 0.108 mmol) in MeOH (3 mL) was heated at reflux for 4 hours. The solvent was removed by evaporation, and the crude product was purified by chromatography on silica gel with dichloromethane/MeOH/ammonium hydroxide (10:1:0.05) to give the title compound (70 mg). HRMS: calculated m/z for (M+H)+: C₅₂H₇₈N₃O₁₄: 968.5484; Found: 968.5485.

### Example 229

### Compound of Formula (VII): W is absent; R^{b} is H; R^{c} is H; R^{d} is H; R^{e} is methoxy; R^{f} is H; R^{g} is H; R is -CH₂CH=CH₂-(3-Quinolinyl)

A sample of compound from Example 228 (2.53 g, 2.51 mmol) from above in MeOH (50 mL) was added 7.5 mL 2 N NaOH. The mixture was stirred at room temperature for 24 hours before it was diluted with ethyl acetate. The organic layers were washed with brine, dried over MgSO₄ and concentrated *in vacuo.* Chromatography on silica gel eluting with dichloromethane/MeOH/ammonium hydroxide (10:1:0.05) gave the title compound (1.42 g, 61%). H. Res. MS 926.5396.

### Example 230

### Compound of Formula (VII): W is absent; R^{b} is H; R^{c} is H; R^{d} is H; R^{e} is methoxy; R^{f} is acetyl; R^{g} is H; R is -CH₂CH=CH₂-(3-quinolinyl)

A sample of compound from Example 227 (1.42 g, 1.53 mmol) in dichloromethane (30 mL) was treated with triethylamine (0.25 mL, 1.79 mmol) and acetic anhydride (0.29 mL, 2.12 mmol) at room temperature for 12 hours. The mixture was washed with a saturated solution of NaHCO3, dried over MgSO4, and concentrated *in vacuo**.* Crude product was further purifed by recrystalization from hot acetonitrile to give 1.40 g of the title compound.

### Example 231

### Compound of Formula (VII): W is absent; R^{b} is H; R^{c} is H; R^{d} is methoxy; R^{e} is methoxy; R^{f} is H; R^{g} is H; R is -CH₂CH=CH₂-(3-quinolinyl)

A sample of compound from Example 230 (0.1g, 0.103 mmol) in THF (2 mL) at 8 °C was added NaN(TMS)2 (1 M in THF, 0.19 mL). After 30 min. iodomethane (0.027 g, 0.189 mmol) was added. After stirred at room temperature for 12 hours,the mixture was diluted with AcOEt. Organic layers were washed with water, brine, and dried over Na2SO4, concentrated *in vacuo* to give crude product. This material was then dissolved in methanol (3 mL) and heated at reflux for 4 hours. Solvent was removed by evaporation *in vacuo*, residue was purified by chromatography on silica gel eluted with 95:5:1 dichloromethane:MeOH:NH4OH to give title compound.

### Example 232

### Compound of Formula (VII): W is absent; R^{b} is H; R^{c} is H; R^{e} is methoxy; R^{d} is ethenesulfonyloxy; R^{f} is acetyl; R^{g} is H; R is -CH₂CH=CH₂-(3-quinolinyl)

A sample of compound from Example 231 (0.49 g, 0.51 mmol) was dissolved in pyridine (15 mL) and cooled to 0 °C. 2-Chloroethanesulfonyl chloride (0.262 g, 1.61 mmol) was added dropwise, the mixture was stirred at 8 °C for 15 minutes and at room temperature for 48 hours. The mixture was diluted with AcOEt, and washed with 5% NaHCO₃. The aqueous phase was extracted with AcOEt, and the combined organic layers were washed with brine, dried over MgSO₄, filtered and concentraded *in vacuo**.* The crude product was purified by flash chromatography with 95:5:1 dichloromethane:MeOH:NH4OH to give 0.28 g title compound as a yellow foam.

### Example 233

### Compound of Formula (VII): W is absent; R^{b} is H; R^{c} is H; R^{e} is methoxy; Rd is 2-(dimethylamino)ethylsulfonyloxy; R^{f} is acety); R^{g} is H; R is -CH₂CH=CH₂-(3-quinolinyl)

To a sample of compound from Example 232 (70 mg, 0.066 mmol) in CH₃CN (3 mL) was added dimethylamine (2 M in THF, 0.4 mL, 0.079 mmol), and the mixture was stirred for 12 hours at room temperature. The mixture was evaporated to dryness, and the residue was dissolved in 5 mL MeOH and heated at reflux for 4 hours. The solvent was removed by evaporation, and the crude product was purified by chromatography on silica gel with dichloromethane/MeOH/ammonium hydroxide (20:1:0.05) to give the title compound (32 mg). MS m/z 1061 [M+H]⁺.

### Example 234

### Compound of Formula (VII): W is absent; Rb is H; R^{c} is H; R^{d} is methoxy; R^{e} is 2-(phenylthio)ethox; R^{f} is acetyl; R^{g} is H; R is -CH₂CH=CH₂-(3-quinolinyl)

Following the procedure of Example 233, except substituting thiophenol for the dimethylamine thereof, the title compound was prepared (25 mg).

### Example 235

### Compound of Formula (VII): W is absent; R^{b} is H; R^{c} and R^{e} taken together is =O; R^{e} is methoxy; R^{f} is H; R^{g} is H; R is -CH₂CH=CH₂-(3-quinolinyl)

To a solution of N-chlorosuccinimide (110.5 mg, 0.827 mmol) in dichlomethane (3 mL) was added dimethylsulfide (64.3 mg, 1.03 mmol) at -18 °C. After 10 min, a solution of compound from Example 231 (400 mg, 0.414 mmol) in dichlomethane (3 mL) was added. The mixture was stirred at -10 °C to 0 °C for 45 minutes and triethylamine (144 mL, 1.03 mmol) was added. The mixture was diluted with dichlomethane (10 mL), washed with NaHCO₃ and brine, dried over MgSO₄, and concentrated *in vacuo* to give 400 mg of the 2 acetyl compound. A 40 mg sample of this material was heated in refluxing MeOH (3 mL) for 3 hours. Methanol was evaporated, and the residue was purified by chromatography on silica gel eluting with dichloromethane/MeOH/ammonium hydroxide (20:1:0.05) to give the title compound (32 mg). MS: [M+H]+ at m/z 924.

### Example 236

### Compound of Formula (VII): W is absent; R^{b} is H; R^{c} is H; R^{d} is methoxy; R^{e} is (2-nitrophenyl)aminocarbonyloxy; Rf is H; R^{g} is H; R is -CH₂CH=CH₂-(3-quinolinyl)

A sample of the compound from Example 231 (50 mg, 0.054 mmol), 2-nitrophenylisocyanate (13 mg, 0.081 mmol) and DAMP (7.0 mg, 0.057 mmol) in toluene (2 mL) was heated at 108 °C for 3 hrs. After 5 mL MeOH was added, the mixture was heated at reflux for 4 hours. Solvents were removed *in vacuo**,* and the residue was purified by flash chromatography on silica gel eluted with dichloromethane/MeOH/ammonium hydroxide (20:1:0.05) to give the title compound, 38mg. MS: [M+H]+ at m/z 1090.

### Examples 237-283

Using the procedures described in the preceeding examples and schemes and methods known in the synthetic organic chemistry art, the following compounds having the formula (X) wherein R is as described below can be prepared.

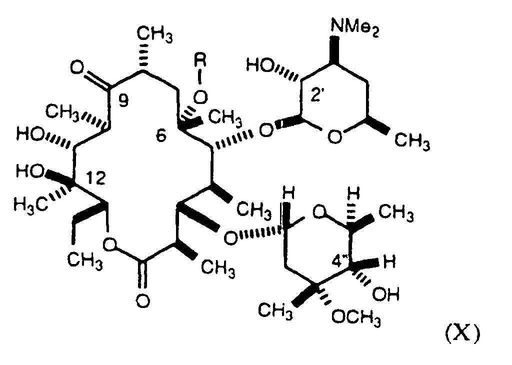

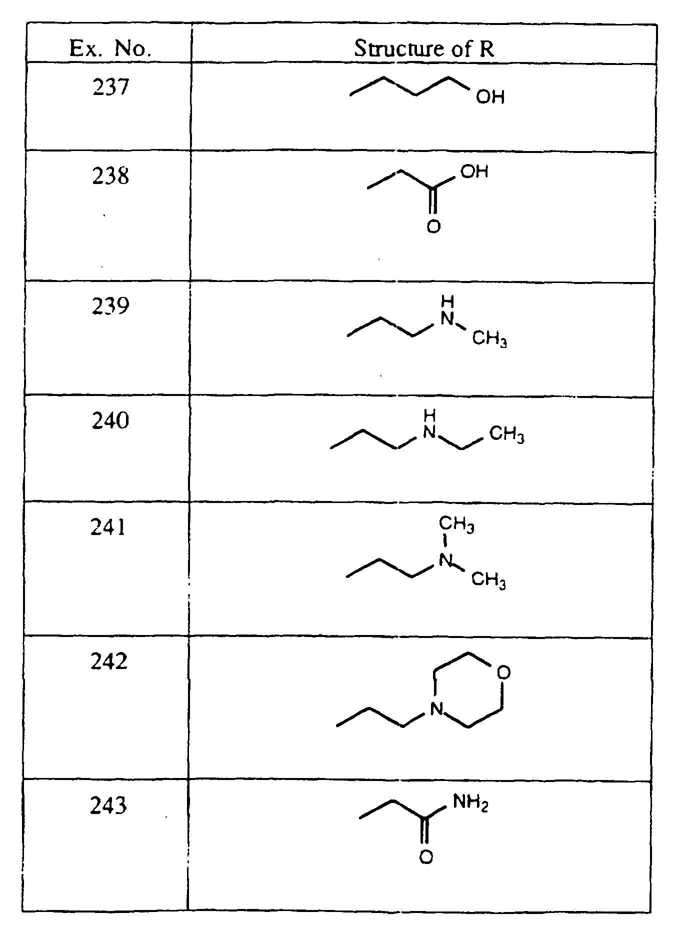

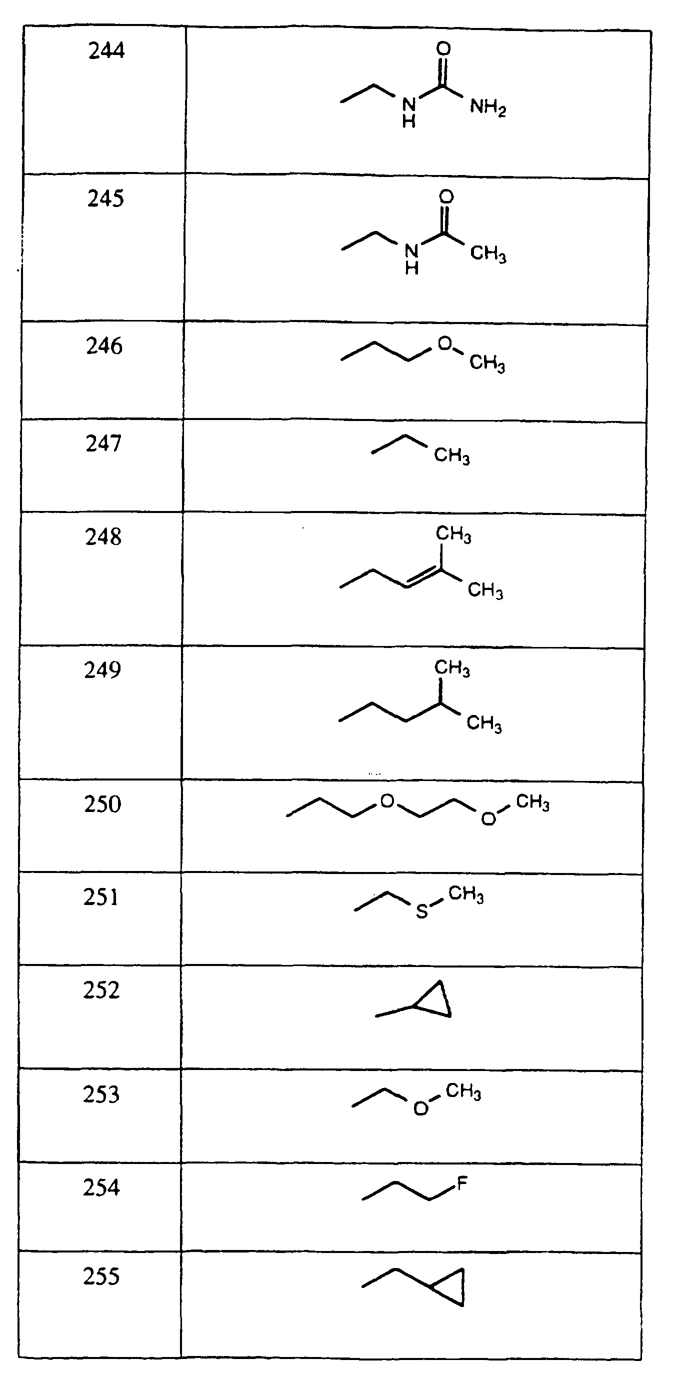

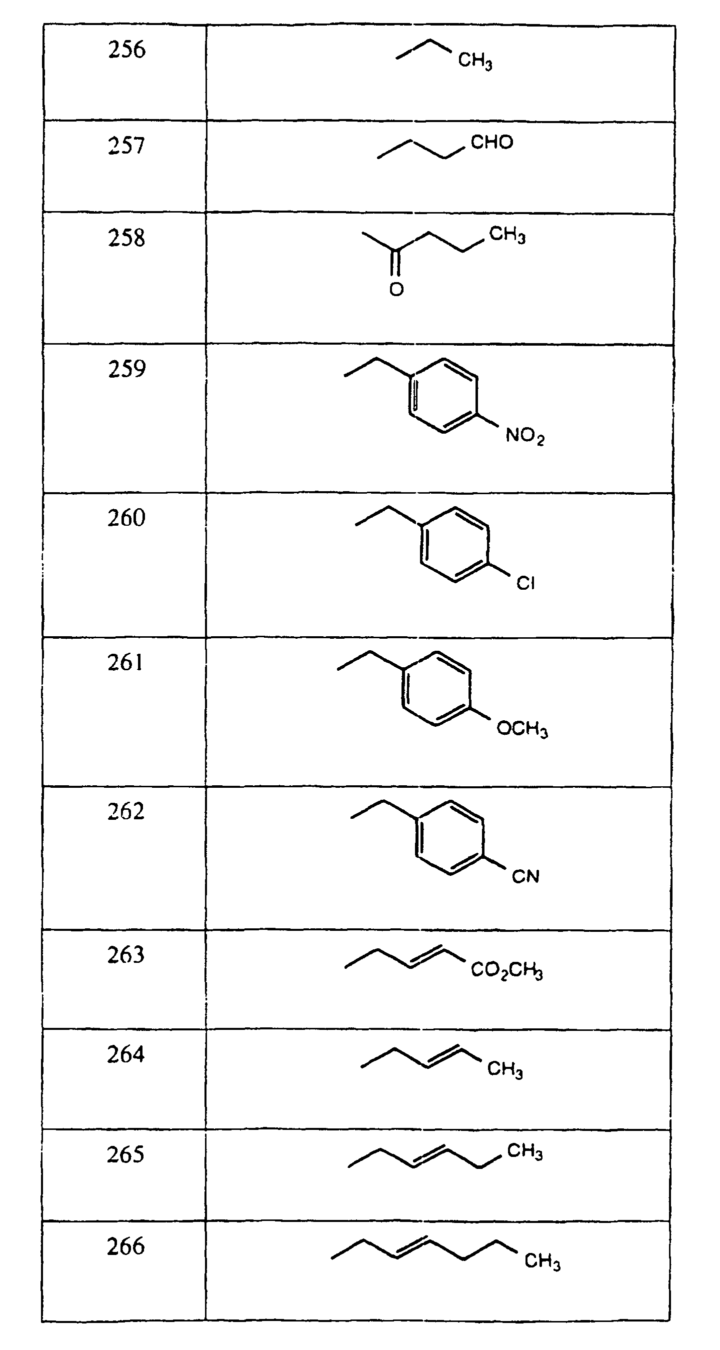

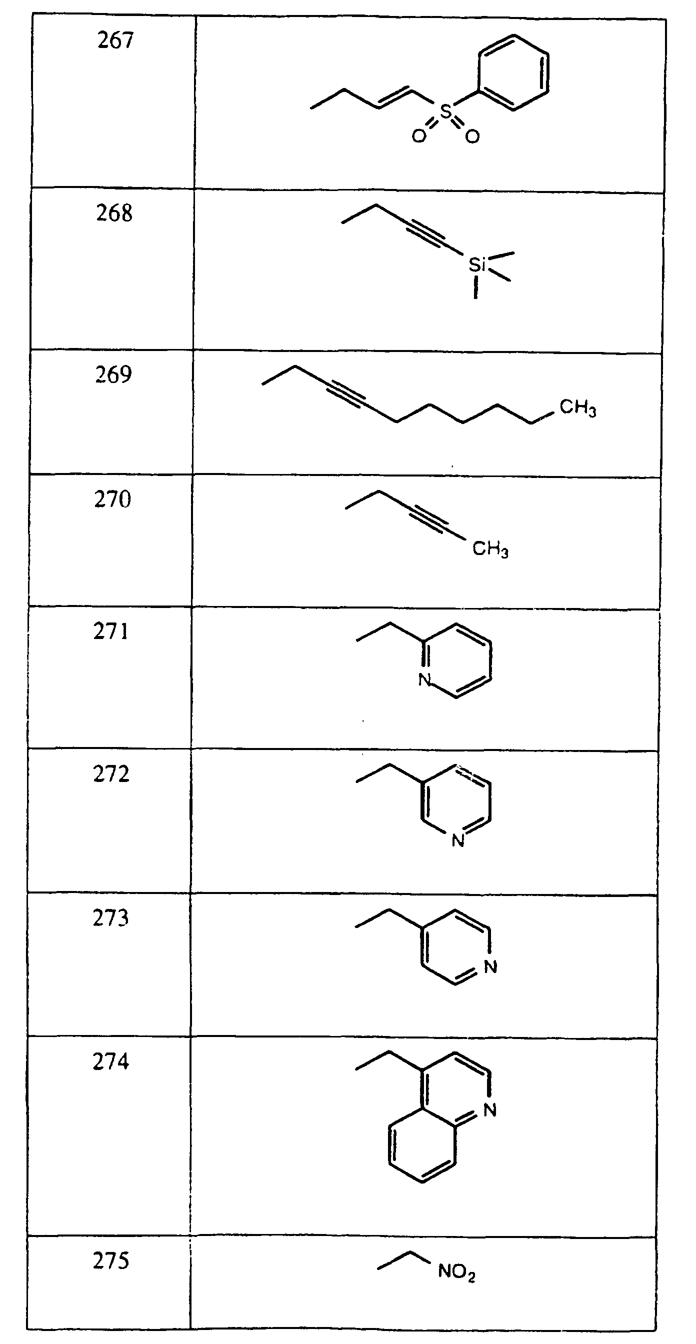

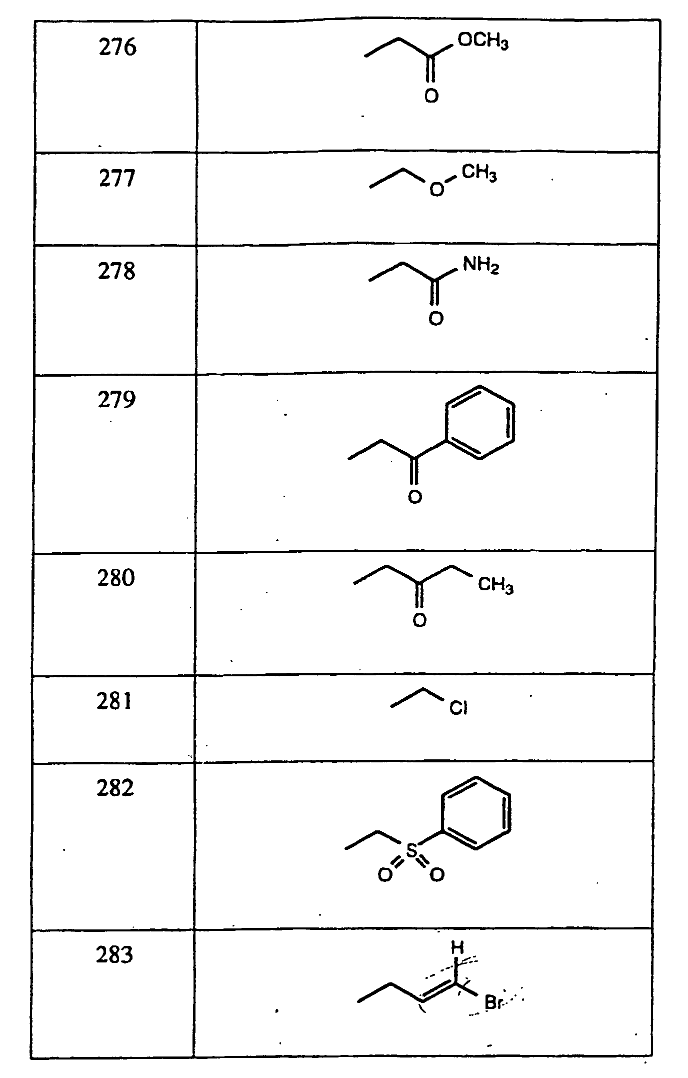

### Example 316

### In Vitro Assay of Antibacterial Activity

Representative compounds of the present invention were assayed *in vitro* for antibacterial activity as follows: Twelve petri dishes containing successive aqueous dilutions of the test compound mixed with 10 mL of sterilized Brain Heart Infusion (BHI) agar (Difco 0418-01-5) were prepared. Each plate was inoculated with 1:100 (or 1:10 for slow-growing strains, such as *Micrococcus* and *Streptococcus*) dilutions of up to 32 different microorganisms, using a Steers replicator block. The inoculated plates were incubated at 35-37 °C for 20 to 24 hours, In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was visually inspected. The minimum inhibitory concentration (MIC) was defined as the lowest concentration of drug yielding no growth, a slight haze, or sparsely isolated colonies on the inoculum spot as compared to the growth control. The results of this assay, shown below in Table 4 demonstrate the antibacterial activity of the compounds of the invention.

**Table 4**

| Antibacterial Activity (MIC's) of Selected Compounds | | | |
|---|---|---|---|
| Microorganism | Ery. A | Example 1B | Example 1C |
| *Staphylococcus aureus* ATCC 6538P | 0.2 | 0.78 | 0.78 |
| *Staphylococcus aureus* A5177 | 3.1 | 12.5 | 12.5 |
| *Staphylococcus aureus* A-5278 | > 100 | > 100 | > 100 |
| *Staphylococcus aureus* CMX 642A | 0.39 | 1.56 | 1.56 |
| *Staphylococcus aureus* NCTC10649M | 0.39 | 3.1 | 0.78 |
| *Staphylococcus aureus* CMX 553 | 0.39 | 1.56 | 0.78 |
| *Staphylococcus aureus* 1775 | > 100 | > 100 | > 100 |
| *Staphylococcus epidermidis* 3519 | 0.39 | 0.39 | 0.39 |
| *Enterococcus faecium* ATCC 8043 | 0.05 | 0.2 | 0.2 |
| *Streptococcus bovis* A-5169 | 0.02 | 0.02 | 0.01 |
| *Streptococcus agalactiae* CMX 508 | 0.05 | 0.1 | 0.01 |
| *Streptococcus pyogenes* EES61 | - | - | - |
| *Streptococcus pyogenes* 930 | > 100 | > 100 | > 100 |
| *Streptococcus pyogenes* PIU 2548 | 3.1 | 6.2 | 3.1 |
| *Micrococcus luteus* ATCC 9341 | 0.05 | 0.2 | 0.1 |
| *Micrococcus luteus* ATCC 4698 | 0.2 | 3.1 | 1.56 |
| *Escherichia coli* JUHL | > 100 | > 100 | 100 |
| *Escherichia coli* SS | 0.78 | 3.1 | 0.78 |
| *Escherichia coli* DC-2 | > 100 | > 100 | > 100 |
| *Escherichia coli* H560 | 50 | 100 | 100 |
| *Escherichia coli* KNK 437 | 100 | > 100 | > 100 |
| *Enterobacter aerogenes* ATCC 13048 | > 100 | > 100 | > 100 |
| *Klebsiella pneumoniae* ATCC 8045 | > 100 | > 100 | > 100 |
| *Providencia struartii* CMX 640 | > 100 | > 100 | > 100 |
| *Pseudomonas aeruginosa* BMH10 | > 100 | > 100 | > 100 |
| *Pseudomonas aeruginosa* 5007 | > 100 | > 100 | > 100 |
| *Pseudomonas aeruginosa* K799/WT | 100 | > 100 | > 100 |
| *Pseudomonas aeruginosa* K799/61 | 1.56 | 6.2 | 6.2 |
| *Pseudomonas capacia* 2961 | > 100 | > 100 | > 100 |
| *Actinetobacter calcoaceticus* CMX 669 | 12.5 | 50 | 50 |
| *Pseudomonas aeruginosa* DPHD-5263 | >100 | > 100 | > 100 |
| *Pseudomonas aeruginosa* DPHD-2862 | > 100 | > 100 | > 100 |
| *Candida albicans* CCH 442 | > 100 | > 100 | > 100 |
| *Mycobacterium smegmatis* ATCC 114 | 3.1 | - | 0.2 |

**Table 2**

| Antibacterial Activity (MIC's) of Selected Compounds Continued wherein compound of Example 2 is a comparative compound | | | |
|---|---|---|---|
| Microorganism | Ery. A | Example 2 | Example 3 |
| *Staphylococcus aureus* ATCC 6538P | 0.2 | 3.1 | 1.56 |
| *Staphylococcus aureus* A5177 | 3.1 | 25 | 50 |
| *Staphylococcus aureus* A-5278 | > 100 | > 100 | > 100 |
| *Staphylococcus aureus* CMX 642A | 0.39 | 12.5 | 1.56 |
| *Staphylococcus aureus* NCTC10649M | 0.39 | 6.2 | 1.56 |
| *Staphylococcus aureus* CMX 553 | 0.39 | 3.1 | 3.1 |
| *Staphylococcus aureus* 1775 | > 100 | > 100 | > 100 |
| *Staphylococcus epidermidis* 3519 | 0.39 | 1.56 | 0.78 |
| *Enterococcus faecium* ATCC 8043 | 0.05 | 3.1 | 0.39 |
| *Streptococcus bovis* A-5169 | 0.02 | 0.05 | 0.2 |
| *Streptococcus agalactiae* CMX 508 | 0.05 | 0.2 | 0.39 |
| *Streptococcus pyogenes* EES61 | - | 0.05 | 0.2 |
| *Streptococcus pyogenes* 930 | > 100 | >100 | > 100 |
| *Streptococcus pyogenes* PIU 2548 | 3.1 | 6.2 | 12.5 |
| *Micrococcus luteus* ATCC.9341 | 0.05 | 0.2 | 0.1 |
| *Micrococcus luteus* ATCC 4698 | 0.2 | 3.1 | 1.56 |
| *Escherichia coli* JUHL | >100 | >100 | 50 |
| *Escherichia coli* SS | 0.78 | 1.56 | 1.56 |
| *Escherichia coli* DC-2 | > 100 | > 100 | 50 |
| *Escherichia coli* H560 | 50 | >100 | 25 |
| *Escherichia coli* KNK 437 | 100 | >100 | 100 |
| *Enterobacter aerogenes* ATCC 13048 | > 100 | > 100 | > 100 |
| *Klebsiella pneumoniae* ATCC 8045 | > 100 | >100 | > 100 |
| *Providencia struartii* CMX 640 | > 100 | >100 | > 100 |
| *Pseudomonas aeruginosa* BMH10 | >100 | >100 | >100 |
| *Pseudomonas aeruginosa* 5007 | > 100 | > 100 | > 100 |
| *Pseudomonas aeruginosa* K799/WT | 100 | 6.2 | >100 |
| *Pseudomonas aeruginosa* K799/61 | 1.56 | >100 | 1.56 |
| *Pseudomonas capacia* 2961 | >100 | 50 | >100 |
| *Actinetobacter calcoaceticus* CMX 669 | 12.5 | >100 | 12.5 |
| *Pseudomonas aeruginosa* DPHD-5263 | > 100 | >100 | > 100 |
| *Pseudomonas aeruginosa* DPHD-2862 | > 100 | > 100 | > 100 |
| *Candida albicans* CCH 442 | > 100 | 0.1 | > 100 |
| *Mycobacterium smegmatis* ATCC 114 | 3.1 - | | 0.78 |

**Table 2**

| Antibacterial Activity (MIC's) of Selected Compounds Continued | | | |
|---|---|---|---|
| Microorganism | Ery. A | Example 5 | Example 6 |
| *Staphylococcus aureus* ATCC 6538P | 0.2 | 0.78 | 0.78 |
| *Staphylococcus aureus* A5177 | 3.1 | 12.5 | 25 |
| *Staphylococcus aureus* A-5278 | > 100 | > 100 | >100 |
| *Staphylococcus aureus* CMX 642A | 0.39 | 0.78 | 1.56 |
| *Staphylococcus aureus* NCTC10649M | 0.39 | 0.78 | 0.78 |
| *Staphylococcus aureus* CMX 553 | 0.39 | 0.78 | 0.78 |
| *Staphylococcus aureus* 1775 | > 100 | > 100 | > 100 |
| *Staphylococcus epidermidis* 3519 | 0.39 | 1.56 | 0.78 |
| *Enterococcus faecium* ATCC 8043 | 0.05 | 0.39 | 0.39 |
| *Streptococcus bovis* A-5169 | 0.02 | 0.05 | - |
| *Streptococcus agalactiae* CMX 508 | 0.05 | 0.1 | 0.1 |
| *Streptococcus pyogenes* EES61 | - | 0.05 | 0.05 |
| *Streptococcus pyogenes* 930 | > 100 | > 100 | > 100 |
| *Streptococcus pyogenes* PIU 2548 | 3.1 | - | 6.2 |
| *Micrococcus luteus* ATCC 9341 | 0.05 | 0.1 | 0.1 |
| *Micrococcus luteus* ATCC 4698 | 0.2 | 3.1 | 1.56 |
| *Escherichia coli* JUHL | > 100 | 25 | > 100 |
| *Escherichia coli* SS | 0.78 | 0.78 | 0.78 |
| *Escherichia coli* DC-2 | > 100 | 50 | > 100 |
| *Escherichia coli* H560 | 50 | 50 | 100 |
| *Escherichia coli* KNK 437 | 100 | 50 | >100 |
| *Enterobacter aerogenes* ATCC 13048 | > 100 | 100 | > 100 |
| *Klebsiella pneumoniae* ATCC 8045 | > 100 | 100 | > 100 |
| *Providencia struartii* CMX 640 | > 100 | > 100 | > 100 |
| *Pseudomonas aeruginosa* BMH 10 | > 100 | 100 | > 100 |
| *Pseudomonas aeruginosa* 5007 | > 100 | >100 | > 100 |
| *Pseudomonas aeruginosa* K799/WT | 100 | >100 | >100 |
| *Pseudomonas aeruginosa* K799/61 | 1.56 | 1.56 | 12.5 |
| *Pseudomonas capacia* 2961 | >100 | > 100 | > 100 |
| *Actinetobacter calcoaceticus* CMX 669 | 12.5 | 12.5 | 25 |
| *Pseudomonas aeruginosa* DPHD-5263 | >100 | > 100 | > 100 |
| *Pseudomonas aeruginosa* DPHD-2862 | > 100 | > 100 | >100 |
| *Candida albicans* CCH 442 | > 100 | > 100 | > 100 |
| *Mycobacterium smegmatis* ATCC 114 | 3.1 | 1.56 | 0.2 |

**Table 2**

| Antibacterial Activity (MIC's) of Selected Compounds Continued | | | |
|---|---|---|---|
| Microorganism | Ery. A | Example 7 | Example 8 |
| *Staphylococcus aureus* ATCC 6538P | 0.2 | 0.39 | 0.78 |
| *Staphylococcus aureus* A5177 | 3.1 | 3.1 | 12.5 |
| *Staphylococcus aureus* A-5278 | > 100 | > 100 | > 100 |
| *Staphylococcus aureus* CMX 642A | 0.39 | 0.39 | 0.78 |
| *Staphylococcus aureus* NCTC10649M | 0.39 | 0.39 | 0.78 |
| *Staphylococcus aureus* CMX 553 | 0.39 | 0.39 | 0.78 |
| *Staphylococcus aureus* 1775 | > 100 | > 100 | > 100 |
| *Staphylococcus epidermidis* 3519 | 0.39 | 0.39 | 0.78 |
| *Enterococcus faecium* ATCC 8043 | 0.05 | 0.39 | 0.39 |
| *Streptococcus bovis* A-5169 | 0.02 | 0.01 | 0.2 |
| *Streptococcus agalactiae* CMX 508 | 0.05 | 0.01 | 0.39 |
| *Streptococcus pyogenes* EES61 | - | 0.01 | 0.1 |
| *Streptococcus pyogenes* 930 | > 100 | > 100 | >100 |
| *Streptococcus pyogenes* PIU 2548 | 3.1 | - | 25 |
| *Micrococcus luteus* ATCC 9341 | 0.05 | 0.02 | 0.1 |
| *Micrococcus luteus* ATCC 4698 | 0.2 | 0.78 | 1.56 |
| *Escherichia coli* JUHL | > 100 | 12.5 | 100 |
| *Escherichia coli* SS | 0.78 | 0.2 | 1.56 |
| *Escherichia coli* DC-2 | > 100 | 6.2 | > 100 |
| *Escherichia coli* H560 | 50 | 1.56 | 50 |
| *Escherichia coli* KNK 437 | 100 | 12.5 | > 100 |
| *Enterobacter aerogenes* ATCC 13048 | > 100 | 50 | >100 |
| *Klebsiella pneumoniae* ATCC 8045 | > 100 | 25 | >100 |
| *Providencia struartii* CMX 640 | > 100 | > 100 | > 100 |
| *Pseudomonas aeruginosa* BMH10 | > 100 | 25 | > 100 |
| *Pseudomonas aeruginosa* 5007 | > 100 | > 100 | > 100 |
| *Pseudomonas aeruginosa* K799AVT | 100 | 100 | > 100 |
| *Pseudomonas aeruginosa* K799/61 | 1.56 | 0.39 | 3.1 |
| *Pseudomonas capacia* 2961 | > 100 | > 100 | > 100 |
| *Actinetobacter calcoaceticus* CMX 669 | 12.5 | 12.5 | 50 |
| *Pseudomonas aeruginosa* DPHD-5263 | > 100 | > 100 | > 100 |
| *Pseudomonas aeruginosa* DPHD-2862 | > 100 | > 100 | > 100 |
| *Candida albicans* CCH 442 | > 100 | > 100 | > 100 |
| *Mycobacterium smegmatis* ATCC 114 | 3.1 | 0.2 | 6.2 |
| | | | 0.39 |

**Table 2**

| Antibacterial Active (MIC's) of Selected Compounds Continued | | | |
|---|---|---|---|
| Microorganism | Ery. A | Example 9 | Example 10 |
| *Staphylococcus aureus* ATCC 6538P | 0.2 | 3.1 | 0.2 |
| *Staphylococcus aureus* A5177 | 3.1 | - | 6.2 |
| *Staphylococcus aureus* A-5278 | > 100 | > 100 | > 100 |
| *Staphylococcus aureus* CMX 642A | 0.39 | 3.1 | 0.39 |
| *Staphylococcus aureus* NCTC10649M | 0.39 | 3.1 | 0.39 |
| *Staphylococcus aureus* CMX 553 | 0.39 | - | 0.39 |
| *Staphylococcus aureus* 1775 | > 100 | > 100 | > 100 |
| *Staphylococcus epidermidis* 3519 | 0.39 | 3.1 | 0.2 |
| *Enterococcus faecium* ATCC 8043 | 0.05 | 3.1 | 0.1 |
| *Streptococcus bovis* A-5169 | 0.02 | 3.1 | 0.01 |
| *Streptococcus agalactiae* CMX 508 | 0.05 | 3.1 | 0.05 |
| *Streptococcus pyogenes* EES61 | - | - | 0.01 |
| *Streptococcus pyogenes* 930 | > 100 | > 100 | > 100 |
| *Streptococcus pyogenes* PIU 2548 | 3.1 | 12.5 | - |
| *Micrococcus luteus* ATCC 9341 | 0.05 | 3.1 | 0.05 |
| *Micrococcus luteus* ATCC 4698 | 0.2 | - | 0.78 |
| *Escherichia coli* JUHL | > 100 | > 100 | 50 |
| *Escherichia coli* SS | 0.78 | - | 0.78 |
| *Escherichia coli* DC-2 | > 100 | - | 100 |
| *Escherichia coli* H560 | 50 | > 100 | 25 |
| *Escherichia coli* KNK 437 | 100 | > 100 | > 100 |
| *Enterobacter aerogenes* ATCC 13048 | > 100 | > 100 | > 100 |
| *Klebsiella pneumoniae* ATCC 8045 | > 100 | > 100 | > 100 |
| *Providencia struartii* CMX 640 | > 100 | > 100 | > 100 |
| *Pseudomonas aeruginosa* BMH10 | > 100 | > 100 | 100 |
| *Pseudomonas aeruginosa* 5007 | > 100 | > 100 | > 100 |
| *Pseudomonas aeruginosa* K799/WT | 100 | > 100 | > 100 |
| *Pseudomonas aeruginosa* K799/61 | 1.56 | 12.5 | 3.1 |
| *Pseudomonas capacia* 2961 | > 100 | > 100 | >100 |
| *Actinetobacter calcoaceticus* CMX 669 | 12.5 | 50 | 12.5 |
| *Pseudomonas aeruginosa* DPHD-5263 | > 100 | > 100 | > 100 |
| *Pseudomonas aeruginosa* DPHD-2862 | > 100 | > 100 | > 100 |
| *Candida albicans* CCH 442 | > 100 | > 100 | > 100 |
| *Mycobacterium smegmatis* ATCC 114 | 3.1 | 3.1 | 0.39 |
| *Nocarrdia asteroides* ATCC 9970 | | | |

**Table 2**

| Antibacterial Activity (MIC's) of Selected Compounds Continued | | | |
|---|---|---|---|
| Microorganism | Ery. A | Example 11 | Example 12 |
| *Staphylococcus aureus* ATCC 6538P | 0.2 | 0.78 | 0.39 |
| *Staphylococcus aureus* A5177 | 3.1 | 6.2 | - |
| *Staphylococcus aureus* A-5278 | > 100 | > 100 | > 100 |
| *Staphylococcus aureus* CMX 642A | 0.39 | 1.56 | 0.39 |
| *Staphylococcus aureus* NCTC10649M | 0.39 | 0.78 | 0.39 |
| *Staphylococcus aureus* CMX 553 | 0.39 | 0.78 | 0.39 |
| *Staphylococcus aureus* 1775 | > 100 | > 100 | > 100 |
| *Staphylococcus epidermidis* 3519 | 0.39 | 0.78 | 0.39 |
| *Enterococcus faecium* ATCC 8043 | 0.05 | 0.39 | 0.1 |
| *Streptococcus bovis* A-5169 | 0.02 | 0.2 | 0.05 |
| *Streptococcus agalactiae* CMX 508 | 0.05 | 0.05 | 0.05 |
| *Streptococcus pyogenes* EES61 | - | 0.05 | 0.05 |
| *Streptococcus pyogenes* 930 | > 100 | > 100 | > 100 |
| *Streptococcus pyogenes* PIU 2548 | 3.1 | 25 | 12.5 |
| *Micrococcus luteus* ATCC 9341 | 0.05 | 0.05 | 0.05 |
| *Micrococcus luteus* ATCC 4698 | 0.2 | - | 0.78 |
| *Escherichia coli* JUHL | > 100 | 100 | 50 |
| *Escherichia coli* SS | 0.78 | 1.56 | 0.78 |
| *Escherichia coli* DC-2 | > 100 | > 100 | > 100 |
| *Escherichia coli* H560 | 50 | 50 | 25 |
| *Escherichia coli* KNK 437 | 100 | > 100 | 100 |
| *Enterobacter aerogenes* ATCC 13048 | > 100 | > 100 | > 100 |
| *Klebsiella pneumoniae* ATCC 8045 | > 100 | > 100 | - |
| *Providencia struartii* CMX 640 | > 100 | > 100 | > 100 |
| *Pseudomonas aeruginosa* BMH10 | > 100 | > 100 | > 100 |
| *Pseudomonas aeruginosa* 5007 | > 100 | > 100 | > 100 |
| *Pseudomonas aeruginosa* K799/WT | 100 | > 100 | > 100 |
| *Pseudomonas aeruginosa* K799/61 | 1.56 | - | 1.56 |
| *Pseudomonas capacia* 296I | > 100 | > 100 | > 100 |
| *Actinetobacter calcoaceticus* CMX 669 | 12.5 | 25 | 12.5 |
| *Pseudomonas aeruginosa* DPHD-5263 | > 100 | > 100 | > 100 |
| *Pseudomonas aeruginosa* DPHD-2862 | > 100 | > 100 | > 100 |
| *Candida albicans* CCH 442 | > 100 | > 100 | > 100 |
| *Mycobacterium smegmatis* ATCC 114 | 3.1 | 0.39 | 0.78 |
| *Nocarrdia asteroides* ATCC 9970 | | 0.1 | |

## Claims

1. A compound having the formula 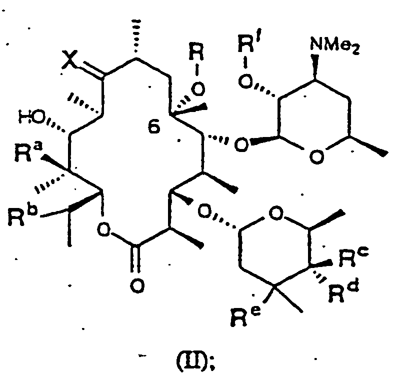 or a pharmaceutically acceptable salt thereof, wherein
X is O,
Ra is hydroxy;
Rb is hydrogen;
Rc is hydrogen;
Rd is hydroxy,
Re is methoxy;
Rf is hydrogen; and
R is selected from the group consisting of
(2) C₂-C₁₀-alkyl substituted with one substituent selected from the group consisting of
(a) halogen,
(b) hydroxy,
(c) C₁-C₃-alkoxy,
(d) C₁-C₃-alkoxy-C₁-C₃-alkoxy,
(e) oxo,
(f) -N₃,
(g) -CHO,
(h) O-SO₂-(substituted C₁-C₆-alkyl),
(i) -NR¹⁵R¹⁶ wherein R¹⁵ and R¹⁶ are selected from the group consisting of
(i) hydrogen,
(ii) C₁-C₁₂-alkyl,
(iii) substituted C₁-C₁₂-alkyl,
(iv) C₁-C₁₂-alkenyl,
(v) substituted C₁-C₁₂-alkenyl,
(vi) C₁-C₁₂-alkynyl,
(vii) substituted C₁-C₁₂-alkynyl,
(viii) aryl, (ix) C₃-C₈-cycloalkyl, (x) substituted C₃-C₈-cycloalkyl, (xi) substituted aryl,
(xii) heterocycloalkyl,
(xiii) substituted heterocycloalkyl,
(xiv) C₁-C₁₂-alkyl substituted with aryl,
(xv) C₁-C₁₂-alkyl substituted with substituted aryl,
(xvi) C₁-C₁₂-alkyl substituted with heterocycloalkyl,
(xvii) C₁-C₁₂-alkyl substituted with substituted heterocycloalkyl,
(xviii) C₁-C₁₂-alkyl substituted with C₃-C₈-cycloalkyl,
(xix) C₁-C₁₂-alkyl substituted with substituted C₃-C₈-cycloalkyl,
(xx) heteroaryl,
(xxi) substituted heteroaryl,
(xxii) C₁-C₁₂-alkyl substituted with heteroaryl, and
(xxiii) C₁-C₁₂-alkyl substituted with substituted heteroaryl,
or
R¹⁵ and R¹⁶ are taken together with the atom to which they are attached form morpholinyl;
(3) C₄-C₁₀-alkenyl; and
(4) C₃-C₁₀-alkynyl.

2. A compound according to claim 1 which is selected from the group consisting of compounds wherein
(1) R is -CH2-CH=CH2-(3-quinolinyl);
(2) R is allyl;
(4) R is 2,3-dihydroxypropyl;
(5) R is 2,3-epoxypropyl;
(6) R is 2-hydroxy-3-(imidazol-1-yl)propyl;
(7) R is 2-hydroxy-3-(morpholin-4-yl)propyl;
(8) R is 2-hydroxy-3-(benzylamino)propyl;
(9) R is 2-oxoethyl;
(10) R is 2-oxopropyl;
(11) R is -CH2-C≡CH;
(12) R is -CH2-CHOH-CH2-N3 ;
(13) R is -CH2-CH=N-OH;
(14) R is -CH2-CH2OH;
(15) R is -CH2-CH2NH2 ;
(16) R is -CH2-CN;
(17) R is -CH2-Phenyl;
(18) R is -CH2-CH=CH-Phenyl-;
(19) R is -CH2-CH=N-O-CH3 ;
(20) R is -CH2-CH=N-O-CH2-Phenyl;
(21) R is -CH2-CH=N-N(CH3)2 ;
(22) R is -CH2-CH=N-NH(CH3);
(23) R is -CH2-CH=N-(4-Morpholinyl);
(24) R is -CH2-CH=N-NH(Phenyl); and
(25) R is -CH2-CH=N-N(Phenyl)2 ;
(26) R is Phenylpropyl;
(27) R is -CH2CH=CH-(4-methylphenyl);
(28) R is -CH2-CH(OH)-Phenyl;
(29) R is -CH2-CH(Br)-CH2Br;
(30) R is -CH2CH2NHCH2CH2CH2-Phenyl;
(31) R is -CH2CH2 NHCH(CH2 Phenyl)CO2Me;
(32) R is -CH2CH2NHCH2CH2CH3;
(33) R is -CH2CH2NHCH2CO2CH2CH2;
(34) R is -CH2CH2NHCH2CH2-Phenyl;
(35) R is -CH2CH2NHCH2CH2-(4-hydroxyphenyl);
(36) R is -CH2CH2NHCH2CH2-(3-hydroxyphenyl);
(37) R is -CH2CH2NHCH2CH2-(3-methoxyphenyl);
(38) R is -CH2CH2NHCH2CH2-(2-methoxyphenyl);
(39) R is -CH2CH2NHCH2CH2-(4-methoxyphenyl);
(40) R is -CH2CH2NHCH2-phenyl;
(41) R is -CH2CH2NHCH2CH2-(3-chlorophenyl);
(42) R is -CH2CH2NHCH2CH2-(2-chlorophenyl);
(43) R is -CH2CH2NHCH2CH2-(4-chlorophenyl);
(44) R is -CH2CH2NHCH2CH2-O-phenyl);
(45) R is -CH2CH2NHCH2CH2CH2-(4-quinolinyl);
(46) R is -CH2CH2NHCH2CH2CH2-(3-quinolinyl);
(47) R is -CH2CH2NHCH2CH2CH2CH2-phenyl;
(48) R is -CH2-CH=N-NH-C(O)-NH2 ;
(49) R is -CH2-CH=N-NH-(2-pyridinyl);
(50) R is -CH2=CH=N-(4-methylpiperazinyl);
(51) R is -CH2-CH=N-O-phenyl;
(52) R is -CH2CH(OH)CH2NHCH2CH2-phenyl;
(53) R is -CH2CH(OH)CH2NHCH2-(4-pyridinyl);
(54) R is (3-iodophenyl)methyl; and
(55) R is (4-fluorophenyl)methyl.

3. A compound according to claim 2 wherein
R is -CH2 -CH=CH2-(3-quinolinyl);
R is allyl;
R is 2-oxopropyl;
R is -CH2-C≡H;
R is -CH2-CH=N-OH;
R is -CH2-CH2OH;
R is -CH2-CH2NH2 ; and
R is -CH2-CN.

4. A pharmaceutical composition comprising a therapeutically effective amount of a compound of any one of claims 1, 2 and 3 in combination with a pharmaceutically acceptable carrier.

5. Use of a compound according to claims 1, 2 or 3 for manufacturing a medicament for controlling a bacterial infection in a mammal.

6. Compound according to claims 1, 2 or 3 for use as a medicament.

## Patentansprüche

1. Eine Verbindung, welche die folgende Formel hat 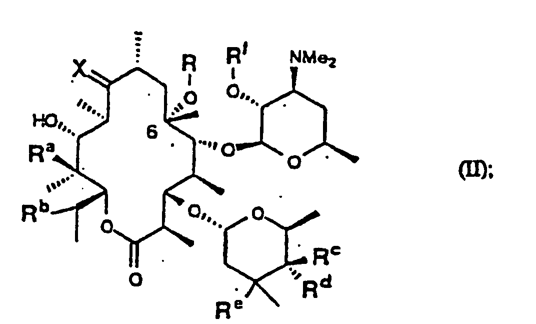 oder ein pharmazeutisch verträgliches Salz davon, worin
X O ist,
Ra Hydroxy ist;
Rb Wasserstoff ist;
Rc Wasserstoff ist;
Rd Hydroxy ist,
Re Methoxy ist;
Rf Wasserstoff ist; und
R gewählt ist aus der Gruppe bestehend aus
(2) C₂-C₁₀-Alkyl substituiert mit einem Substituenten gewählt aus der Gruppe bestehend aus
(a) Halogen,
(b) Hydroxy,
(c) C₁-C₃-Alkoxy,
(d) C₁-C₃-Alkoxy-C₁-C₃-alkoxy,
(e) Oxo,
(f) -N₃,
(g) -CHO,
(h) O-SO₂-(substituiertes C₁-C₆-Alkyl),
(i) -NR¹⁵R¹⁶, worin R¹⁵ und R¹⁶ gewählt sind aus der Gruppe bestehend aus
(i) Wasserstoff,
(ii) C₁-C₁₂-Alkyl,
(iii) substituiertem C₁-C₁₂-Alkyl,
(iv) C₁-C₁₂-Alkenyl,
(v) substituiertem C₁-C₁₂-Alkenyl,
(vi) C₁-C₁₂-Alkinyl,
(vii) substituiertem C₁-C₁₂-Alkinyl,
(viii) Aryl,
(ix) C₃-C₈-Cycloalkyl,
(x) substituiertem C₃-C₈-Cycloalkyl,
(xi) substituiertem Aryl,
(xii) Heterocycloalkyl,
(xiii) substituiertem Heterocycloalkyl,
(xiv) C₁-C₁₂-Alkyl substituiert mit Aryl,
(xv) C₁-C₁₂-Alkyl substituiert mit substituiertem Aryl,
(xvi) C₁-C₁₂-Alkyl substituiert mit Heterocycloalkyl,
(xvii) C₁-C₁₂-Alkyl substituiert mit substituiertem Heterocycloalkyl,
(xviii) C₁-C₁₂-Alkyl substituiert mit C₃-C₈-Cycloalkyl,
(xix) C₁-C₁₂-Alkyl substituiert mit substituiertem C₃-C₈-Cycloalkyl,
(xx) Heteroaryl,
(xxi) substituiertem Heteroaryl,
(xxii) C₁-C₁₂-Alkyl substituiert mit Heteroaryl, und
(xxiii) C₁-C₁₂-Alkyl substituiert mit substituiertem Heteroaryl,
oder
R¹⁵ und R¹⁶ bilden zusammengenommen mit dem Atom, an welches sie gebunden sind, Morpholinyl;
(3) C₄-C₁₀-Alkenyl; und
(4) C₃-C₁₀-Alkinyl.

2. Eine Verbindung gemäß Anspruch 1, welche gewählt ist aus der Gruppe bestehend aus Verbindungen, worin
(1) R -CH2-CH=CH2-(3-Chinolinyl) ist;
(2) R Allyl ist,
(4) R 2,3-Dihydroxypropyl ist;
(5) R 2,3-Epoxypropyl ist;
(6) R 2-Hydroxy-3-(imidazol-1-yl)propyl ist;
(7) R 2-Hydroxy-3-(morpholin-4-yl)propyl ist;
(8) R 2-Hydroxy-3-(benzylamino)propyl ist;
(9) R 2-Oxoethyl ist;
(10) R 2-Oxopropyl ist;
(11) R -CH2-C≡CH ist;
(12) R -CH2-CHOH-CH2-N3 ist;
(13) R -CH2-CH=N-OH ist;
(14) R -CH2-CH2OH ist;
(15) R -CH2-CH2NH2 ist;
(16) R -CH2-CN ist;
(17) R -CH2-Phenyl ist;
(18) R -CH2-CH=CH-Phenyl ist;
(19) R -CH2-CH=N-O-CH3 ist;
(20) R -CH2-CH=N-O-CH2-Phenyl ist;
(21) R -CH2-CH=N-N(CH3)2 ist;
(22) R -CH2-CH=N-NH(CH3) ist;
(23) R -CH2-CH=N-(4-Morpholinyl) ist;
(24) R -CH2-CH=N-NH(Phenyl) ist; und
(25) R -CH2-CH=N-N(Phenyl)2 ist;
(26) R Phenylpropyl ist;
(27) R -CH2CH=CH-(4-Methylphenyl) ist;
(28) R -CH2-CH(OH)-Phenyl ist;
(29) R -CH2-CH(Br)-CH2Br ist;
(30) R -CH2CH2NHCH2CH2CH2-Phenyl ist;
(31) R -CH2CH2 NHCH(CH2 Phenyl)CO2Me ist;
(32) R -CH2CH2NHCH2CH2CH3 ist;
(33) R -CH2CH2NHCH2CO2CH2CH2 ist;
(34) R -CH2CH2NHCH2CH2-Phenyl ist;
(35) R -CH2CH2NHCH2CH2-(4-Hydroxyphenyl) ist;
(36) R -CH2CH2NHCH2CH2-(3-Hydroxyphenyl) ist;
(37) R -CH2CH2NHCH2CH2-(3-Methoxyphenyl) ist;
(38) R -CH2CH2NHCH2CH2-(2-Methoxyphenyl) ist;
(39) R -CH2CH2NHCH2CH2-(4-Methoxyphenyl) ist;
(40) R -CH2CH2NHCH2-Phenyl ist;
(41) R -CH2CH2NHCH2CH2-(3-Chlorphenyl) ist;
(42) R -CH2CH2NHCH2CH2-(2-Chlorphenyl) ist;
(43) R -CH2CH2NHCH2CH2-(4-Chlorphenyl) ist;
(44) R -CH2CH2NHCH2CH2-O-Phenyl) ist;
(45) R -CH2CH2NHCH2CH2CH2-(4-Chinolinyl) ist;
(46) R -CH2CH2NHC2CH2CH2-(3-Chinolinyl) ist;
(47) R -CH2CH2NHCH2CH2CH2CH2-Phenyl ist;
(48) R -CH2-CH=N-NH-C(O)-NH2 ist;
(49) R -CH2-CH=N-NH-(2-Pyridinyl) ist;
(50) R -CH2-CH=N-(4-Methylpiperazinyl) ist;
(51) R -CH2-CH=N-O-Phenyl ist;
(52) R -CH2CH(OH)CH2NHCH2CH2-Phenyl ist;
(53) R -CH2CH(OH)CH2NHCH2-(4-Pyridinyl) ist;
(54) R (3-Jodphenyl)methyl ist; und
(55) R (4-Fluorphenyl)methyl ist.

3. Eine Verbindung gemäß Anspruch 2, worin
R -CH2-CH=CH2-(3-Chinolinyl) ist;
R Allyl ist;
R 2-Oxopropyl ist;
R -CH2-C=CH ist;
R -CH2-CH=N-OH ist;
R -CH2-CH2OH ist;
R -CH2-CH2NH2 ist; und
R -CH2-CN ist.

4. Eine pharmazeutische Zusammensetzung, die eine therapeutisch wirksame Menge einer Verbindung von irgendeinem der Ansprüche 1, 2 und 3 in Kombination mit einem pharmazeutisch verträglichen Träger umfaßt.

5. Verwendung einer Verbindung gemäß den Ansprüchen 1, 2 oder 3 zur Herstellung eines Medikaments zur Behandlung einer bakteriellen Infektion in einem Säugetier.

6. Verbindung gemäß Ansprüchen 1, 2 oder 3 für die Verwendung als ein Medikament.

## Revendications

1. Composé ayant la formule 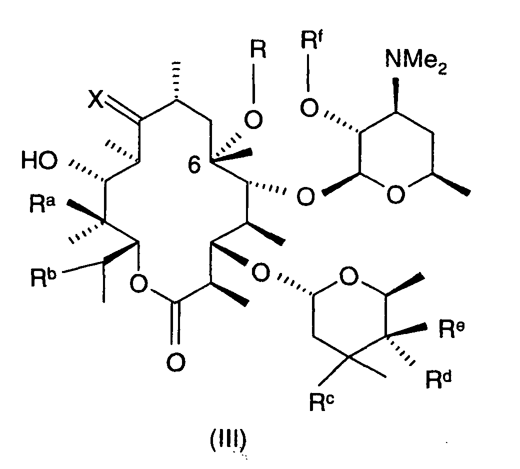 ou sel pharmaceutiquement acceptable de celui-ci, où
X est O,
Ra est hydroxy ;
Rb est l'hydrogène ;
Rc est l'hydrogène ;
Rd est hydroxy ;
Re est méthoxy ;
Rf est l'hydrogène ; et
R est choisi dans le groupe consistant en :
(2) C₂-C₁₀-alkyle substitué avec un substituant choisi dans le groupe consistant en
(a) halogène,
(b) hydroxy,
(c) C₁-C₃-alcoxy,
(d) C₁-C₃-alcoxy-C₁-C₃-alcoxy,
(e) oxo,
(f) -N₃,
(g) -CHO,
(h) O-SO₂-(C₁-C₆-alkyle substitué),
(i) -NR¹⁵R¹⁶ où R¹⁵ et R¹⁶ sont choisis dans le groupe consistant en
(i) hydrogène,
(ii) C₁-C₁₂-alkyle,
(iii) C₁-C₁₂-alkyle substitué,
(iv) C₁-C₁₂-alcényle,
(v) C₁-C₁₂-alcényle substitué,
(vi) C₁-C₁₂-alcynyle,
(vii) C₁-C₁₂-alcynyle substitué,
(viii) aryle,
(ix) C₃-C₈-cycloalkyle,
(x) C₃-C₈-cycloalkyle substitué,
(xi) aryle substitué,
(xii) hétérocycloalkyle,
(xiii) hétérocycloalkyle substitué,
(xiv) C₁-C₁₂-alkyle substitué avec aryle,
(xv) C₁-C₁₂-alkyle substitué avec aryle substitué,
(xvi) C₁-C₁₂-alkyle substitué avec hétérocycloalkyle,
(xvii) C₁-C₁₂-alkyle substitué avec hétérocycloalkyle substitué,
(xviii) C₁-C₁₂-alkyle substitué avec C₃-C₈-cycloalkyle,
(xix) C₁-C₁₂-alkyle substitué avec C₃-C₈-cycloalkyle substitué,
(xx) hétéroaryle,
(xxi) hétéroaryle substitué,
(xxii) C₁-C₁₂-alkyle substitué avec hétéroaryle, et
(xxiii) C₁-C₁₂-alkyle substitué avec hétéroaryle substitué,
ou
R¹⁵ et R¹⁶ combinés avec l'atome auquel ils sont liés forment morpholinyle ;
(3) C₄-C₁₀-alcényle ; et
(4) C₃-C₁₀-alcynyle.

2. Composé selon la revendication 1 qui est choisi dans le groupe consistant en les composés où
(1) R est -CH₂-CH=CH₂-(3-quinolinyle) ;
(2) R est allyle ;
(4) R est 2,3-dihydroxypropyle ;
(5) R est 2,3-époxypropyle ;
(6) R est 2-hydroxy-3-(imidazol-1-yl)propyle ;
(7) R est 2-hydroxy-3-(morpholin-4-yl)propyle ;
(8) R est 2-hydroxy-3-(benzylamino)propyle ;
(9) R est 2-oxoéthyle ;
(10) R est 2-oxopropyle ;
(11) R est -CH₂-C=CH ;
(12) R est -CH₂-CHOH-CH₂-N₃ ;
(13) R est -CH₂-CH=N-OH ;
(14) R est -CH₂-CH₂OH ;
(15) R est -CH₂-CH₂NH₂ ;
(16) R est -CH₂-CN ;
(17) R est -CH₂-phényle ;
(18) R est -CH₂-CH=CH-phény)e ;
(19) R est -CH₂-CH=N-O-CH₃ ;
(20) R est -CH₂-CH=N-O-CH₂-phényle ;
(21) R est -CH₂-CH=N-N(CH₃)₂ ;
(22) R est -CH₂-C_{H}=N-NH(CH₃) ;
(23) R est -CH₂-CH=N-(4-morpholinyle) ;
(24) R est -CH₂-CH=N-NH(phényle) ; et
(25) R est -CH₂-CH=N-N(phényle) 2 ;
(26) R et phénylpropyle ;
(27) R est -CH₂CH=CH-(4-méthylphényle) ;
(28) R est -CH₂-CH(OH)-phényle ;
(29) R est -CH₂-CH(Br)-CH₂Br ;
(30) R est -CH₂CH₂NHCH₂CH₂CH₂-phényle ;
(31) R est -CH₂CH₂NHCH(CH₂-phényl)CO₂Me ;
(32) R est -CH₂CH₂NHCH₂CH₂CH₃ ;
(33) R est -CH₂CH₂NHCH₂CO₂CH₂CH₂ ;
(34) R est -CH₂CH₂NHCH₂CH₂-phényle ;
(35) R est -CH₂CH₂NHCH₂CH₂-(4-hydroxyphényle) ;
(36) R est -CH₂CH₂NHCH₂CH₂-(3-hydroxyphényle) ;
(37) R est -CH₂CH₂NHCH₂CH₂-(3-méthoxyphényle) ;
(38) R est -CH₂CH₂NHCH₂CH₂-(2-méthoxyphényle) ;
(39) R est -CH₂CH₂NHCH₂CH₂-(4-méthoxyphényle) ;
(40) R est -CH₂CH₂NHCH₂-phényle ;
(41) R est -CH₂CH₂NHCH₂CH₂-(3-chlorophényle) ;
(42) R est -CH₂CH₂NHCH₂CH₂-(2-chlorophényle) ;
(43) R est -CH₂CH₂NHCH₂CH₂-(4-chlorophényle) ;
(44) R est -CH₂CH₂NHCH₂CH₂-O-phényle ;
(45) R est -CH₂CH₂NHCH₂CH₂CH₂-(4-quinolinyle) ;
(46) R est -CH₂CH₂NHCH₂CH₂CH₂-(3-quinolinyle) ;
(47) R est -CH₂CH₂NHCH₂CH₂CH₂CH₂-phényle ;
(48) R est -CH₂-CH=N-NH-C(O)-NH₂ ;
(49) R est -CH₂-CH=N-NH-(2-pyridinyle) ;
(50) R est -CH₂-CH=N-(4-méthylpipérazinyle) ;
(51) R est -CH₂-CH=_{N}-O-phényle ;
(52) R est -CH₂CH(OH)CH₂NHCH₂CH₂-phényle ;
(53) R est -CH₂CH(OH)CH₂NHCH₂-(4-pyridinyle) ;
(54) R est (3-iodophényl)méthyle ; et
(55) R est (4-fluorophényl)méthyle.

3. Composé selon la revendication 2 où
R est -CH₂-CH=CH₂-(3-quinolinyle) ;
R est allyle ;
R est 2-oxopropyle ;
R est -CH₂-C=CH ;
R est -CH₂-CH=N-OH ;
R est -CH₂-CH₂OH ;
R est -CH₂-CH₂NH₂ ; et
R est -CH₂-CN.

4. Composition pharmaceutique comprenant une quantité thérapeutiquement efficace d'un composé selon l'une quelconque des revendications 1, 2 et 3 en combinaison avec un support pharmaceutiquement acceptable.

5. Utilisation d'un composé selon la revendication 1, 2 ou 3 pour la fabrication d'un médicament pour maîtriser une infection bactérienne chez un mammifère.

6. Composé selon la revendication 1, 2 ou 3 destiné à être utilisé comme médicament.
